# FASCICULE DE BREVET EUROPEEN

(11) **EP 3 377 114 B1**
(45) Date de publication et mention de la délivrance du brevet: **08.07.2020**
(21) Numéro de dépôt: 16798706.4
(22) Date de dépôt: 18.11.2016
(51) Int. Cl.: A61K 47/69, A61K 47/54, A61K 9/113, A61K 9/107, A61P 37/04, A61K 38/38, A61K 39/00

(54) **COMPOSITION IMMUNOGÈNE SOUS FORME D'ÉMULSION COMPRENANT DEUX PHASES DISPERSÉES, L'UNE COMPRENANT UN ANTIGÈNE ET L'AUTRE COMPRENANT UN AGENT IMMUNOSTIMULANT**
IMMUNOGENE ZUSAMMENSETZUNG IN FORM EINER EMULSION MIT ZWEI DISPERGIERTEN PHASEN, EINE MIT EINEM ANTIGEN UND DIE ANDERE MIT EINER IMMUNOSTIMULIERENDEN VERBINDUNG
IMMUNOGENIC COMPOSITION IN THE FORM OF AN EMULSION COMPRISING TWO DISPERSED PHASES, ONE COMPRISING AN ANTIGEN AND THE OTHER COMPRISING AN IMMUNOSTIMULATING AGENT

(30) Priorité: 18.11.2015 FR 1561107; 18.11.2015 US 201514944825
(43) Date de publication de la demande: 26.09.2018
(73) Titulaire: Commissariat à l'Énergie Atomique et aux Énergies Alternatives, 75015 Paris (FR); Institut National de la Santé et de la Recherche Médicale (INSERM), 75013 Paris (FR)
(72) Inventeur: NAVARRO Y GARCIA, Fabrice, 38600 Fontaine (FR); BAYON, Emilie, 38000 Grenoble (FR); MARCHE, Patrice, 38240 Meylan (FR); COURANT, Thomas, 51100 Reims (FR)
(74) Mandataire: Lavoix
(86) Numéro de dépôt international: PCT/EP2016/078126
(87) Numéro de publication internationale: WO 2017/085248

(56) Documents cités:
- EP-A1- 3 103 485
- WO-A1-93/00160
- WO-A1-2010/018223
- WO-A1-2012/117377
- WO-A1-2014/032953
- WO-A1-2014/131809
- WO-A2-00/37045
- WO-A2-2007/015167

## Description

La présente invention concerne une composition immunogène sous forme d'émulsion comprenant une phase aqueuse continue, une première phase lipidique dispersée sous forme de gouttelettes et comprenant un antigène lié de façon covalente auxdites gouttelettes, et une deuxième phase dispersée comprenant un agent immunostimulant, son procédé de préparation et ses utilisations, notamment pour la production d'anticorps, en tant que médicament ou vaccin, ou dans une méthode d'immunisation.

Afin d'améliorer leur immunogénicité, les antigènes doivent souvent être co-administrés avec des agents immunostimulants.

La demande WO 2014/131809 décrit une composition immunogène comprenant une phase aqueuse continue et une phase dispersée sous forme de gouttelettes et comprenant :
- un lipide amphiphile,
- un lipide solubilisant comprenant au moins un glycéride d'acide gras,
- un co-tensioactif comprenant au moins une chaine composée de motifs d'oxyde d'alkylène,
- un tensioactif porteur d'un antigène, et
- éventuellement un agent immunostimulant,
et ses applications pour traiter ou prévenir une infection, un cancer, une maladie inflammatoire ou une allergie, pour induire une réponse immune contre l'antigène ou pour produire des anticorps.

Il est de plus en plus reconnu que les cellules dendritiques jouent un rôle central, dans l'initiation des réponses immunitaires et dans les communications intercellulaires avec les lymphocytes. L'activation des cellules dendritiques est particulièrement efficace via les « Toll-like récepteurs » (TLR) dont elles sont porteuses. De fait, la littérature rapporte l'utilisation de ligand de ces TLR comme agent immunostimulant. En particulier, des nanoparticules polymériques, par exemple à base de poly(acide lactique-co-glycolique (PLGA) ou à base d'un copolymère à blocs acide polylactique-co-hydroxyméthylglycolique (pLHMGA), ont été utilisées pour vectoriser des antigènes, et ont été co-administrées avec un ligand de TLR à titre d'agent immunostimmulant. Les particules de PLGA se dégradent rapidement en milieu biologique.

Ainsi, la littérature rapporte que la réponse immunitaire induite (en termes d'anticorps spécifiques produits et de cellules T cytotoxiques activées) est considérablement améliorée lors de la co-administration d'un antigène vectorisé sur une nanoparticule polymérique et d'un ligand de TLR (libre, non vectorisé sur la nanoparticule). La vectorisation de l'agent immunostimulant peut être bénéfique lorsque sont co-administrées deux types de particules polymériques distinctes : d'une part des particules vectorisant l'antigène, et d'autre part des particules vectorisant le ligand de TLR (Kasturi et Al, Nature, 470, 543-547, 2011). Les particules de PLGA utilisées avaient un diamètre de l'ordre de 300 nm. Les particules de PLGA ont généralement tendance à se dégrader rapidement en milieu biologique. Bien que des particules polymériques et lipidiques soient toutes deux décrites dans la littérature comme utilisables comme vecteur d'antigène, leurs comportements en milieux biologiques sont souvent différents, notamment car leurs tailles sont différentes (les particules de PLGA sont généralement plus grosses) et car les mécanismes impliqués pour leur interaction avec les cellules de l'immunité et pour le relargage de l'antigène sont différents.

Par conséquent, il existe un besoin de fournir des compositions immunogènes alternatives à celles existantes, et présentant au moins l'un des avantages suivants :
- pouvoir être facilement utilisées pour la production d'anticorps, en tant que médicament ou vaccin, ou dans une méthode d'immunisation,
- amplifier la réponse immunitaire, notamment par rapport à la co-administration d'un antigène vectorisé et d'un agent immunostimulant libre,
- être stables au stockage, notamment en évitant la fuite de l'antigène hors des vecteurs,
- pouvoir être préparées facilement, notamment en utilisant des méthodes industrielles et sans qu'il soit nécessaire d'adapter le procédé de préparation et les composants de l'émulsion pour chaque antigène.
- posséder une petite taille (diamètre hydrodynamique < 250 nm) pour faciliter la capture cellulaire par les cellules immunes et leur drainage lymphatique (Bachmann et al., Nature Reviews Immunology, 2010, 10, 787).

### [Composition immunogène]

L'invention est définie par les revendications. Tout objet ne relevant pas de la portée des revendications est fourni à titre informatif uniquement. Selon un premier objet, l'invention concerne une composition immunogène comprenant une phase aqueuse continue et au moins deux phases dispersées 1 et 2 sous forme de gouttelettes, où :
- la phase dispersée 1 comprend :
   - un lipide amphiphile 1,
   - un lipide solubilisant 1 comprenant au moins un glycéride d'acide gras,
   - un co-tensioactif 1 comprenant au moins une chaine composée de motifs d'oxyde d'alkylène,
   - un tensioactif 1 porteur d'un antigène de formule (I) suivante :

      (Li-X₁-H₁-Y₁)ᵥ-G-Z₁-Ag (I),

      dans laquelle :
      - L₁ représente un groupe lipophile,
      - X₁, Y₁, Z₁ et G représentent indépendamment un groupe de liaison,
      - H₁ représente un groupe hydrophile comprenant une chaîne polyalcoxylée,
      - v est un nombre entier de 1 à 8, et
      - Ag représente un antigène,
   dans laquelle le rapport molaire du tensioactif 1 porteur d'un antigène de formule (I) sur la somme du co-tensioactif 1 et du tensioactif 1 porteur d'un antigène de formule (I) est de 0,01 % à 5%, et
- la phase dispersée 2 comprend :
   - un lipide amphiphile 2,
   - un lipide solubilisant 2 comprenant au moins un glycéride d'acide gras,
   - un co-tensioactif 2 comprenant au moins une chaine composée de motifs d'oxyde d'alkylène,
   - un agent immunostimulant 2 porteur d'au moins un groupe anionique, et
- un tensioactif 2 cationique,
sous réserve que la phase dispersée 2 soit exempte de tensioactif porteur d'un antigène de formule (I).

Les inventeurs ont montré que la vectorisation d'un antigène par des gouttelettes permettait d'augmenter et d'améliorer la réponse immune dirigée contre ledit antigène. La réponse immune obtenue après injection d'un antigène vectorisé par les gouttelettes est notamment significativement plus importante et plus homogène que la réponse immune obtenue après injection de l'antigène seul. La composition immunogène peut donc être considérée comme une formulation adjuvante d'un antigène. L'utilisation des gouttelettes est donc particulièrement adaptée pour produire efficacement des anticorps dirigés contre un antigène, ainsi que dans le traitement de pathologies infectieuses, d'allergie, de cancers, d'une maladie liée à l'âge comme la dégénérescence maculaire liée à l'âge (DMLA), ou d'une maladie neuro-dégénérative, selon la nature de l'antigène inclus dans la composition immunogène.

L'invention repose également sur la découverte qu'une composition immunogène comprenant des gouttelettes dont certaines comprennent un antigène (phase dispersée 1), et d'autres comprennent un agent immunostimulant (phase dispersée 2), permet d'induire de fortes réponses immunes, en particulier des réponses immunes à la fois humorales et cellulaires.

La composition immunogène se présente sous forme d'une émulsion huile dans l'eau, de préférence une nanoémulsion huile dans l'eau. L'émulsion peut être simple ou multiple, notamment en comportant dans la phase dispersée 1 et/ou dans la phase dispersée 2 une seconde phase aqueuse.

### • Définitions

Au sens de la présente demande, l'expression « phase dispersée 1 » signifie les gouttelettes comprenant l'éventuelle huile 1/le lipide solubilisant 1/ le lipide amphiphile 1/le co-tensioactif 1/ l'éventuel agent d'intérêt 1 lipophile/le tensioactif 1 porteur d'un antigène de formule (1)/l'éventuel agent immunostimulant 1/l'éventuel ligand biologique de ciblage 1 (libre ou greffé au co-tensioactif 1)/l'éventuel tensioactif 1 cationique. La phase dispersée 1 est généralement exempte de phase aqueuse.

L'expression « phase dispersée 2 » signifie les gouttelettes comprenant l'éventuelle huile 2/le lipide solubilisant 2/ le lipide amphiphile 2/le co-tensioactif 2/ l'éventuel agent d'intérêt 2 lipophile/ l'agent immunostimulant 2/l'éventuel ligand biologique de ciblage 2 (libre ou greffé au co-tensioactif 2)/l'éventuel tensioactif 2 cationique. La phase dispersée 2 est généralement exempte de phase aqueuse.

La composition immunogène est typiquement exempte de liposomes.

Le terme « gouttelette » englobe à la fois les gouttelettes d'huile liquide proprement dites ainsi que les particules solides issues d'émulsions de type huile-dans-eau dans lesquelles la phase dispersée 1 ou 2 est solide. On utilise également l'abréviation LNP pour désigner les gouttelettes lorsque leur taille est nanométrique (pour « lipid nanoparticle »).

Les gouttelettes de la composition immunogène sont avantageusement monodisperses. L'écart type entre les diamètres minimum et maximum des gouttelettes par rapport au diamètre moyen est généralement inférieur ou égal à 30%, de préférence 20%. Le diamètre moyen des gouttelettes de la phase dispersée 1 et/ou 2 est de préférence de 20 à 300 nm, notamment de 40 à 250 nm et en particulier de 50 à 200 nm. Ces diamètres sont mesurés par diffusion quasi élastique de la lumière, par exemple sur un appareil ZetaSizer, Malvern. On peut également obtenir la taille de gouttelettes par microscopie électronique en transmission (TEM), par cryomicroscopie électronique en transmission (cryoTEM) ou encore par microscopie à force atomique (AFM). Des diamètres inférieurs à 20 nm et supérieurs à 250 nm sont difficiles à atteindre en pratique.

Le terme « lipide » désigne dans le cadre de cet exposé l'ensemble des corps gras ou des substances contenant des acides gras présents dans les graisses d'origine animale et dans les huiles végétales. Ce sont des molécules hydrophobes ou amphiphiles principalement constituées de carbone, d'hydrogène et d'oxygène et ayant une densité inférieure à celle de l'eau. Les lipides peuvent être à l'état solide à température ambiante (25°C), comme dans les cires, ou liquide, comme dans les huiles.

Le terme « amphiphile » désigne une molécule possédant une partie hydrophobe et une partie hydrophile, par exemple une partie apolaire hydrophobe et une partie polaire hydrophile.

Le terme « phospholipide » vise des lipides possédant un groupe phosphate, notamment les phosphoglycérides. Le plus souvent, les phospholipides comportent une extrémité hydrophile formée par le groupe phosphate éventuellement substitué et deux extrémités hydrophobes formées par des chaînes d'acides gras. Parmi les phospholipides, on peut citer en particulier la phosphatidylcholine, la phosphatidyl éthanolamine, la phosphatidyl inositol, la phosphatidyl sérine et la sphingomyéline.

Le terme « lécithine » désigne la phosphatidylcholine, c'est-à-dire un lipide formé à partir d'une choline, d'un phosphate, d'un glycérol et de deux acides gras. Il couvre de manière plus large les phospholipides extraits du vivant, d'origine végétale ou animale, dans la mesure où ils sont majoritairement constitués de phosphatidylcholine. Ces lécithines constituent généralement des mélanges de lécithines portant différents acides gras.

On entend par le terme « tensioactif » des composés à structure amphiphile qui leur confère une affinité particulière pour les interfaces de type huile/eau et eau/huile ce qui leur donne la capacité d'abaisser l'énergie libre de ces interfaces et de stabiliser des systèmes dispersés.

On entend par le terme « co-tensioactif » un tensioactif agissant en plus d'un premier tensioactif (à savoir le lipide amphiphile) pour abaisser davantage l'énergie de l'interface.

On entend par agent d'intérêt « lipophile », un agent d'intérêt qui se situe majoritairement, de préférence totalement, dans la phase dispersée, à l'intérieur ou en surface des gouttelettes. Un agent d'intérêt lipophile a des affinités pour des composés huileux (graisses, huiles, cires...) et solvants apolaires (toluène, hexane...). Les forces permettant la solubilisation de l'agent d'intérêt lipophile sont majoritairement des forces de London (interactions de Van der Waals). Un agent d'intérêt lipophile présente un coefficient de partage huile/eau élevé.

On entend par agent d'intérêt « hydrophile », un agent d'intérêt qui se situe majoritairement, de préférence totalement, dans la phase aqueuse continue. Sa solubilité dans l'eau est généralement supérieure à 1% en poids. La solubilisation dans l'eau des agents d'intérêt hydrophiles provient généralement de liaisons hydrogène et/ou ioniques entre les agents d'intérêt hydrophiles et l'eau.

Par « composition immunogène », on entend une composition susceptible d'être administrée à l'homme ou à l'animal afin d'induire une réponse immunitaire. Ladite réponse immunitaire peut être une réponse immunitaire humorale, i.e. une réponse immunitaire qui se traduit par une production d'anticorps neutralisants, et/ou une réponse immunitaire cellulaire cytotoxique, i.e. une réponse immunitaire qui se traduit par une activation de certaines cellules, notamment les cellules présentatrices d'antigènes (par exemple les cellules dendritiques), les lymphocytes T, les lymphocytes B, les lymphocytes NK (pour « Natural Killer » en anglais).

Par « antigène » (« Ag » dans la présente demande), on entend tout antigène (y compris un épitope spécifique) susceptible d'être utilisé dans un vaccin, à savoir toute molécule qui peut être spécifiquement reconnue par les cellules du système immunitaire, telles que les cellules dendritiques, les cellules B, et/ou les cellules T (Pulendran et al., Nature Immunology, 2011, 12, 509-517).

Dans certains modes de réalisation, l'antigène est un vecteur comprenant un polynucléotide codant un polypeptide antigénique, ledit polynucléotide étant lié de manière opérationnelle à une ou plusieurs séquence(s) régulatrice(s) qui permettent la régulation de l'expression dudit polynucléotide.

Dans certains modes de réalisation, l'antigène est un allergène. Des exemples d'allergènes peuvent être, à titre non limitatif, des allergènes de pollen (d'arbres, de graminées, etc.), des allergènes d'acarien (de la poussière domestique ou de stockage), des allergènes d'insecte (hyménoptères, de blattes, etc.), des allergènes d'animaux (de chien, de chat, de cheval, de rat, de souris, etc.), des allergènes de moisissure et des allergènes alimentaires. Les allergènes alimentaires peuvent provenir du lait, des oeufs, des légumes (dont cacahuète et soja), des noix et noisettes, du blé, de crustacés, de poissons et de mollusques et des produits qui en sont dérivés. En particulier, les allergènes alimentaires peuvent être l'ovalbumine ou le gluten.

Dans certains modes de réalisation, l'antigène utilisé dans l'invention peut également être dérivé de n'importe quel organisme vivant ou non vivant ; de fragments cellulaires ; d'anatoxine. L'antigène peut également être dérivé d'un microorganisme naturel ou atténué, tel qu'un virus, une bactérie, un parasite ou une levure.

Dans certains modes de réalisation, l'antigène peut être par exemple une portion d'une molécule antigénique, ou une molécule synthétique ou une molécule obtenue par les technologies recombinantes.

Dans certains modes de réalisation, l'antigène est un polypeptide, un carbohydrate ou un lipide.

Des exemples non limitatifs d'antigènes sont des antigènes dérivés :
(i) de virus, tels que des antigènes dérivés du virus de l'immunodéficience acquise humaine de type 1 ou 2 (VIH ou HIV pour « Human immunodeficiency virus » en anglais) (e.g. tat, nef, gp120, gp160, gp40, p24, gag, env, vif, vpr, vpu, rev) ; du virus de l'herpès humain de type 1 ou 2 (HSV pour « Herpès Simplex Virus » en anglais) (e.g. gH, gL, gM, gB, gC, gK, gE, gD, ICP27 , ICP 47, IC P 4, ICP36 from HSV1 or HSV2) ; du cytomegalovirus tels que gB, du virus Epstein Barr (e.g. gp350) ; du virus de la varicelle (e.g. gpl, II, III et 1E63) ; ou du virus de l'hépatite A, B (e.g. l'antigène de surface de l'hépatite B (« Hepatitis B Surface antigen » en anglais) ou l'antigène nucléocapsidique de l'hépatite (« Hepatitis core antigen » en anglais)) ; des paramyxoviruses tels que le virus respiratoire syncytial (« Respiratory Syncytial virus » en anglais), le parainfluenza virus, le virus de la rougeole, ou le virus des oreillons ; des papilloma virus (e.g. HPV6, 11, 16, 18, eg L1, L2, EI, E2, E3, E4, E5, E6, E7) ; des flavivirus tels que le virus de la fièvre jaune, le virus de la dengue, le virus de l'encéphalite de Saint-Louis, le virus de l'encéphalite japonaise ; le virus influenza (e.g. les proteins HA, NP, NA, ou M);
(ii) de bactéries, tels que des antigènes dérivés de bactéries du genre *Neisseria,* incluant *N. gonorrhea* et *N. meningitidis* (e.g, les proteins de liaison à la transferrine, les proteins de liaison à la lactoferrine, PiIC, les adhésines); du genre *Streptococcus,* incluant *S. pyogenes* (e.g. les protéines M, la protease C5A), *S. pneumoniae* (e.g. PsaA, PspA, streptolysine, les protéines de liaison à la choline), *S. agalactiae* et S. *mutans;* du genre *Haemophilus,* incluant *H. ducreyi, H. influenzae* de type B (e.g. PRP), *H. influenzae* non typable (e.g. OMP26, les adhésines de haut poids moléculaire, P5, P6, protéine D, lipoprotéine D, la fibrine); du genre *Moraxella,* incluant *M catarrhalis* (e.g. les adhésines de haut et bas poids moléculaire et les invasines); du genre *Bordetella,* incluant *B. pertussis* (e.g. pertactine, la toxine pertussique, l'hemagglutinine fimlenteuse, l'adénylate cyclase), *B. parapertussis* et B. *bronchiseptica;* du genre *Mycobacterium,* incluant *M. tuberculosis* (e.g. ESAT6, l'antigène 85A, -B ou -C, MPT 44, MPT59, MPT45, HSP10, HSP65, HSP70, HSP75, HSP90, PPD 19kDa [Rv3763], PPD 38kDa [Rv0934]), *M. bovis, M. leprae, M. avium, M. paratuberculosis* et *M. smegmatis;* du genre *Legionella,* incluant *L. pneumophila;* du genre *Escherichia,* incluant *E. coli* entérotoxique (e.g. les facteurs de colonization, la toxine thermolabile ou la toxine thermostable), *E. coli* entérohémorragique, *E. coli* entéropathogénique (e.g. la vérotoxine); du genre *Vibrio,* incluant *V. cholera* (la toxine cholérique); du genre *Shigella,* incluant *S. sonnei, S. dysenteriae* et *S. flexnerii;* du genre *Yersinia,* incluant *Y. enterocolitica* (e.g. la protéine Yop), *Y. pestis, Y. pseudotuberculosis;* du genre *Campylobacter,* incluant *C. jejuni* (e.g. les toxines, adhésines et invasines) et *C. coli;* du genre *Salmonella,* incluant *S. typhi, S. paratyphi, S. choleraesuis, S. enteritidis;* du genre *Listeria,* incluant *L. monocytogenes;* du genre *Helicobacter,* incluant *H. pylon* (e.g. l'uréase, la catalase, la toxine vacuolaire); du genre *Pseudomonas,* incluant *P. aeruginosa;* du genre *Staphylococcus,* incluant S. *aureus, S. epidermidis;* du genre *Enterococcus,* incluant *E. faecalis, E. faecium;* du genre *Clostridium,* incluant *C. tetani* (e.g. la toxine tétanique), *C. botulinum* (e.g. la toxine botulique), *C. difficile* (e.g. les toxines A et B); du genre *Bacillus,* incluant *B. anthracis;* du genre *Corynebacterium,* incluant *C. diphtheriae* (e.g. la toxine diphtérique); du genre *Borrelia,* incluant *B. burgdorferi* (e.g. OspA, OspC, DbpA, DbpB), *B. garinii* (e.g. OspA, OspC, DbpA, DbpB), *B. afzelii* (e.g. OspA, OspC, DbpA, DbpB), *B. andersonii* (e.g. OspA, OspC, DbpA, DbpB), *B. hermsii;* du genre *Ehrlichia,* incluant *E. equi* et l'agent de l'Ehrlichiose granulocytaire humaine; du genre *Rickettsia,* incluant *R. rickettsii;* du genre *Chlamydia,* incluant *C. trachomatis* (e.g. MOMP, les protéines de liaison à l'héparine), *C. pneumoniae* (e.g. MOMP, les protéines de liaison à l'héparine), *C. psittaci;* du genre *Leptospira,* incluant *L. interrogans;* du genre *Treponema,* incluant *T. pallidum* (e.g. les protéines rares de la membrane externe), *T. denticola, T. hyodysenteriae;*
(iii) de parasites, tels que des antigènes dérivés de parasites du genre *Plasmodium,* incluant *P. falciparum* (e.g. RTS,S et TRAP); du genre *Toxoplasma,* incluant *T. gondii* (e.g. SAG2, SAG3, Tg34); du genre *Entamoeba,* incluant *E. histolytica;* du genre *Babesia,* incluant *B. microti;* du genre *Trypanosoma,* incluant *T. cruzi;* du genre *Giardia,* incluant *G. lamblia;* du genre *Leishmania,* incluant *L. major;* du genre *Pneumocystis,* incluant *P. carinii;* du genre *Trichomonas,* incluant *T. vaginalis;* du genre *Schisostoma,* incluant *S. mansoni,* ou de levures du genre *Candida,* incluant *C. albicans;* du genre *Cryptococcus,* incluant *C. neoformans.*

Dans certains modes de réalisation, l'antigène est un antigène tumoral et peut être utile pour le traitement immunothérapeutique de cancers. Les antigènes tumoraux peuvent dériver de cancer de la prostate, du sein, du colon, du poumon, du foie, du pancréas, du rein, de la vessie, de mélanome, de carcinome, de sarcome. Des exemples non limitatifs d'antigènes tumoraux dérivés de mélanome, de carcinome (poumon, vessie), ou de sarcome sont MAGE 1, 3 et MAGE 4, PRAME, BAGE, Lage, SAGE, HAGE ou GAGE. Des exemples non limitatifs d'antigènes dérivés de cancer de la prostate sont l'antigène spécifique de la prostate (PSA pour « Prostate specific antigen » en anglais), PAP, PSCA, PSMA, P501S ou la prostase. Des exemples non limitatifs d'antigènes dérivés de cancer du sein sont Muc-1, Muc-2, EpCAM, her 2/ Neu, la mammaglobine. D'autres exemples d'antigènes tumoraux utiles dans le contexte de la présente invention sont Plu-1, HASH-1, HasH-2, Cripto, Criptin, tyrosinase, survivine.

Dans certains modes de réalisation, l'antigène comprend ou consiste en un fragment d'au moins 6, 7, 8, 10, 15, 20, 30, 40, 50, 75, 100, 150, 200, 300, 400, 500 acides aminés contiguës d'un antigène tel que défini ci-dessus.

Dans certains modes de réalisation, l'antigène est une protéine de fusion comprenant ou consistant en au moins 2 antigènes ou fragments d'antigène tels que définis ci-dessus.

On entend par « agent immunostimulant », aussi dénommé « adjuvant » ou «molécule immunomodulatrice », une substance capable d'améliorer, ou d'augmenter, la réponse immune induite par l'antigène tel que défini ci-dessus. Des agents immunostimulants appropriés incluent des sels d'aluminium, des sels de calcium, magnésium, fer ou zinc, la saponine, le lipide A (aussi connu sous le nom de MPLA pour « Monophosphoryl Lipid A » en anglais) ou l'un de ses dérivés, un oligonucléotide immunostimulant, un alkyl phosphate glucosamide, des cytokines, des chimiokines, des ligands de « Toll-like récepteurs » (TLR) ou une combinaison de ces composés. Des exemples de saponines sont Quil A et de ses fragments purifiés sont QS7 et QS21. Des exemples de cytokines sont l'interleukine 1 beta (IL-1β), l'interleukine 6 (IL-6), l'interféron gamma (IFN-γ), le facteur de nécrose alpha (« Tumor Necrosis Factor alpha » (TNF-α) en anglais). Des exemples de chimiokines sont MCP-1 (Monocyte chimoattractant protein 1, aussi connue sous la dénomination CCL-2), MIP-1 alpha (aussi connue sous la dénomination CCL-3) et MIP-1 beta (aussi connue sous la dénomination CCL-4).

On entend par le terme « acide gras » désigner des acides carboxyliques aliphatiques présentant une chaîne carbonée d'au moins 4 atomes de carbone. Les acides gras naturels possèdent une chaîne carbonée de 4 à 28 atomes de carbone (généralement un nombre pair). On parle d'acide gras à longue chaîne pour une longueur de 14 à 22 carbones et à très longue chaîne s'il y a plus de 22 carbones.

On entend par le terme « chaîne hydrocarbonée » désigner une chaîne composée d'atomes de carbone et d'hydrogène, saturée ou insaturée (double ou triple liaison). Les chaînes hydrocarbonées préférées sont les alkyle ou alcényle.

On entend par le terme « alkylène » désigner un groupe divalent aliphatique hydrocarboné, saturé, linéaire ou ramifié, de préférence linéaire.

Par « ester activé », on entend un groupe de formule -CO-LG, par « carbonate activé », on entend un groupe de formule -O-CO-LG, par « carbamate activé », on entend un groupe de formule -NH-CO-LG, où LG est un bon groupe partant notamment choisi parmi un brome, un chlore, un imidazolyle, un pentafluorophénolate, un pentachlorophénolate, un 2,4,5-trichlorophénolate, 2,4,6-trichlorophénolate, un groupe -O-succinimidyle, -O-benzotriazolyle, -O-(7-aza-benzotriazolyle) et -O-(4-nitrophényle).

Par composant « libre » ou « sans vecteur », on entend que le composant n'est pas lié aux gouttelettes des phases dispersées 1 et 2 (ni encapsulé, ni lié par liaison covalente ou électrostatique).

Les modes de réalisation décrits pour chacun des composants de la composition immunogène peuvent bien sûr être combinés entre eux. De plus, lorsqu'un composant est constitué de plusieurs radicaux (par exemple le tensioactif 1 porteur d'un antigène de formule (I) est composé de différents radicaux L₁-, -X₁-, -H₁-, -Y₁, -G-, -Z₁- et Ag), les différents modes de réalisation de chacun des radicaux peuvent bien sûr être combinés entre eux.

La composition immunogène selon l'invention comprend au moins deux phases dispersées, généralement deux phases dispersées : la phase dispersée 1 et la phase dispersée 2.

Dans le cadre de cette demande, les composés désignés « 1 » sont des composants de la phase dispersée 1, et les composés désignés « 2 » sont des composants de la phase dispersée 2. Par « la phase dispersée 1 et/ou 2 » comprend un « composant 1 et/ou 2 », on entend que la phase dispersée 1 peut comprendre un composant 1 et/ou que la phase dispersée 2 peut comprendre un composant 2.

Chacune des phases dispersées 1 et 2 comprend un lipide amphiphile, un lipide solubilisant, et un co-tensioactif.

La phase dispersée 1 et la phase dispersée 2 sont nécessairement différentes : la phase dispersée 1 comprend un tensioactif 1 porteur d'un antigène de formule (I), alors que la phase dispersée 2 en est exempte.
La phase dispersée 2 comprend un agent immunostimulant 2. La phase dispersée 1 peut comprendre un agent immunostimulant 1, ou, de manière préférée, en être exempte.

### • Tensioactif 1 porteur d'un antigène de formule (I) (composant de la phase dispersée 1)

La phase dispersée 1 de la composition immunogène selon l'invention comprend un tensioactif 1 de formule (I) qui est porteur d'un antigène. Ce tensioactif 1 permet de lier de façon covalente l'antigène Ag aux gouttelettes de la phase dispersée 1.

Le tensioactif 1 de formule (I) se situe dans la couronne des gouttelettes de l'émulsion, le(s) groupe(s) L₁ étant dirigés vers l'intérieur des gouttelettes alors que l'antigène Ag est dirigé vers l'extérieur des gouttelettes, vers la phase aqueuse continue.

Le groupe X₁ est un groupe de liaison liant les groupes lipophile et hydrophobe. Le groupe G est un groupe de liaison entre les parties [lipophile-hydrophile] et l'antigène. Le groupe Y₁ est un groupe de liaison liant le groupe G aux parties [lipophile-hydrophile].

Dans un mode de réalisation, dans la formule (I) susmentionnée :
- L₁ est choisi parmi :
   - un groupe R ou R-(C=O)-, où R représente une chaîne hydrocarbonée linéaire comprenant de 7 à 23 atomes de carbone, de préférence de 11 à 23 atomes de carbone,
   - un ester ou un amide d'acides gras comprenant de 8 à 24 atomes de carbone (de préférence de 12 à 24 atomes de carbone) et de phosphatidyléthanolamine, tel que le distéaryl phosphatidyléthanolamine (DSPE), et
   - un poly(oxyde de propylène), et/ou
- X₁, Y₁ et Z₁ sont indépendamment choisis parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
   - un groupe Alk étant un alkylène comprenant de 1 à 8 atomes de carbone comprenant éventuellement un cycle (par exemple un radical 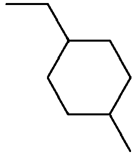 ), et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents, et/ou
- H₁ est choisi parmi un poly(oxyde d'éthylène) comprenant typiquement de 3 à 500 unités oxyde d'éthylène, de préférence de 20 à 200 unités oxyde d'éthylène, et/ou
- G comprend au moins un groupe G' ayant l'une des formules suivantes (le(s) groupe(s) Y₁ et Z₁ pouvant être reliés à gauche ou à droite des formules décrites ci-dessous, par exemple, lorsque G est un groupe G' de formule (XI), le tensioactif 1 de formule (I) peut avoir la formule la formule L₁-X₁-H₁-Y₁-CONH-Z₁-Ag ou L₁-X₁-H₁-Y₁-NHCO-Z₁-Ag) : 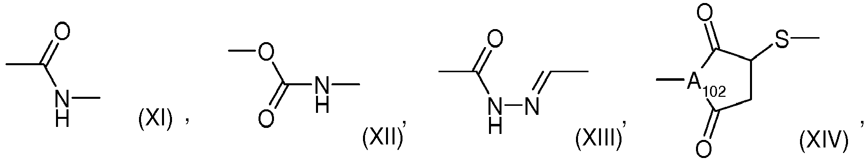 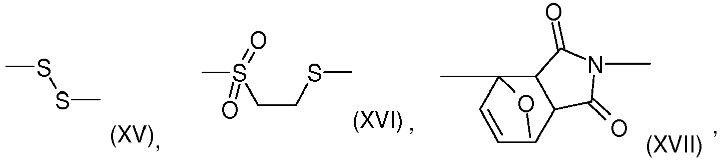 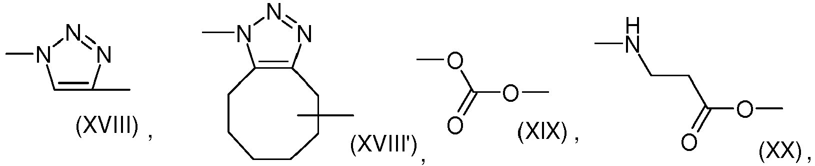 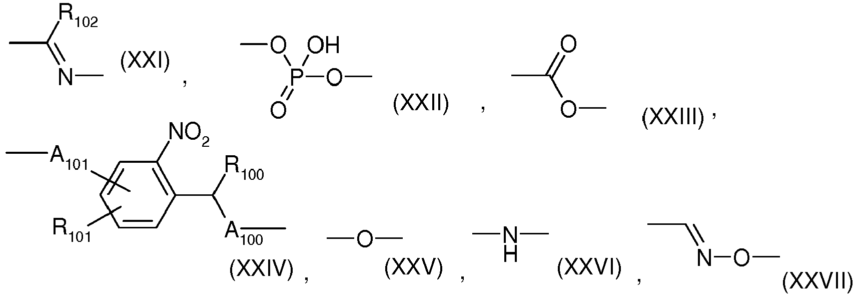 où A₁₀₂ représente CH ou N, R₁₀₂ représente H ou une chaîne hydrocarbonée linéaire comprenant de 1 à 6 atomes de carbone, A₁₀₁ représente -O-, -NH-(CO)- ou -O-(CO)-, R₁₀₀ représente H ou un méthyle, A₁₀₀ représente -O- ou -NH- et R₁₀₁ représente H, Me ou -OMe.

Par la formule 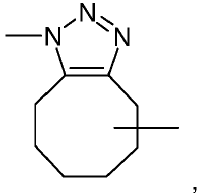 on entend que le groupe Z₁ ou le groupe Y₁ peut être relié à n'importe lequel des six atomes du cyclooctyle et par la formule 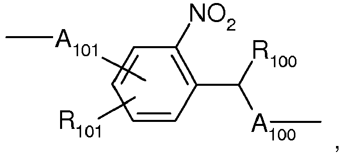 on entend que les groupe A₁₀₁ et R₁₀₁ peuvent être relié à n'importe lequel des quatre atomes du phényle.

Notamment, v représente 1 ou 2. v représente de préférence 1.

Le groupe G peut comprendre un ou plusieurs des groupes G' définis ci-dessus.

Ainsi, dans un premier mode de réalisation, le groupe G est constitué d'un groupe G'. Dans ce mode de réalisation, dans la formule (I), v représente 1.

Dans un second mode de réalisation, le groupe G répond à la formule -G'-Y₃-G'-dans laquelle :
- Y₃ représente un groupe de liaison, notamment choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
   - un groupe Alk étant un alkylène comprenant de 1 à 6 atomes de carbone, et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents
- chacun des G' représente indépendamment un groupe de formule (XI) à (XXVI) susmentionnés, et de préférence, les deux groupes G' de la formule -G'-Y₃-G'- sont identiques.
Ce mode de réalisation est particulièrement intéressant lorsque les deux groupes G' sont identiques et comprennent une fonction clivable. En effet, il suffit alors de cliver une seule des deux fonctions pour rompre les liaisons covalentes entre les gouttelettes de la composition immunogène et l'antigène, ce qui améliore les chances de succès du clivage et donc la libération de l'antigène hors des gouttelettes après capture cellulaire.

Lorsque L₁ représente un groupe R-(C=O)-, où R représente une chaîne hydrocarbonée linéaire comprenant de 7 à 23 atomes de carbone, L₁ représente un groupe issu d'acide gras comprenant de 8 à 24 atomes de carbone.

Par «L₁ représente un ester ou un amide d'acides gras comprenant de 8 à 24 atomes de carbone et de phosphatidyléthanolamine», on entend qu'il représente un groupe de formule : 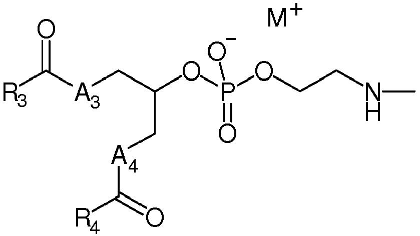 dans laquelle
- R₃ et R₄ représentent indépendamment une chaîne hydrocarbonée linéaire comprenant de 7 à 23 atomes de carbone, de préférence de 11 à 23 atomes de carbone,
- A₃ et A₄ représentent O ou NH, et
- M représente H ou un cation.

Dans un mode de réalisation, dans la formule (I) susmentionnée, le radical L₁-X₁-H₁- consiste en l'un des groupes de formules suivantes (le radical -Y₁-G-Z₁-Ag étant relié à droite des formules décrites ci-dessous): 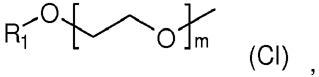 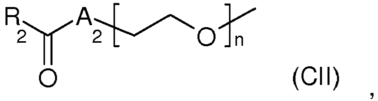 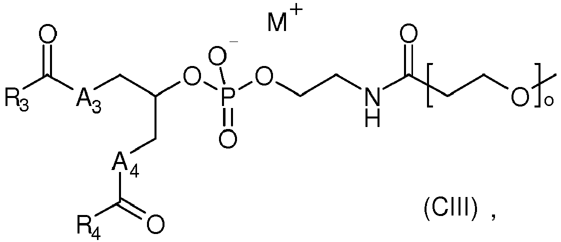 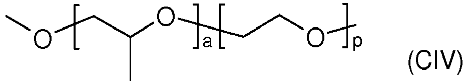 dans lesquelles :
- R₁, R₂, R₃ et R₄ représentent indépendamment une chaîne hydrocarbonée linéaire comprenant de 7 à 23 atomes de carbone, de préférence de 11 à 23 atomes de carbone,
- A₂, A₃ et A₄ représentent O ou NH,
- m, n, o et p représentent indépendamment des nombres entiers de 3 à 500, de préférence 20 à 200, et
- a représente un nombre entier de 20 à 120,
- M représente H ou un cation.

Le radical L₁-X₁-H₁- de formule (CII) est préféré. En effet, il est facile à préparer (notamment par formation d'un ester ou d'une amide entre un acide gras et un dérivé de poly(éthylène glycol)).

Le radical L₁-X₁-H₁- de formule (CII) avec A₂ représente NH sont particulièrement préférés, car les tensioactifs 1 comprenant de tels radicaux permettent d'éviter la fuite des agents d'intérêts 1 lipophiles et/ou ligand de ciblage 1 et/ou agent immunostimulant 1 éventuellement présents hors des gouttelettes de la composition immunogène plus efficacement que des tensioactifs 1 comprenant un radical L₁-X₁-H₁- de formule (CII) avec A₂ représente O.

Dans un mode de réalisation, dans la formule (I) :
- v représente 1,
- L₁ est R-(C=O)-, où R représente une chaîne hydrocarbonée linéaire comprenant de 7 à 23 atomes de carbone, de préférence de 11 à 23 atomes de carbone,
- H₁ est un poly(oxyde d'éthylène) comprenant de 3 à 500 unités oxyde d'éthylène,
- X₁ représente -O- ou -NH-,
- G est constitué d'un groupe G' de formule (XIV) dans laquelle A₁₀₂ représente N,
- Y₁ représente -CH₂-CH₂-NH-CO-CH₂-CH₂- (groupe Alk-Z-Alk ci-dessus avec Alk représente -CH₂-CH₂- et Z représente -NH-(CO)-) et Z₁ représente 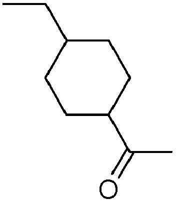 (groupe Alk-Z ci-dessus avec Alk représente 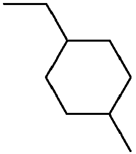 et Z représente (CO)), et le tensioactif 1 de formule (I) de la composition immunogène a alors la formule (I') suivante : 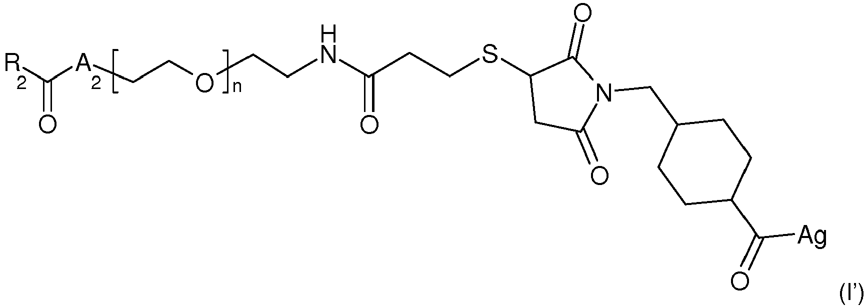 dans laquelle :
   - R₂ représente une chaîne hydrocarbonée linéaire comprenant de 7 à 23 atomes de carbone, notamment de 11 à 23 atomes de carbone, de préférence 17,
   - A₂ représente O ou NH, de préférence NH, et
   - n représente un nombre entier de 3 à 500, de préférence de 20 à 200, notamment 100,
   - Ag représente un antigène.

Dans un mode de réalisation, le groupe H₁ est choisi parmi un poly(oxyde d'éthylène) comprenant plus de 3 unités de poly(oxyde d'éthylène), voire plus de 20 unités, notamment plus de 50 (dans les formules susmentionnées, m, n, o ou p sont de préférence supérieurs à 3, voire 20, notamment plus de 50).

Dans un mode de réalisation, le groupe G du tensioactif 1 de formule (I) de la composition immunogène comprend une fonction clivable, notamment chimiquement (lorsque le tensioactif 1 de formule (I) est mis en contact avec un composé chimique capable de cliver la fonction du groupe G), électrochimiquement, à certains pH (pH basique ou acide), par des enzymes, par la lumière (lumière visible, ultraviolets ou infrarouge) et/ou au-delà de certaines températures. Généralement, le groupe G comprend alors un groupe G' comprenant une fonction clivable.

Ce mode de réalisation est intéressant car il peut permettre la délivrance de l'antigène Ag localisé à l'endroit souhaité où le stimulus chimique, électrochimique, de pH ou de température se produit. Par exemple, il est possible de délivrer l'antigène dans le compartiment intracellulaire des cellules cibles lorsque l'antigène est lié à la gouttelette par une liaison dithiol (-S-S-) (c'est-à-dire lorsque le groupe G comprend au moins un groupe G' comprenant un groupe (XV)). Une fois phagocyté, la particule porteuse d'antigène se retrouve dans l'endosome où la liaison (-S-S-) sera réduite par la gluthathione. L'antigène libre pourra alors s'échapper de l'endosome vers le cytosol pour y subir une présentation croisée, on parle alors d'échappement endosomal (Joffre et al., Nature Reviews Immunology 2012, 12, 557-569).

Par exemple :
- la fonction β-cétoaminoester du groupe G' de formule (XX) est clivable à pH acide (typiquement autour de 5),
- la fonction disulfure du groupe G' de formule (XV) est clivable aux ultraviolets, électrochimiquement, chimiquement (par exemple par mise en contact avec un agent réducteur, tel que la tris(2-carboxyéthyl)phosphine (TCEP) ou le dithiothréitol (DTT)), ou par des enzymes telles que des thioréductases,
- la fonction amide du groupe G' de formule (XI) est clivable par des enzymes telles que des protéases,
- la fonction phosphate du groupe G' de formule (XXII) est clivable par des enzymes telles que des phosphatases,
- la fonction imine des groupes G' de formules (XXI) et (XIII) sont clivables à pH acide ou au-delà de certaines températures,
- le cycle cyclohexène du groupe G' de formule (XVII) est clivable au-delà de certaines températures (par rétro Diels-Alder),
- la fonction carbonate du groupe G' de formule (XIX) et la fonction carbamate du groupe G de formule (XII) sont clivables à pH acide ou chimiquement (par exemple par réaction avec un agent nucléophile),
- la fonction orthonitrobenzyle du groupe G' de formule (XXIV) est clivable sous l'action de la lumière à 365 nm.

L'homme du métier, au vu de ses connaissances générales, sait quelles fonctions sont clivables et dans quelles conditions. Il est notamment à même de choisir la fonction du groupe G' du tensioactif 1 de formule (I) pour qu'elle soit clivable dans les conditions rencontrées dans l'application souhaitée de la composition immunogène selon l'invention.

Généralement, la phase dispersée 1 comporte de 0,01 à 3% en poids, de préférence de 0,1 à 1,3% en poids et tout particulièrement de 0,2 à 0,7% en poids de tensioactif 1 de formule (I).

Dans un mode de réalisation, la composition immunogène selon l'invention comprend plusieurs tensioactifs 1 de formule (I) (par exemple deux tensioactifs 1 de formule (I)), chacun des tensioactifs 1 de formule (I) étant porteur d'un antigène de nature différente. Certaines utilisations biologiques peuvent en effet nécessiter l'administration de plusieurs antigènes en même temps.

### • Lipide amphiphile 1 ou 2 (composant de la phase dispersée 1 ou 2)

La composition immunogène comprend un lipide amphiphile 1 à titre de tensioactif qui permet la formation des gouttelettes de la phase dispersée 1, et un lipide amphiphile 2 à titre de tensioactif qui permet la formation des gouttelettes de la phase dispersée 2. La nature amphiphile du tensioactif 1 ou 2 assure la stabilisation des gouttelettes au sein de la phase continue aqueuse.

Les lipides amphiphiles 1 et 2 peuvent être identiques ou différents. De préférence, ils sont identiques.

Les lipides amphiphiles comportent une partie hydrophile et une partie lipophile. Ils sont généralement choisis parmi les composés dont la partie lipophile comprend une chaîne saturée ou insaturée, linéaire ou ramifiée, ayant de 8 à 30 atomes de carbone. Ils peuvent être choisis parmi les phospholipides, les cholestérols, les lysolipides, les sphingomyélines, les tocophérols (non estérifiés), les glucolipides, stéarylamines, les cardiolipines d'origine naturelle ou synthétique ; les molécules composées d'un acide gras couplé à un groupement hydrophile par une fonction éther ou ester tels que les esters de sorbitan comme par exemple les monooléate et monolaurate de sorbitan vendus sous les dénominations Span® par la société Sigma; les lipides polymérisés ; les lipides conjugués à de courtes chaînes d'oxyde de polyéthylène (PEG) tels que les tensioactifs non-ioniques vendus sous les dénominations commerciales Tween® par la société ICI Americas, Inc. et Triton® par la société Union Carbide Corp.; les esters de sucre tels que les mono- et di-laurate, mono- et di-palmitate, mono- et distéarate de saccharose; lesdits tensioactifs pouvant être utilisés seuls ou en mélanges.

Les phospholipides sont des lipides amphiphiles particulièrement préférés, notamment les phospholipides choisis parmi la phosphatidylcholine, la phosphatidyléthanolamine, la phosphatidylsérine, le phosphatidylglycérol, le phosphatidylinositol, le phosphatidyl-acide phosphatidique non-hydrogéné ou hydrogéné, notamment vendu par la société Lipoid.

La lécithine est le lipide amphiphile 1 et/ou 2 préféré.

Généralement, la phase dispersée 1 comporte de 0,01 à 99% en poids, de préférence de 5 à 75% en poids, en particulier de 5 à 60% et tout particulièrement de 5 à 45% en poids de lipide amphiphile 1. De même, généralement, la phase dispersée 2 comporte de 0,01 à 99% en poids, de préférence de 5 à 75% en poids, en particulier de 5 à 60% et tout particulièrement de 5 à 45% en poids de lipide amphiphile 2.

La quantité de lipide amphiphile 1 et 2 contribue avantageusement à contrôler la taille des gouttelettes de la phase dispersée 1 et 2 de la composition immunogène.

### • Lipide solubilisant 1 ou 2 (composant de la phase dispersée 1 ou 2)

La composition immunogène comprend un lipide solubilisant 1 et un lipide solubilisant 2, qui permettent notamment :
- d'augmenter la stabilité physicochimique de la composition immunogène, et
- lorsque la composition immunogène comprend un agent d'intérêt 1 et/ou 2 lipophile et/ou un ligand de ciblage 1 et/ou 2 et/ou un agent immunostimulant 1 et/ou 2 encapsulé(s) dans les gouttelettes:
   - de solubiliser l'agent d'intérêt 1 et/ou 2 lipophile et/ou le ligand de ciblage 1 et/ou 2 et/ou l'agent immunostimulant 1 et/ou 2, et
   - d'améliorer le contrôle du relargage de l'agent d'intérêt 1 et/ou 2 lipophile et/ou du ligand de ciblage 1 et/ou 2 et/ou de l'agent immunostimulant 1 et/ou 2.

Les lipides solubilisants 1 et 2 peuvent être identiques ou différents. De préférence, ils sont identiques.

De préférence, le lipide solubilisant 1 et/ou le lipide solubilisant est(sont) solide(s) à température ambiante (20°C).

Le lipide solubilisant 1 et/ou 2 peut(vent) notamment être constitué(s) de dérivés du glycérol, et en particulier de glycérides obtenues par estérification de glycérol avec des acides gras, notamment dans le cas où le lipide amphiphile 1 et/ou 2 est un phospholipide.

Les lipides solubilisants préférés, en particulier pour les phospholipides, sont les glycérides d'acides gras, notamment d'acides gras saturés, et en particulier d'acides gras saturés comportant 8 à 18 atomes de carbone, encore préféré 12 à 18 atomes de carbone. Avantageusement, le lipide solubilisant 1 et/ou 2 est(sont) constitué(s) d'un mélange complexe de différents glycérides. Par « mélange complexe », on entend un mélange de mono, di et triglycérides, comprenant des chaînes grasses de différentes longueurs, les dites longueurs s'étendant préférentiellement de C8 à C18, par exemple, en association, des chaînes en C8, C10, C12, C14, C16 et C18, ou de C10 à C18, comprenant par exemple en association, des chaînes en C10, C12, C14, C16 et C18.

Selon un mode de réalisation, lesdites chaînes grasses peuvent contenir une ou plusieurs insaturations.

De préférence, le lipide solubilisant 1 et/ou 2 est(sont) constitué(s) d'un mélange de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18.

De préférence, le lipide solubilisant 1 et/ou 2 est(sont) constitué(s) d'un mélange de glycérides d'acides gras saturés comportant 0% à 20% en poids d'acides gras en C8, 0% à 20% en poids d'acides gras en C10, 10% à 70% en poids d'acides gras en C12, 5% à 30% en poids d'acides gras en C14, 5% à 30% en poids d'acides gras en C16 et 5% à 30% en poids d'acides gras en C18.

Les mélanges des glycérides semi-synthétiques solides à température ambiante vendus sous la dénomination commerciale Suppocire®NB par la société Gattefossé et approuvé pour un usage chez l'homme sont des lipides solubilisants particulièrement préférés. Les Suppocire® de type N sont obtenues par estérification directe d'acides gras et de glycérol. Il s'agit de glycérides hémi-synthétiques d'acides gras saturés de C8 à C18, dont la composition quali-quantitative est indiquée dans le tableau 1 ci-dessous.

**Tableau 1 : Composition en acides gras du Suppocire® NB de Gattefossé**

| Longueur de chaînes | [% en poids] |
|---|---|
| C8 | 0,1 à 0,9 |
| C10 | 0,1 à 0,9 |
| C12 | 25 à 50 |
| C14 | 10 à 24,9 |
| C16 | 10 à 24,9 |
| C18 | 10 à 24,9 |

Les lipides solubilisants précités permettent d'obtenir une composition immunogène avantageusement stable. Sans vouloir être lié à une théorie particulière, il est supposé que les lipides solubilisants précités permettent d'obtenir dans la composition immunogène des gouttelettes présentant un cœur amorphe. Le cœur ainsi obtenu présente une viscosité interne élevée sans pour autant présenter de cristallinité. Or, la cristallisation est néfaste pour la stabilité de la composition immunogène car elle conduit généralement à une agrégation des gouttelettes et/ou à une expulsion de l'agent d'intérêt 1 et/ou 2 lipophile et/ou du ligand de ciblage 1 et/ou 2 et/ou de l'agent immunostimulant 1 et/ou 2, si présent(s), à l'extérieur des gouttelettes. Ces propriétés physiques favorisent la stabilité physique de la composition immunogène.

La quantité de lipide solubilisant 1 et 2 peut varier largement en fonction de la nature et de la quantité de lipide amphiphile 1 et 2 présent dans les phases dispersées 1 et 2. Généralement, la phase dispersée 1 comporte de 1 à 99% en poids, de préférence de 5 à 80% en poids et tout particulièrement de 30 à 75% en poids de lipide solubilisant 1. De même, généralement, la phase dispersée 2 comporte de 1 à 99% en poids, de préférence de 5 à 80% en poids et tout particulièrement de 30 à 75% en poids de lipide solubilisant 2.

### • Co-tensioactif 1 ou 2 (composant de la phase dispersée 1 ou 2)

La composition immunogène selon l'invention comprend un co-tensioactif 1 et un co-tensioactif 2. Ces co-tensioactifs 1 et 2 se situent partiellement dans la phase aqueuse continue et partiellement dans les gouttelettes des phases dispersées 1 et 2.

Les co-tensioactifs 1 et 2 peuvent être identiques ou différents. De préférence, ils sont identiques.De préférence, le co-tensioactif 1 et/ou 2 comporte(nt) au moins une chaîne composée de motifs d'oxyde d'éthylène ou d'oxyde d'éthylène et d'oxyde de propylène. Ils peuvent indépendamment être choisi en particulier parmi les composés conjugués polyéthylèneglycol/phosphatidyl-éthanolamine (PEG-PE), les éthers d'acide gras et de polyéthylèneglycol, les esters d'acide gras et de polyéthylèneglycol et les copolymères blocs d'oxyde d'éthylène et d'oxyde de propylène.

La chaîne polyalcoxylée du co-tensioactif 1 et/ou 2 comprend de préférence de 10 à 200, typiquement de 10 à 150, notamment de 20 à 100 motifs oxyde d'éthylène/oxyde de propylène. De préférence, lorsque la phase dispersée 1 et/ou 2 comprend un tensioactif 1 et/ou 2 cationique et un agent immunostimulant 1 et/ou 2 de type séquence nucléotidique, le co-tensioactif 1 et/ou 2 comprend au moins une chaîne poly(oxyde d'éthylène) comprenant au moins 25 unités d'oxyde d'éthylène.

A titre d'exemple de co-tensioactifs 1 et/ou 2, on peut en particulier citer les composés conjugués à base de polyéthylèneglycol/phosphatidyl-éthanolamine (PEG-PE), les éthers d'acide gras et de polyéthylèneglycol tels que les produits vendus sous les dénominations commerciales Brij® (par exemple Brij® 35, 58, 78 ou 98) par la société ICI Americas Inc., les esters d'acide gras et de polyéthylèneglycol tels que les produits vendus sous les dénominations commerciales Myrj® par la société ICI Americas Inc. (par exemple Myrj® s20, s40 ou s100, anciennement nommés 49, 52 ou 59) et les copolymères blocs d'oxyde d'éthylène et d'oxyde de propylène tels que les produits vendus sous les dénominations commerciales Pluronic® par la société BASF AG (par exemple Pluronic® F68, F127, L64, L61, 10R4, 17R2, 17R4, 25R2 ou 25R4) ou les produits vendus sous la dénomination commerciale Synperonic® par la société Unichema Chemie BV (par exemple Synperonic® PE/F68, PE/L61 ou PE/L64).

Dans la phase dispersée 1, le rapport molaire du tensioactif 1 porteur d'un antigène de formule (I) sur la somme du co-tensioactif 1 et du tensioactif 1 porteur d'un antigène de formule (I) est de 0,01 à 5%, notamment de 0,1 à 3%.

En effet, en dessous de 0,01%, et parfois en dessous de 0,1%, la quantité d'antigène est trop faible pour les applications de la composition explicitées ci-après.

Au-delà de 5%, et parfois au-delà de 3%, la composition immunogène est difficile à préparer et/ou est peu stable. En effet, comme explicité ci-après, la préparation de la composition immunogène nécessite une émulsion prémix 1 comprenant un tensioactif 1 (LI) comprenant un groupe G₁ fonctionnalisable. Pour obtenir une composition immunogène dans laquelle ledit rapport est supérieur à 5%, il est nécessaire de préparer une émulsion prémix 1 dans laquelle le rapport molaire du tensioactif 1 de formule (LI) sur la somme du co-tensioactif 1 et du tensioactif 1 de formule (LI) est supérieur à 5%, ce qui est difficile. En effet, les gouttelettes d'une telle émulsion ont une trop grande densité de surface de groupes fonctionnalisables G₁ et l'émulsion est donc peu stable. De plus, le greffage ultérieur de l'antigène sur l'émulsion prémix 1 est difficile. II n'a en effet pas été possible de formuler des émulsions pour des proportions molaires en tensioactif 1 porteur d'un antigène de formule (I) sur la somme du co-tensioactif 1 et du tensioactif 1 porteur d'un antigène de formule (I) supérieur à 5%.

Généralement, la phase dispersée 1 comporte de 0,1 à 75% en poids, de préférence de 1 à 50% en poids, notamment de 10 à 50% en poids, tout particulièrement de 20 à 45% en poids, de préférence de 30 à 45% en poids de co-tensioactif 1.

Généralement, la phase dispersée 2 comporte de 0,1 à 75% en poids, de préférence de 1 à 50% en poids, notamment de 10 à 50% en poids, tout particulièrement de 20 à 45% en poids, de préférence de 30 à 45% en poids de co-tensioactif 2.

Généralement, la fraction massique de lipide amphiphile 1 par rapport au poids de co-tensioactif 1 est de 0,005 % à 100 %, notamment de 0,01 % à 50 %, de préférence de 0,1 % à 30 %. De même, généralement, la fraction massique de lipide amphiphile 2 par rapport au poids de tensioactif 2 est de 0,005 % à 100 %, notamment de 0,01 % à 50 %, de préférence de 0,1 % à 30 %. En effet, en dessous de 0,005 % et au delà de 100 %, les gouttelettes de la phase dispersée 1 ou 2 ne sont souvent pas suffisamment stables et coalescent en quelques heures et il est souvent difficile d'obtenir des gouttelettes de diamètre inférieur à 250 nm.

Généralement, la composition immunogène ne comporte pas de tensioactifs supplémentaires : les seuls tensioactifs de la composition immunogène sont les lipides amphiphiles 1 et 2, les co-tensioactifs 1 et 2, le tensioactif 1 de formule (I) et l'(es) éventuel(s) tensioactif(s) 1 et/ou 2 cationiques.

Dans un mode de réalisation, une proportion (100 - x₁) %, où 0 < x₁ < 100, du co-tensioactif 1 est greffé de façon covalente à un ligand biologique de ciblage 1.

La phase dispersée 1 comprend toujours une proportion x₁ non nulle de co-tensioactif 1 « libre » (ne comportant pas de ligand biologique de ciblage 1 greffé). Le co-tensioactif 1 est constitué de x₁% de co-tensioactif 1 « libre » et de (100-x₁)% de co-tensioactif 1 sur lequel un ligand biologique de ciblage 1 est greffé. Dans ce mode de réalisation, par « le rapport molaire du tensioactif 1 porteur d'un antigène de formule (I) sur la somme du co-tensioactif 1 et du tensioactif 1 porteur d'un antigène de formule (I) est de 0,01 à 5% », on entend que le rapport molaire du tensioactif 1 porteur d'un antigène de formule (I) sur la somme du co-tensioactif 1 « libre » et du tensioactif 1 porteur d'un antigène de formule (I) est de 0,01 à 5%, c'est-à-dire que le rapport molaire du tensioactif 1 porteur d'un antigène de formule (I) sur la somme de x₁% du co-tensioactif 1, et du tensioactif 1 porteur d'un antigène de formule (I) est de 0,01 à 5%.

De même, une proportion (100 - x₂) %, où 0 < x₂ < 100, du co-tensioactif 2 peut être greffé de façon covalente à un ligand biologique de ciblage 2. x₁ et x₂ peuvent être identiques ou différents.

Typiquement, le ligand biologique de ciblage 1 et/ou 2 a été greffé par liaison covalente au co-tensioactif 1 et/ou 2 tel que défini ci-dessus. Le greffage peut être réalisé avant ou après la formation de l'émulsion 1 ou 2. Le dernier cas peut être préconisé lorsque les réactions chimiques employées sont compatibles avec la stabilité des émulsions, notamment en termes de pH. De préférence, le pH lors de la réaction de greffage est compris entre 5 et 11.

Généralement, ce greffage a été effectué à une extrémité de la ou des chaînes polyalcoxylée(s) du co-tensioactif 1 et/ou 2, et le ligand biologique de ciblage 1 et/ou 2 est ainsi situé à la surface des gouttelettes de la phase dispersée 1 et/ou 2 de la composition immunogène.

### • Agent immunostimulant 1 ou 2 (composant de la phase dispersée 1 ou 2)

La phase dispersée 2 de la composition immunogène comprend un agent immunostimulant 2, qui permet d'améliorer ou d'augmenter l'immunogénicité des antigènes.

Dans un mode de réalisation, la phase dispersée 1 de la composition immunogène comprend un agent immunostimulant 1. Les agents immunostimulants 1 et 2 peuvent être identiques ou différents. De préférence, ils sont identiques.

Dans un autre mode de réalisation, la phase dispersée 1 de la composition immunogène est exempte d'agent immunostimulant.

L'agent immunostimulant 1 et/ou 2 est(sont) notamment choisi(s) parmi un sel d'aluminium, un sel de calcium, magnésium, fer ou zinc, la saponine (e.g. Quil A et ses fragments purifiés tels que QS7 et QS21), un oligonucléotide immunostimulant, un alkyl phosphate glucosamide, l'imiquimod et le résiquimod. De préférence, l'agent immunostimulant 1 et/ou 2 n'est(ne sont) pas un sel d'aluminium. Typiquement, la composition immunogène est alors exempte de sel d'aluminium.

Dans un mode de réalisation préféré, l'agent immunostimulant 1 et/ou 2 est(sont) un ligand de « Toll-like-récepteurs » (TLR). Les TLR sont exprimés par les cellules immunes et en particulier par les cellules « sentinelles » de l'immunité, telles que les cellules dendritiques. La reconnaissance ligand-récepteur conduit à l'activation des cellules dendritiques qui vont initier le déclenchement de réponses immunes spécifiques et contribuer au recrutement de l'ensemble des autres cellules immunitaires plus efficacement. En particulier, l'agent immunostimulant 1 et/ou 2 est un ligand de TLR choisi parmi le monophosphoryl lipid A (MPLA), l'oligodéoxynucléotide (ODN) CpG, l'imiquimod et le resiquimod.

La localisation de l'agent immunostimulant 1 et/ou 2 dans les gouttelettes de la phase dispersée 1 et/ou 2 dépend de la nature de l'agent immunostimulant 1 et/ou 2.

L'agent immunostimulant 1 et/ou 2 peut être lipophile, comme l'imiquimod ou le resiquimod. Il se situe alors dans le cœur des gouttelettes.

L'agent immunostimulant 1 et/ou 2 peut être amphiphile, par exemple le MPLA qui est composé d'une chaîne lipidique et d'une tête sucrée hydrophile. Il se situe alors dans la couronne des gouttelettes.

L'agent immunostimulant 1 et/ou 2 peut être porteur d'au moins un groupe anionique. Il peut notamment s'agir d'une séquence nucléotidique mono ou double brin, comprenant moins de 200 bases pour une séquence nucléotidique mono brin ou moins de 200 paires de base pour une séquence nucléotidique double brin, comme l'oligodéoxynucléotide (ODN) CpG. L'oligodéoxynucléotide (ODN) CpG est une molécule d'ADN synthétique contenant l'enchaînement de nucléotides cytosine (C) puis guanine (G) séparés par une liaison phosphodiester ou phosphorothioate, ces liaisons étant chargées négativement. La phase dispersée 1 et/ou 2 comprend alors généralement un tensioactif 1 et/ou 2 cationique, et éventuellement un lipide fusogène 1 et/ou 2. Ledit agent immunostimulant 1 et/ou 2 porteur d'au moins un groupe anionique est alors maintenu à la surface des gouttelettes de la phase dispersée 1 et/ou 2 grâce aux interactions électrostatiques avec le tensioactif 1 et/ou 2 cationique. Il est donc localisé à la surface des gouttelettes, au niveau de la couronne des gouttelettes, du côté hydrophile de la couronne.

La proportion d'agent immunostimulant 1 dans la phase dispersée 1 dépend de la nature et de l'efficacité de l'agent immunostimulant 1 et de la présence ou non d'un ligand biologique de ciblage 1 dans la phase dispersée 1 de la composition immunogène. Typiquement, la proportion massique d'agent immunostimulant 1 dans la phase dispersée 1 est de 0 à 10%, notamment de 0 à 5%, par exemple de 0 à 3%.

La proportion d'agent immunostimulant 2 dans la phase dispersée 2 dépend de la nature et de l'efficacité de l'agent immunostimulant 2 et de la présence ou non d'un ligand biologique de ciblage 2 dans la phase dispersée 2 de la composition immunogène. Typiquement, la proportion massique d'agent immunostimulant 2 dans la phase dispersée 2 est de 0,01 à 30%, notamment de 0,05 à 10%, par exemple de 0,1 à 5%.

Dans le mode de réalisation dans lequel l'agent immunostimulant 2 est le MPLA, généralement la phase dispersée 2 comporte moins de 40% en poids de co-tensioactif 2 et plus de 0,1% en poids de MPLA. En effet, le MPLA est un ligand de TLR4 situé principalement sur la surface externe des cellules dendritiques. Ainsi, la reconnaissance MPLA-TLR4 doit se faire avant l'ingestion des gouttelettes par la cellule dendritique. Le co-tensioactif comprend au moins une chaine composée de motifs d'oxyde d'alkylène, qui se situe dans la couronne des gouttelettes, et qui peut masquer partiellement le MPLA. Pour assurer une bonne reconnaissance MPLA-TLR, il est donc préférable que la proportion massique en co-tensioactif 2 dans la phase dispersée 2 n'excède pas 40% et que les gouttelettes comprennent une proportion de plus de 0,1% de MPLA. Ainsi, typiquement, lorsque l'agent immunostimulant 2 est le MPLA, la phase dispersée 2 comporte de 0,1 à 40% en poids de co-tensioactif 2 et de 0,1% à 10% en poids de MPLA, de préférence de 1% à 10% en poids de MPLA.

Cette limitation de la proportion massique spécifique du co-tensioactif 2 dans la phase dispersée 2 n'a pas lieu d'être lorsque l'agent immunostimulant 2 est choisi parmi l'oligodéoxynucléotide (ODN) CpG, l'imiquimod et le resiquimod. En effet, L'ODN CpG est un ligand de TLR9, qui est interne aux cellules dendritiques. L'imiquimod est un ligand de TLR7 et le resiquimod un ligand de TLR7/8, qui sont tous deux intracellulaires. Ainsi, pour ces trois agents immunostimulants, la gouttelette de la phase dispersée 2 va dans un premier temps être ingérée/internalisée par la cellule dendritique, puis la reconnaissance agent immunostimulant-récepteur TLR aura lieu en interne, résultant en l'activation de la cellule dendritique

### • Ligand biologique de ciblage 1 ou 2 (composant de la phase dispersée 1 ou 2)

Dans un mode de réalisation, la phase dispersée 1 de la composition immunogène peut comprendre un ligand biologique de ciblage 1, et/ou la phase dispersée 2 de la composition immunogène peut comprendre un ligand biologique de ciblage 2.

On entend par « ligand biologique de ciblage » ou « ligand de ciblage » une molécule permettant d'augmenter la reconnaissance spécifique d'une cellule ou d'un organe que l'on souhaite cibler, notamment d'une cellule immunitaire, comme par exemple les lymphocytes T, les lymphocytes B, les lymphocytes NK, les cellules dendritiques, les macrophages, et de favoriser l'internalisation des gouttelettes par les cellules cibles. Le ligand biologique de ciblage 1 et/ou 2 peut notamment être choisi parmi les anticorps, peptides, saccharides, aptamères, oligonucléotides ou les composés comme l'acide folique.

De préférence, le ligand biologique de ciblage 1 et/ou 2 permet de cibler les cellules dendritiques. Selon ce mode de réalisation, le ligand biologique de ciblage 1 et/ou 2 peut donc être une molécule mannosylée, tel qu'un peptide ou un lipide mannosylé, un polymère de mannose, tel que le mannan, qui peut être reconnu par les récepteurs au mannose présents à la surface des cellules dendritiques, et/ou un anticorps, fragment d'anticorps ou un ligand reconnaissant spécifiquement les cellules dendritiques tel que anti-DC-SIGN anti-DEC-205, anti-CD-207.

Les ligands biologiques de ciblage 1 et 2 peuvent être identiques ou différents. De préférence, ils sont identiques.

Le ligand biologique de ciblage 1 peut être greffé ou non sur le co-tensioactif 1, c'est à dire :
- sous forme libre dans les gouttelettes, c'est-à-dire qu'il est encapsulé dans les gouttelettes, soit dans la couronne s'il a un caractère amphiphile, soit dans le cœur s'il a un caractère lipophile, et/ou
- sous forme greffée de façon covalente au co-tensioactif 1, comme explicité ci-dessus.

De même, le ligand biologique de ciblage 2 peut être greffé ou non sur le co-tensioactif 2.

La proportion de ligand biologique de ciblage 1 (et/ou 2) dans la phase dispersée 1 (et/ou 2) dépend de la nature et de l'efficacité du ligand biologique de ciblage 1 (et/ou 2). Cette proportion peut facilement être déterminée par l'homme du métier.

### • Agent d'intérêt 1 ou 2 (composant de la phase dispersée 1 ou 2)

Dans un mode de réalisation, la phase dispersée 1 de la composition immunogène peut comprendre un ou plusieurs agent(s) d'intérêt 1 et/ou la phase dispersée 2 de la composition immunogène peut comprendre un ou plusieurs agent(s) d'intérêt 2. Les agents d'intérêt 1 et 2 peuvent être identiques ou différents.

L'agent d'intérêt 1 et/ou 2 peut(vent) être de nature très variée. Ainsi, l'agent d'intérêt 1 et/ou 2 peut(vent) être :
- un agent optique tel que un colorant, un chromophore, un fluorophore, par exemple le perchlorate 1,1'-dioctadecyl 3,3,3',3'-tetramethylindodicarbocyanine (DiD), le iodure de 1,1'-dioctadecyl 3,3,3',3'-tetramethylindotricarbocyanine (DiR), le vert d'indocyanine (ICG), ou encore des composants ayant des propriétés optoélectronique, tels que les saturants ou les absorbants optiques,
- un agent physique, tel qu'un isotope radioactif ou un photo-sensibilisateur,
- un agent d'imagerie, notamment pour l'IRM (imagerie par résonance magnétique), le PET (en anglais Positron Emission Tomography), le SPECT (Single Photon Emission Computed Tomography), l'échographie ultrasonore, la radiographie, la tomographie X et l'imagerie optique (fluorescence, bioluminescence, diffusion...). Ces agents peuvent permettre de suivre la position des gouttelettes (et donc de l'antigène) après administration de la composition immunogène au patient, et/ou
- un agent thérapeutique, notamment choisi parmi un antibiotique, un anticancéreux, un antiviral, un antiparasitaire, une protéine thérapeutique (telle qu'une cytokine ou une chimiokine) et un mélange de ceux-ci.

Les quantités d'agent d'intérêt dépendent de l'application visée ainsi que de la nature des agents.

Lorsque la composition immunogène selon l'invention comprend un agent d'intérêt, elle contient le plus souvent une quantité de 0,001 à 30% en poids, de préférence 0,01 à 20% en poids, et encore préférée 0,1 à 10% en poids d'agent d'intérêt.

Si l'agent d'intérêt est hydrophile, il est alors situé dans la phase aqueuse continue de la composition immunogène. S'il est amphiphile ou lipophile, il est alors encapsulé dans les gouttelettes formant la phase dispersée 1 et/ou la phase dispersée 2 de la composition immunogène ou se situe dans la couronne des gouttelettes, selon son affinité lipophile ou amphiphile.

### • Tensioactif 1 ou 2 cationique (composant de la phase dispersée 1 ou 2)

La composition immunogène selon l'invention peut comprendre dans sa phase dispersée 1 et/ou 2 un tensioactif 1 et/ ou 2 cationique. Les tensioactifs 1 et 2 cationiques peuvent être identiques ou différents. Ils sont notamment indépendamment choisi parmi :
- le *N*[1-(2,3-dioléyloxy)propyl]-*N,N,N*-triméthylammonium (DOTMA),
- le 1,2-dioleyl-3-trimethylamonium-propane (DOTAP),
- le *N*-(2-hydroxyethyl)-*N,N*-dimethyl-2,3-bis(tetradecyloxy-1-propananium) (DMRIE),
- le 1-[2-(oleoyloxy)ethyl]-2-oleyl-3-(2-hydroxyethyl)imidazolinium (DOTIM), et
- le dioctadecylamidoglycylspermine (DOGS) (sous forme protonée),
et étant de préférence le 1,2-dioleyl-3-trimethylamonium-propane (DOTAP).

Le tensioactif 1 et/ou 2 cationique se situe dans la couronne des gouttelettes de la phase dispersée 1 et/ou 2. Le tensioactif 1 et/ou 2 cationique permet notamment d'améliorer, ou d'accélérer la capture des gouttelettes par les cellules immunitaires. Le tensioactif 1 et/ou 2 cationique a également pour effet de créer au site d'administration une légère inflammation locale qui attire les cellules immunitaires sur le site d'administration des gouttelettes, permettant ainsi une accélération de la capture des gouttelettes par les cellules immunitaires (Doroud et al. / Journal of Controlled Release 153 (2011) 154-162).

Le mode de réalisation dans lequel la phase dispersée 2 comprend un tensioactif 2 cationique est particulièrement adapté lorsque l'agent immunostimulant 2 est porteur d'au moins un groupe anionique, par exemple il s'agit d'une séquence nucléotidique mono ou double brin, comprenant moins de 200 bases pour une séquence nucléotidique mono brin ou moins de 200 paires de base pour une séquence nucléotidique double brin par exemple l'ODN CpG. En effet, le tensioactif 2 cationique permet de maintenir un tel agent immunostimulant à la surface des gouttelettes de la phase dispersée 2 grâce aux interactions électrostatiques. L'agent immunostimulant 2 est alors localisé à la surface des gouttelettes, au niveau de la couronne des gouttelettes, du côté hydrophile de la couronne. De préférence, lorsque la phase dispersée 2 comprend un tensioactif 2 cationique et un agent immunostimulant 2 de type séquence nucléotidique, les proportions de composants décrits dans la demande WO 2014/032953 sont respectées et la phase dispersée 2 comprend :
- au moins 5% molaire de lipide amphiphile 2,
- de 15 à 70% molaire du tensioactif cationique 2, et
- de 10% à 55% molaire du co-tensioactif 2.

Généralement, la phase dispersée 1 comporte de 0 à 50% en poids, de préférence de 0 à 40% en poids de tensioactif 1 cationique. Typiquement, la phase dispersée 1 est exempte de tensioactif 1 cationique.

Généralement, la phase dispersée 2 comporte de 0 à 50% en poids, de préférence de 0 à 40% en poids de tensioactif 2 cationique. Ces proportions sont particulièrement adaptées pour améliorer, ou accélérer la capture des gouttelettes par les cellules immunitaires.

Lorsque la phase dispersée 1 et/ou 2 comprend un tensioactif cationique 1 et/ou 2, elle peut également comprendre un lipide fusogène 1 et/ou 2 (« helper lipid » en anglais). Ce lipide fusogène est susceptible de faciliter la libération cytosolique par déstabilisation de la membrane endosomale, et est dit lipide « helper ». De préférence, le lipide fusogène 1 et/ou 2 est le dioléylphosphatidyléthanolamine (DOPE).

Ce lipide permet de favoriser l'échappement endosomal des gouttelettes de la composition immunogène selon l'invention, et notamment de l'agent immunostimulant 1 et/ou 2 porteur de groupe anionique. Le lipide fusogène 1 et/ou 2 se situe dans la couronne des gouttelettes de la phase dispersée 1 et/ou 2.

### • Huile 1 ou 2 (composant de la phase dispersée 1 ou 2)

La phase dispersée 1 de la composition immunogène peut également comporter une ou plusieurs huiles 1. La phase dispersée 2 de la composition immunogène peut également comporter une ou plusieurs huiles 2.

L'huile 1 et 2 peuvent être identiques ou différents, de préférence elles sont identiques.

Les huiles utilisées présentent de préférence une balance hydrophile-lipophile (HLB) inférieure à 10 et encore plus préférentiellement de 3 à 9. Avantageusement, les huiles sont utilisées sans modification chimique ou physique préalablement à la formation de la composition immunogène.

Selon les applications envisagées, les huiles peuvent être choisies parmi les huiles biocompatibles, et en particulier parmi les huiles d'origine naturelle (végétale ou animale) ou synthétique. Parmi de telles huiles, on peut notamment citer les huiles d'origine naturelle végétale parmi lesquelles figurent notamment les huiles de soja, de lin, de palme, d'arachide, d'olives, de sésame, de pépin de raisins et de tournesol ; les huiles synthétiques parmi lesquelles figurent notamment les triglycérides, diglycérides et les mono glycérides ; et les huiles issues de graisses animales. Ces huiles peuvent être de premières expressions, raffinées ou inter-estérifiées.

De préférence, l'huile 1 et/ou 2 est liquide a 20°C.

L'huile préférée est l'huile de soja.

Généralement, si présente, l'huile 1 est contenue dans la phase dispersée 1 dans une proportion allant de 1 à 80% en poids, de préférence de 5 à 50 % en poids et tout particulièrement de 10 à 30% en poids par rapport au poids total de la phase dispersée 1. De même, généralement, si présente, l'huile 2 est contenue dans la phase dispersée 1 dans une proportion allant de 1 à 80% en poids, de préférence de 5 à 50 % en poids et tout particulièrement de 10 à 30% en poids par rapport au poids total de la phase dispersée 2.

### • Phase aqueuse

La phase aqueuse continue de la composition immunogène selon l'invention est de préférence constituée d'eau et/ou d'un tampon tel qu'un tampon phosphate comme par exemple du PBS ("Phosphate Buffer Saline") ou d'une solution saline, notamment de chlorure de sodium. Généralement, le pH de la phase aqueuse est de l'ordre du pH physiologique.

Selon un mode de réalisation, la phase continue comporte également un agent épaississant tel que du glycérol, un saccharide, oligosaccharide ou polysaccharide, une gomme ou encore une protéine, de préférence du glycérol. En effet, l'utilisation d'une phase continue de viscosité plus élevée facilite l'émulsification et permet de ce fait de réduire le temps de sonication.

La phase aqueuse comporte avantageusement de 0 à 50% en poids, de préférence de 1 à 30% en poids et tout particulièrement de 5 à 20% en poids d'agent épaississant.

Bien entendu, la phase aqueuse peut contenir en outre d'autres additifs tels que des colorants, stabilisants et conservateurs en quantité appropriée.

### • Composition immunogène sous forme de gel

Dans un mode de réalisation, la viscosité de la composition immunogène est supérieure à 1 poise (0,1 Pa.s) à 25°C.

Dans ce mode de réalisation, la composition immunogène se présente sous forme de « gel ». On entend par le terme « gel » habituellement un système biphasique solide-liquide thermodynamiquement stable, constitué d'un double réseau interpénétré continu tridimensionnel, l'un solide et le second liquide. Un tel gel est un système biphasique liquide-solide dont le réseau solide retient une phase liquide. Bien que les gels puissent être considérés comme solides, ils présentent des propriétés propres aux solides (rigidité structurelle, élasticité à la déformation) comme aux liquides (pression de vapeur, de compressibilité et conductivité électrique). Les interactions entre les gouttelettes peuvent être dues à des forces de Van der Waals, des liaisons électrostatiques, des liaisons hydrogènes ou des liaisons covalentes.

Dans le cas d'une nanoémulsion sous forme de gel, le réseau tridimensionnel est formé par les gouttelettes, les interstices entre gouttelettes étant remplis de phase continue. Les liaisons entre les unités du réseau, à savoir les gouttelettes, reposent principalement sur des interactions électrostatiques (paires d'ions). Ces interactions électrostatiques existent principalement entre les séquences nucléotidiques et les tensioactifs 1 et/ou 2 cationiques de gouttelettes adjacentes.

Une nanoémulsion sous forme de gel montre donc une résistance à la pression et est capable de maintenir une forme définie, ce qui peut être avantageux selon la forme et/ou la voie d'administration souhaitée.

Pour mettre en évidence que la composition immunogène est sous forme de gel, on peut réaliser des études rhéologiques permettant d'évaluer les propriétés viscoélastiques, et/ou des études plus structurelles montrant les liaisons entre les gouttelettes formant le réseau tridimensionnel (diffraction aux rayons X, neutrons...). En effet, une nanoémulsion sous forme de gel possède une viscosité et un coefficient d'élasticité plus important qu'une nanoémulsion liquide. La nanoémulsion sous forme de gel peut, en fonction de la concentration de gouttelettes et donc de la fraction massique en phase dispersée 1 et en phase dispersée 2, se trouver à l'état de liquide visqueux, de solide viscoélastique ou de solide élastique. Par rapport à la phase dispersante aqueuse, dont la viscosité est proche de celle de l'eau (1 mPa.s à 25°C), la nanoémulsion est considérée comme un liquide visqueux lorsque sa viscosité est 10 fois plus élevée que celle de l'eau, soit > 10 mPa.s à 25°C. Par ailleurs, lorsque l'on procède à la mesure rhéologique des modules de G' (module de conservation en cisaillement) et G" (module de perte en cisaillement), on considère que la nanoémulsion est sous forme d'un liquide visqueux lorsque G" >G'. Lorsque G' devient proche de G", la nanoémulsion est à l'état de solide viscoélastique. Lorsque G" < G', on est à l'état de solide élastique. Dans ce mode de réalisation, la nanoémulsion se présente de préférence à l'état liquide visqueux, de solide viscoélastique, ou solide. La viscosité et le coefficient d'élasticité peuvent être mesurés par un rhéomètre cône-plan ou par un rhéomètre Couette. La viscosité d'une nanoémulsion liquide est généralement inférieure à 1 poise, voire même souvent inférieure à 0,01 poise. La nanoémulsion utilisée dans ce mode de réalisation de l'invention a généralement une viscosité supérieure à 1 poise, et pourra avoir une viscosité allant jusqu'à celle d'un solide (plus de 1000 poises). Les études structurelles, notamment les diffractions aux rayons X ou aux neutrons, permettent également de différencier l'organisation d'une nanoémulsion liquide, de l'organisation d'une nanoémulsion sous forme de gel. En effet, les pics du diffractogramme obtenu pour une nanoémulsion liquide sont caractéristiques de la structure des gouttelettes de phase dispersée 1 et de phase dispersée 2 (grand angles de diffraction caractéristiques de distances courtes), alors que les pics du diffractogramme d'une nanoémulsion sous forme de gel sont caractéristiques non seulement de la structure des gouttelettes (grand angles de diffraction caractéristiques de distances courtes) mais aussi de l'organisation de ces gouttelettes en réseau tridimensionnel (faibles angles de diffraction caractéristiques de distances plus grandes).

La composition immunogène sous forme de gel est avantageusement sous forme de gel dispersible, c'est-à-dire que les gouttelettes formant le réseau tridimensionnel peuvent être relarguées dans la phase continue sous certaines conditions par « dégélification » du système gel, également dénommée « désagrégation » dans la présente demande. La désagrégation est observée par ajout de phase continue au gel, par mise en contact avec les fluides physiologiques lors de l'administration de la nanoémulsion ou par augmentation de la température. En effet, ajouter de la phase continue entraîne une différence de pression osmotique entre l'intérieur du gel et la phase continue. Le système tendra donc à diminuer, jusqu'à annuler, cette différence de pression osmotique en libérant les gouttelettes dans l'excès de phase continue, jusqu'à obtenir une concentration en gouttelettes homogène dans l'ensemble du volume de phase continue. La mise en contact avec les fluides physiologiques peut aussi induire une réaction chimique (exemple : clivage de liaisons covalentes de type pont disulfure ou liaison peptidique, et ainsi libérer les gouttelettes). De même augmenter suffisamment la température du système revient à donner aux différentes gouttelettes une énergie thermique supérieure aux énergies mises en jeu dans les liaisons, par exemple les liaisons hydrogène, et ainsi à rompre ces liaisons et libérer les gouttelettes du réseau tridimensionnel. Ces températures dépendent de la composition du gel et plus particulièrement de la taille des gouttelettes et de la longueur des chaînes polyalcoxylées des co-tensioactifs 1 et 2. La désagrégation de la nanoémulsion sous forme de gel peut être suivie par diffraction aux rayons X, par calorimétrie différentielle à balayage (DSC) ou par résonance magnétique nucléaire (RMN).

### • Localisation des composants des gouttelettes

Les gouttelettes de la phase dispersée 1 de la composition immunogène selon l'invention s'organisent sous forme de cœur - couronne, où :
- le cœur comprend :
   - le lipide solubilisant 1,
   - l'éventuelle huile 1,
   - l'éventuel agent d'intérêt 1 lipophile,
   - l'éventuel agent immunostimulant 1 lipophile,
- la couronne comprend :
   - le lipide amphiphile 1,
   - le tensioactif 1 porteur d'un antigène de formule (I),
   - l'éventuel tensioactif 1 cationique,
   - l'éventuel agent immunostimulant 1 porteur d'au moins un groupe anionique,
   - le co-tensioactif 1 (dont une proportion (1-x₁)% est éventuellement greffé avec un ligand de ciblage 1),
   - l'éventuel ligand biologique de ciblage 1 amphiphile,
   - l'éventuel agent immunostimulant 1 amphiphile,
   - l'éventuel agent d'intérêt 1 amphiphile.

Les gouttelettes de la phase dispersée 2 de la composition immunogène selon l'invention s'organisent sous forme de cœur - couronne, où :
- le cœur comprend :
   - le lipide solubilisant 2,
   - l'éventuelle huile 2,
   - l'éventuel agent d'intérêt 2 lipophile,
   - l'éventuel agent immunostimulant 2 lipophile,
- la couronne comprend :
   - le lipide amphiphile 2,
   - l'éventuel tensioactif 2 cationique,
   - l'éventuel agent immunostimulant 2 porteur d'au moins un groupe anionique
   - le co-tensioactif 2 (dont une proportion (1-x₂)% est éventuellement greffé avec un ligand de ciblage 2),
   - l'éventuel ligand biologique de ciblage 2 amphiphile,
   - l'éventuel agent immunostimulant 2 amphiphile,
   - l'éventuel agent d'intérêt 2 amphiphile,
sous réserve que les gouttelettes de la phase dispersée 2 comprennent au moins un agent immunostimulant 2 (par exemple elles peuvent contenir un agent immunostimulant 2 lipophile, mais pas d'agent immunostimulant 2 porteur d'au moins un groupe anionique ou amphiphile).

### • Modes de réalisation préférés

Comme détaillé ci-dessus, l'agent immunostimulant 2 est de préférence un ligand de « Toll-like-récepteurs » (TLR), typiquement choisi parmi le monophosphoryl lipid A (MPLA), l'oligodéoxynucleotide (ODN) CpG, l'imiquimod et le resiquimod. Les trois modes de réalisation suivants sont donc préférés.

Dans un premier mode de réalisation, la composition immunogène comprend une phase aqueuse continue et au moins deux phases dispersées 1 et 2 sous forme de gouttelettes, où :
- la phase dispersée 1 comprend (ou consiste en) :
   - un lipide amphiphile 1,
   - un lipide solubilisant 1 comprenant au moins un glycéride d'acide gras,
   - un co-tensioactif 1 comprenant au moins une chaine composée de motifs d'oxyde d'alkylène,
   - un tensioactif 1 porteur d'un antigène de formule (I) suivante :

      (Li-X₁-H₁-Y₁)ᵥ-G-Z₁-Ag (I),

      dans laquelle :
      - L₁ représente un groupe lipophile,
      - X₁, Y₁, Z₁ et G représentent indépendamment un groupe de liaison,
      - H₁ représente un groupe hydrophile comprenant une chaîne polyalcoxylée,
      - v est un nombre entier de 1 à 8, et
      - Ag représente un antigène,
   - éventuellement une huile 1,
   - éventuellement un ligand biologique de ciblage 1,
   - éventuellement un agent d'intérêt 1,
   dans laquelle le rapport molaire du tensioactif 1 porteur d'un antigène de formule (I) sur la somme du co-tensioactif 1 et du tensioactif 1 porteur d'un antigène de formule (I) est de 0,01 % à 5%, et
   - la phase dispersée 2 comprend (ou consiste en) :
      - un lipide amphiphile 2,
      - un lipide solubilisant 2 comprenant au moins un glycéride d'acide gras,
      - un co-tensioactif 2 comprenant au moins une chaine composée de motifs d'oxyde d'alkylène,
      - du l'imiquimod ou du resiquimod,
      - éventuellement une huile 2,
      - éventuellement un ligand biologique de ciblage 2,
      - éventuellement un agent d'intérêt 2,
sous réserve que la phase dispersée 2 soit exempte de tensioactif porteur d'un antigène de formule (I).

De préférence, la phase dispersée 1 est exempte de tensioactif cationique 1 et la phase dispersée 2 est exempte de tensioactif cationique 2.

Dans un deuxième mode de réalisation, la composition immunogène comprend une phase aqueuse continue et au moins deux phases dispersées 1 et 2 sous forme de gouttelettes, où :
- la phase dispersée 1 comprend (ou consiste en) :
   - un lipide amphiphile 1,
   - un lipide solubilisant 1 comprenant au moins un glycéride d'acide gras,
   - un co-tensioactif 1 comprenant au moins une chaine composée de motifs d'oxyde d'alkylène,
   - un tensioactif 1 porteur d'un antigène de formule (I) suivante :

      (Li-X₁-H₁-Y₁)ᵥ-G-Z₁-Ag (I),

      dans laquelle :
      - L₁ représente un groupe lipophile,
      - X₁, Y₁, Z₁ et G représentent indépendamment un groupe de liaison,
      - H₁ représente un groupe hydrophile comprenant une chaîne polyalcoxylée,
      - v est un nombre entier de 1 à 8, et
      - Ag représente un antigène,
   - éventuellement une huile 1,
   - éventuellement un ligand biologique de ciblage 1,
   - éventuellement un agent d'intérêt 1,
   dans laquelle le rapport molaire du tensioactif 1 porteur d'un antigène de formule (I) sur la somme du co-tensioactif 1 et du tensioactif 1 porteur d'un antigène de formule (I) est de 0,01 % à 5%, et
   - la phase dispersée 2 comprend (ou consiste en) :
      - un lipide amphiphile 2,
      - un lipide solubilisant 2 comprenant au moins un glycéride d'acide gras,
      - de 0,1 à 40% en poids de co-tensioactif 2 comprenant au moins une chaine composée de motifs d'oxyde d'alkylène par rapport au poids de la phase dispersée 2,
      - de 0,1 à 10% en poids de MPLA par rapport au poids de la phase dispersée 2,
      - éventuellement une huile 2,
      - éventuellement un ligand biologique de ciblage 2,
      - éventuellement un agent d'intérêt 2,
sous réserve que la phase dispersée 2 soit exempte de tensioactif porteur d'un antigène de formule (I).

De préférence, la phase dispersée 1 est exempte de tensioactif cationique 1 et la phase dispersée 2 est exempte de tensioactif cationique 2.

Dans un troisième mode de réalisation, la composition immunogène comprend une phase aqueuse continue et au moins deux phases dispersées 1 et 2 sous forme de gouttelettes, où :
- la phase dispersée 1 comprend (ou consiste en) :
   - un lipide amphiphile 1,
   - un lipide solubilisant 1 comprenant au moins un glycéride d'acide gras,
   - un co-tensioactif 1 comprenant au moins une chaine composée de motifs d'oxyde d'alkylène,
   - un tensioactif 1 porteur d'un antigène de formule (I) suivante :

      (Li-X₁-H₁-Y₁)ᵥ-G-Z₁-Ag (I),

      dans laquelle :
      - L₁ représente un groupe lipophile,
      - X₁, Y₁, Z₁ et G représentent indépendamment un groupe de liaison,
      - H₁ représente un groupe hydrophile comprenant une chaîne polyalcoxylée,
      - v est un nombre entier de 1 à 8, et
      - Ag représente un antigène,
   - éventuellement une huile 1,
   - éventuellement un ligand biologique de ciblage 1,
   - éventuellement un agent d'intérêt 1,
dans laquelle le rapport molaire du tensioactif 1 porteur d'un antigène de formule (I) sur la somme du co-tensioactif 1 et du tensioactif 1 porteur d'un antigène de formule (I) est de 0,01% à 5%, et
- la phase dispersée 2 comprend (ou consiste en) :
   - un lipide amphiphile 2,
   - un lipide solubilisant 2 comprenant au moins un glycéride d'acide gras,
   - un co-tensioactif 2 comprenant au moins une chaîne poly(oxyde d'éthylène) comprenant au moins 25 unités d'oxyde d'éthylène,
   - de l'ODN CpG,
   - un tensioactif cationique 2,
   - éventuellement un lipide fusogène 2,
   - éventuellement une huile 2,
   - éventuellement un ligand biologique de ciblage 2,
   - éventuellement un agent d'intérêt 2,
sous réserve que la phase dispersée 2 soit exempte de tensioactif porteur d'un antigène de formule (I).
De préférence, la phase dispersée 1 est exempte de tensioactif cationique 1.

Dans ces trois modes de réalisation, de préférence, la phase dispersée 1 est exempte d'agent immunostimulant 1.

### • Autres propriétés de la composition immunogène

Grâce à sa formulation, la composition immunogène selon l'invention est stable et présente une excellente stabilité au stockage (supérieure à 5 mois voire à 8 mois). En particulier, du fait que l'antigène est lié de façon covalente aux gouttelettes, il ne migre pas dans la phase aqueuse continue, au contraire de compositions immunogènes dans lesquelles l'antigène est simplement encapsulé.

L'antigène greffé aux gouttelettes est également stabilisé par la composition immunogène, du fait que le co-tensioactif 1 et lipide amphiphile 1 le protègent.

Les chaînes polyalcoxylées des co-tensioactifs 1 et 2 et du tensioactif 1 de formule (I), hydratées et non chargées, couvrant la surface des gouttelettes, écrantent les charges apportées par les lipides amphiphiles 1 et 2 à la surface solide des gouttelettes. On se trouve donc dans le cas d'une stabilisation stérique des gouttelettes, et non une stabilisation électrostatique.

### [Procédé de préparation]

Selon un deuxième objet, l'invention concerne un procédé de préparation de la composition immunogène définie ci-dessus, comprenant le mélange d'une émulsion 1 comprenant une phase aqueuse continue et au moins une phase dispersée 1 telles que définies ci-dessus, et d'une émulsion 2 comprenant une phase aqueuse continue et au moins une phase dispersée 2 telles que définies ci-dessus.

Généralement, ce mélange est réalisé avec des proportions d'émulsions 1 et 2 telles que la composition immunogène comprend un ratio massique d'antigène Ag par rapport à l'agent immunostimulant 2 de 0,01/100 à 100/0,01, notamment de 0,1/10 à 10/0,1, de préférence de 1/5 à 5/1, en particulier de 1/2 à 2/1, par exemple de l'ordre de 1/1. Ce ratio, et donc les proportions d'émulsions 1 et 2 à mélanger, peuvent facilement être déterminés dans la mesure où la proportion massique d'antigène Ag dans l'émulsion 1 et la proportion massique d'agent immunostimulant 2 dans l'émulsion 2 sont connues.

Le procédé peut comprendre des étapes préalables de préparation de l'émulsion 1 et/ou de l'émulsion 2. Ainsi, dans un mode de réalisation, le procédé comprend les étapes suivantes :
1) préparation d'une émulsion 1 comprenant une phase aqueuse continue et au moins une phase dispersée 1 telles que définies ci-dessus,
2) préparation d'une émulsion 2 comprenant une phase aqueuse continue et au moins une phase dispersée 2 telles que définies ci-dessus, puis
3) mélange de l'émulsion 1 et de l'émulsion 2.

Les étapes 1) et 2) peuvent être réalisées dans n'importe quel ordre.

### • Etape 1) : préparation de l'émulsion 1

Dans un mode de réalisation, le procédé comprend une étape 1) de préparation de l'émulsion 1, comprenant typiquement la mise en contact :
- d'une émulsion prémix 1 comprenant une phase aqueuse continue et une phase dispersée 1 sous forme de gouttelettes, comprenant un lipide amphiphile 1, un lipide solubilisant 1 comprenant au moins un glycéride d'acide gras, un co-tensioactif 1 comprenant au moins une chaine composée de motifs d'oxyde d'alkylène et un tensioactif 1 de formule (LI) suivante :

   L₁-X₁-H₁-Y₁-G₁ (LI),

   dans laquelle le rapport molaire du tensioactif 1 de formule (LI) sur la somme du co-tensioactif 1 et du tensioactif 1 de formule (LI) est de 0,01 % à 5%,
- avec un composé 1 de formule (LII) suivante :

   G₂-Z₁-Ag (LII)
où L₁, X₁, H₁, Y₁, Z₁ et Ag sont tels que définis ci-dessus, et G₁ et G₂ sont des groupes susceptibles de réagir ensemble pour former le groupe G tel que défini ci-dessus,
dans des conditions permettant la réaction du tensioactif 1 de formules (LI) avec le composé 1 de formule (LII) pour former le tensioactif 1 porteur d'un antigène de formule (I) tel que défini ci-dessus.

Lorsque le groupe G comprend un seul groupe G', les groupes G₁ et G₂ sont typiquement des groupes susceptibles de réagir l'un avec l'autre pour former le groupe G.

Lorsque le groupe G comprend plusieurs groupes G', on met généralement l'émulsion prémix 1 et le composé 1 de formule (LII) en contact avec un composé susceptible de réagir avec eux pour former le groupe G. Ce composé comprend typiquement au moins v fonctions G'₁ susceptibles de réagir avec le groupe G₁ et une fonction G'₂ susceptibles de réagir avec le groupe G₂.

Ainsi, dans le mode de réalisation dans lequel le groupe G répond à la formule -G'-Y₃-G'- définie ci-dessus, le procédé de préparation de l'émulsion 1 comprend typiquement la mise en contact :
- d'une émulsion prémix 1 telle que définie ci-dessus,
- et du composé 1 de formule (LII) tel que définie ci-dessus,
- avec un composé de formule G'₁-Y₃-G'₂ dans laquelle Y₃ est tel que défini ci-dessus, G'₁ est un groupe susceptible de réagir avec G₁ pour former le premier groupe G' tel que défini ci-dessus et G'₂ est un groupe susceptible de réagir avec G₂ pour former le second groupe G' tel que défini ci-dessus (de nature identique ou différente du premier groupe G'),
dans des conditions permettant la réaction du tensioactifs 1 de formule (LI) et du composé 1 de formule (LII) avec le composé de formule G'₁-Y₃-G'₂ pour former le tensioactif 1 porteur d'un antigène de formule (I) dans lequel le groupe G répond à la formule -G'-Y₃-G'- définie ci-dessus.

### • Formation du tensioactif 1 porteur d'antigène de formule (I) par réaction entre le tensioactif 1 de formule (LI) et le composé de formule (LII)

L'émulsion prémix 1 comprend un tensioactif 1 de formule (LI) comprenant un groupe G₁ fonctionnalisable, qui se situe en surface des gouttelettes.

Avantageusement, une même émulsion prémix 1 peut être utilisée pour greffer des anticorps de natures différentes, dès lors que le composé 1 de formule (LII) comprend un groupe G₂ susceptible de réagir avec le groupe G₁ de l'émulsion prémix 1. II n'est pas nécessaire d'adapter les composants de l'émulsion prémix 1 et les conditions de greffage pour chaque antigène différent utilisé. Ainsi, le procédé de préparation de l'émulsion 1 peut être mis en œuvre de façon industrielle et être automatisé.

La formation du tensioactif 1 porteur d'antigène de formule (I) par réaction entre le tensioactif 1 de formule (LI) et le composé 1 de formule (LII) permet le greffage par liaison covalente de l'antigène Ag aux gouttelettes de l'émulsion prémix 1 comprenant le tensioactif 1 fonctionnalisable de formule (LI). L'antigène est lié aux gouttelettes de l'émulsion 1 par une liaison covalente. Le greffage de l'anticorps aux gouttelettes de l'émulsion prémix 1 est avantageusement indépendant du caractère hydrophile, amphiphile ou lipophile de l'antigène. N'importe quel type d'antigène peut donc être greffé, ce qui est un avantage par rapport aux compositions immunogènes à base d'émulsion et de nanoparticules lipidiques solides de l'art antérieur dans lesquelles l'antigène est encapsulé dans les gouttelettes (l'encapsulation n'étant possible que pour des antigènes amphiphiles ou lipophiles).

Au vu de ses connaissances générales en chimie, l'homme du métier est à même de choisir la nature des groupes G'₁, G'₂, Y₃, G₁ et G₂ à utiliser pour former le groupe G et les conditions permettant la réaction. Les réactions de chimie organique usuelles peuvent être suivies, notamment celles décrites dans « Comprehensive Organic Transformations: A Guide to Functional Group Préparations » de Richard C. Larock édité par John Wiley & Sons Inc, et les références qui y sont citées. Ainsi, les exemples de groupes G₁ et G₂ ci-dessous sont cités à titre illustratif et non limitatif.

Typiquement, lorsque le groupe G est constitué d'un groupe G', le groupe G₁ du tensioactif 1 de formule (LI) et le groupe G₂ du composé 1 de formule (LII) peuvent par exemple être choisis comme suit :
- l'un des G₁ et G₂ représente un thiol (-SH) et l'autre G₁ ou G₂ représente :
   - soit un maléimide, un tensioactif 1 de formule (I) dans lequel G comprend un groupe G' représentant un groupe de formule (XIV) où A₁₀₂ représente N étant alors formé, la mise en contact de l'émulsion prémix 1 et du composé 1 de formule (LII) étant de préférence réalisée à une température de 0°C à 15°C, notamment de 0 à 10°C, de préférence de 0 à 5°C,
   - soit une vinylsulfone, un tensioactif 1 de formule (I) dans lequel G comprend un groupe G' représentant un groupe de formule (XVI) étant alors formé,
   - soit un groupe -S-S-pyridinyle ou -SH, un tensioactif 1 de formule (I) dans lequel G comprend un groupe G' représentant un groupe de formule (XV) étant alors formé,
- l'un des G₁ et G₂ représente un hydroxyle et l'autre G₁ ou G₂ représente -COOH ou un ester activé, un tensioactif 1 de formule (I) dans lequel G comprend un groupe G' représentant un groupe de formule (XXIII) étant alors formé,
- l'un des G₁ et G₂ représente une amine -NH₂ et l'autre G₁ ou G₂ représente -COOH ou un ester activé, un tensioactif 1 de formule (I) dans lequel G comprend un groupe G' représentant un groupe de formule (XI) étant alors formé,
- l'un des G₁ et G₂ représente un hydroxyle et l'autre G₁ ou G₂ représente un carbonate activé ou un carbamate activé, un tensioactif 1 de formule (I) dans lequel G comprend un groupe G' représentant un groupe de formule (XIX) étant alors formé,
- l'un des G₁ et G₂ représente une amine -NH₂ et l'autre G₁ ou G₂ représente un carbonate activé ou un carbamate activé, un tensioactif 1 de formule (I) dans lequel G comprend un groupe G' représentant un groupe de formule (XII) étant alors formé,
- l'un des G₁ et G₂ représente une amine -NH₂ et l'autre G₁ ou G₂ représente un aldéhyde -CHO, un tensioactif 1 de formule (I) dans lequel G comprend un groupe G' représentant un groupe de formule (XXI) étant alors formé,
- l'un des G₁ et G₂ représente un hydrazide de formule -(C=O)-NH-NH₂ et l'autre G₁ ou G₂ représente un groupe -(C=O)-R₁₀₂, un tensioactif 1 de formule (I) dans lequel G comprend un groupe G' représentant un groupe de formule (XIII) étant alors formé,
- l'un des G₁ et G₂ représente un alcyne et l'autre G₁ ou G₂ représente un azide, un tensioactif 1 de formule (I) dans lequel G comprend un groupe G' représentant un groupe de formule (XVIII) étant alors formé,
- l'un des G₁ et G₂ représente un cyclooctyne et l'autre G₁ ou G₂ représente un azide, un tensioactif 1 de formule (I) dans lequel G comprend un groupe G' représentant un groupe de formule (XVIII') étant alors formé,
- l'un des G₁ et G₂ représente un furane et l'autre G₁ ou G₂ représente un maléimide, un tensioactif 1 de formule (I) dans lequel G comprend un groupe G' représentant un groupe de formule (XVII) étant alors formé,
- l'un des G₁ et G₂ représente un aldéhyde et l'autre G₁ ou G₂ représente une amine, un tensioactif 1 de formule (I) dans lequel G comprend un groupe G' représentant un groupe de formule (XXI) étant alors formé,
- l'un des G₁ et G₂ représente un phosphate de formule -O-P(=O)(OH)₂ et l'autre G₁ ou G₂ représente un hydroxyle, un tensioactif 1 de formule (I) dans lequel G comprend un groupe G' représentant un groupe de formule (XXII) étant alors formé,
- l'un des G₁ et G₂ représente un bon groupe partant LG et l'autre G₁ ou G₂ représente un groupe de formule suivante 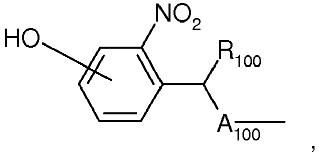 un tensioactif 1 de formule (I) dans lequel G comprend un groupe G' représentant un groupe de formule (XXIV) dans laquelle A₁₀₁ représente O étant alors formé,
- l'un des G₁ et G₂ représente un hydroxyle ou une amine -NH₂ et l'autre G₁ ou G₂ représente un groupe de formule suivante 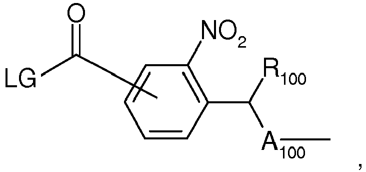 un tensioactif 1 de formule (I) dans lequel G comprend un groupe G' représentant un groupe de formule (XXIV) dans laquelle A₁₀₁ représente respectivement -O-(CO)- ou - NH-(CO) étant alors formé,
- l'un des G₁ et G₂ représente un bon groupe partant LG et l'autre G₁ ou G₂ représente un hydroxyle, un tensioactif 1 de formule (I) dans lequel G comprend un groupe G' représentant un groupe de formule (XXV) étant alors formé,
- l'un des G₁ et G₂ représente un bon groupe partant LG et l'autre G₁ ou G₂ représente une amine -NH₂, un tensioactif 1 de formule (I) dans lequel G comprend un groupe G' représentant un groupe de formule (XXVI) étant alors formé,
- l'un des G₁ et G₂ représente une oxyamine -O-NH₂ et l'autre G₁ ou G₂ représente un aldéhyde, un tensioactif 1 de formule (I) dans lequel G comprend un groupe G' représentant un groupe de formule (XXVII) étant alors formé.

Lorsque le groupe G comprend plusieurs groupes G', le choix des groupes réagissant ensemble : G'₁ et G₁ d'une part et G'₂ et G₂ d'autre part, peut être effectué de la même façon, en remplaçant les groupes G₁ ou G₂ dans les exemples mentionnés ci-dessus par G'₁ ou G'₂.

Le composé 1 de formule (LII) peut être soit un antigène en tant que tel lorsque celui-ci comprend à l'état naturel un groupe -G₂ susceptible de se greffer au tensioactif 1 de formule (LI), soit un antigène modifié chimiquement pour y greffer le groupe G₂ désiré (par l'intermédiaire du groupe de liaison Z₁), cette modification chimique étant réalisée dans des conditions connues de l'homme du métier.

Le procédé peut donc comprendre, préalablement à la mise en contact de l'émulsion prémix 1 et du composé 1 de formule (LII) pour former le tensioactif 1 porteur d'un antigène de formule (I), une étape de préparation du composé 1 de formule (LII) par modification chimique d'un antigène pour y greffer le groupe G₂

Dans un mode de réalisation, le composé 1 de formule (LII) est un antigène naturellement porteur d'une fonction amine -NH₂. On peut notamment citer les antigènes protéiques comprenant au moins une lysine.

Par exemple, pour préparer une émulsion 1 dont le tensioactif 1 de formule (I) a la formule (I') rappelée ci-dessous : 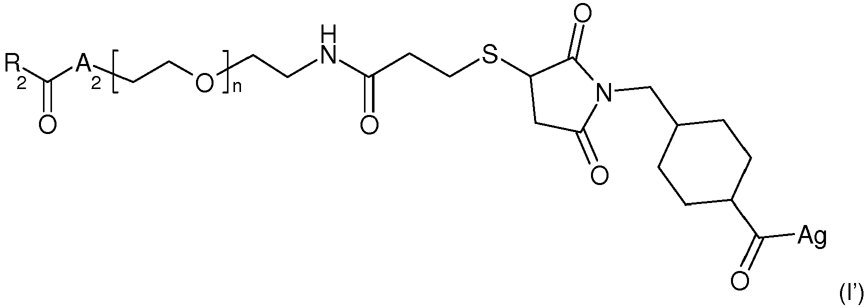 le procédé comprend typiquement :
- la préparation du composé de formule (LII') par modification chimique d'un antigène porteur d'une fonction amine -NH₂ par réaction avec du (Sulfosuccinimidyl-4-N-maleimidomethyl) cyclohexane-1-carboxylate) (Sulfo-SMCC) selon le schéma réactionnel suivant : 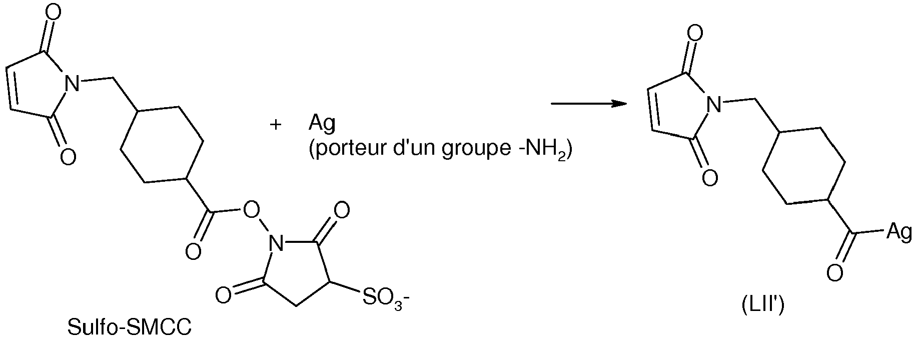
- puis la mise en contact, de préférence réalisée à une température de 0°C à 15°C, notamment de 0 à 10°C, de préférence de 0 à 5°C :
- d'une émulsion prémix 1 comprenant une phase aqueuse continue et une phase dispersée 1 sous forme de gouttelettes, comprenant un lipide amphiphile 1, un lipide solubilisant 1 comprenant au moins un glycéride d'acide gras, un co-tensioactif 1 comprenant au moins une chaine composée de motifs d'oxyde d'alkylène et un tensioactif 1 de formule (LI') suivante : 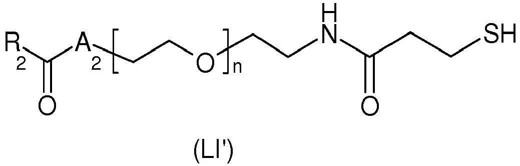 dans laquelle le rapport molaire du tensioactif 1 de formule (LI) sur la somme du co-tensioactif 1 et du tensioactif 1 de formule (LI') est de 0,01 % à 5%,
- avec le composé de formule (LII'),
où R₂, A₂, n et Ag sont tels que définis ci-dessus.

Généralement, le rendement de la réaction entre le tensioactif 1 de formule (LI) et le composé 1 de formule (LII) (c'est-à-dire de la réaction de greffage de l'antigène aux gouttelettes de l'émulsion 1) est supérieur à 40%, notamment supérieur à 50%, typiquement supérieur à 60%. Des rendements supérieurs à 90% peuvent être observés dans certains modes de réalisation. Ces rendements sont variables selon la réaction chimique mise en œuvre (et donc de la nature des groupes G₁ et G₂ ou G'₁ et G'₂), selon la nature du tensioactif 1 de formule (LI) (par exemple, au-delà de 200 motifs d'oxyde d'éthylène dans le groupe H₁, la chaîne poly(oxyde d'éthylène) du tensioactifs 1e replie sur elle-même et les groupes G₁ sont moins accessibles pour réagir avec le composé de formule (LII)) et selon la nature de l'antigène Ag (selon sa taille, sa charge, l'accessibilité de la fonction Z₁-G₂ dans l'espace...).

Les composants de l'émulsion 1 décrits ci-dessus sont disponibles commercialement ou peuvent être préparés en suivant des protocoles décrits dans la littérature.

### • Formation de l'émulsion prémix 1

L'émulsion prémix 1 peut être préparée aisément par dispersion de quantités appropriées de phase huileuse et de phase aqueuse sous l'effet d'un cisaillement, typiquement par un procédé comportant les étapes consistant à :
(i) préparer une phase huileuse comprenant un lipide amphiphile 1 et un lipide solubilisant 1 comprenant au moins un glycéride d'acide gras;
(ii) préparer une phase aqueuse comprenant un co-tensioactif 1 comprenant au moins une chaine composée de motifs d'oxyde d'alkylène et un tensioactif 1 de formule (LI);
(iii) disperser la phase huileuse dans la phase aqueuse sous l'action d'un cisaillement suffisant pour former une émulsion 1; et
(iv) récupérer l'émulsion 1 ainsi formée.

Dans ce procédé, on mélange d'abord les différents constituants huileux pour préparer un pré-mélange huileux pour la phase dispersée 1 de l'émulsion 1. Le mélange des différents constituants huileux peut éventuellement être facilité par mise en solution d'un des constituants ou du mélange complet dans un solvant organique approprié et évaporation subséquente du solvant, pour obtenir un pré-mélange huileux homogène pour la phase dispersée 1. Le choix du solvant organique dépend de la solubilité des constituants. Les solvants employés peuvent être par exemple le méthanol, l'éthanol, le chloroforme, le dichlorométhane, l'hexane, le cyclohexane, le DMSO, le DMF ou encore le toluène. Lorsqu'il s'agit d'une composition immunogène destinée à être administrée, il s'agit de préférence de solvants organiques volatils et/ou non toxiques pour l'homme. Par ailleurs, il est préféré de réaliser le pré-mélange à une température à laquelle l'ensemble des ingrédients est liquide.

Avantageusement, la phase huileuse est dispersée dans la phase aqueuse à l'état liquide. Si l'une des phases se solidifie à température ambiante, il est préférable de réaliser le mélange avec l'une ou de préférence les deux phases chauffées à une température supérieure ou égale à la température de fusion, les deux phases étant chauffées à une température de préférence inférieure à 80°C, et encore préférentiellement inférieure à 70°C, et encore préférentiellement inférieure à 60°C.

L'émulsification sous l'effet de cisaillement est de préférence réalisée à l'aide d'un sonificateur ou d'un microfluidiseur. De préférence, la phase aqueuse puis la phase huileuse sont introduites dans les proportions souhaitées dans un récipient cylindrique approprié puis le sonificateur est plongé dans le milieu et mis en marche pendant une durée suffisante pour obtenir une émulsion, le plus souvent quelques minutes.

L'émulsion prémix 1 est généralement une nanoémulsion. Par le procédé susmentionné, on obtient une nanoémulsion homogène dans laquelle le diamètre moyen des gouttelettes est supérieur à 20 nm et inférieur à 200 nm, notamment de 50 à 120 nm.

De préférence, le potentiel zêta de l'émulsion premix 1 obtenue est inférieur à 25 mV en valeur absolue, c'est-à-dire compris entre -25mV et 25 mV.

Avant préparation de l'émulsion 1, l'émulsion prémix 1 peut être diluée et/ou stérilisée, par exemple par filtration ou dialyse. Cette étape permet d'éliminer les éventuels agrégats qui pourraient s'être formés au cours de la préparation de l'émulsion.

Dans l'émulsion prémix 1, le rapport molaire du tensioactif 1 de formule (LI) sur la somme du co-tensioactif 1 et du tensioactif 1 de formule (LI) est de 0,01 % à 5%. En effet, il a été mis en évidence que le greffage du composé 1 de formule (LII) sur les gouttelettes de l'émulsion prémix 1 (par réaction avec le tensioactif 1 de formule (LI)) n'est pas efficace pour des rapports molaires (tensioactif 1 de formule (LI)) / (co-tensioactif 1 + tensioactif 1 de formule (LI)) supérieur à 5%.

### • Etape 2) : préparation de l'émulsion 2

Dans un mode de réalisation, le procédé comprend une étape 2) de préparation de l'émulsion 2.

Trois alternatives peuvent être distinguées pour préparer l'émulsion 2 selon la nature de l'agent immunostimulant.

Selon une première alternative, l'agent immunostimulant 2 est lipophile (par exemple l'agent immunostimulant 2 est l'imiquimod ou le resiquimod). L'émulsion 2 peut alors être préparée aisément par dispersion de quantités appropriées de phase huileuse et de phase aqueuse sous l'effet d'un cisaillement, typiquement par un procédé comportant les étapes consistant à :
(i) préparer une phase huileuse comprenant un lipide amphiphile 2, un lipide solubilisant 2 comprenant au moins un glycéride d'acide gras et un agent immunostimulant 2 lipophile;
(ii) préparer une phase aqueuse comprenant un co-tensioactif 2 comprenant au moins une chaine composée de motifs d'oxyde d'alkylène;
(iii) disperser la phase huileuse dans la phase aqueuse sous l'action d'un cisaillement suffisant pour former une émulsion 2; et
(iv) récupérer l'émulsion 2 ainsi formée.

Selon une deuxième alternative, l'agent immunostimulant 2 est amphiphile (par exemple l'agent immunostimulant 2 est le MPLA). L'émulsion 2 peut alors être préparée aisément par dispersion de quantités appropriées de phase huileuse et de phase aqueuse sous l'effet d'un cisaillement, typiquement par un procédé comportant les étapes consistant à :
(i) préparer une phase huileuse comprenant un lipide amphiphile 2 et un lipide solubilisant 2 comprenant au moins un glycéride d'acide gras;
(ii) préparer une phase aqueuse comprenant un co-tensioactif 2 comprenant au moins une chaine composée de motifs d'oxyde d'alkylène et un agent immunostimulant 2 amphiphile;
(iii) disperser la phase huileuse dans la phase aqueuse sous l'action d'un cisaillement suffisant pour former une émulsion 2; et
(iv) récupérer l'émulsion 2 ainsi formée.

Dans ces deux alternatives, on mélange d'abord les différents constituants huileux pour préparer un pré-mélange huileux pour la phase dispersée 2 de l'émulsion 2. Le mélange des différents constituants huileux peut éventuellement être facilité par mise en solution d'un des constituants ou du mélange complet dans un solvant organique approprié et évaporation subséquente du solvant, pour obtenir un pré-mélange huileux homogène pour la phase dispersée 2. Le choix du solvant organique dépend de la solubilité des constituants. Les solvants employés peuvent être par exemple le méthanol, l'éthanol, le chloroforme, le dichlorométhane, l'hexane, le cyclohexane, le DMSO, le DMF ou encore le toluène. Lorsqu'il s'agit d'une composition immunogène destinée à être administrée, il s'agit de préférence de solvants organiques volatils et/ou non toxiques pour l'homme. Par ailleurs, il est préféré de réaliser le pré-mélange à une température à laquelle l'ensemble des ingrédients est liquide.

Avantageusement, la phase huileuse est dispersée dans la phase aqueuse à l'état liquide. Si l'une des phases se solidifie à température ambiante, il est préférable de réaliser le mélange avec l'une ou de préférence les deux phases chauffées à une température supérieure ou égale à la température de fusion, les deux phases étant chauffées à une température de préférence inférieure à 80°C, et encore préférentiellement inférieure à 70°C, et encore préférentiellement inférieure à 60°C.

L'émulsification sous l'effet de cisaillement est de préférence réalisée à l'aide d'un sonificateur ou d'un microfluidiseur. De préférence, la phase aqueuse puis la phase huileuse sont introduites dans les proportions souhaitées dans un récipient cylindrique approprié puis le sonificateur est plongé dans le milieu et mis en marche pendant une durée suffisante pour obtenir une émulsion, le plus souvent quelques minutes.

L'émulsion 2 est généralement une nanoémulsion. Par le procédé susmentionné, on obtient une nanoémulsion homogène dans laquelle le diamètre moyen des gouttelettes est supérieur à 20 nm et inférieur à 200 nm, notamment de 50 à 120 nm.

De préférence, le potentiel zêta de l'émulsion 2 obtenue est inférieur à 25 mV en valeur absolue, c'est-à-dire compris entre -25mV et 25 mV. Celui-ci peut être mesuré par un appareil Zetasizer Nano ZS de chez Malvern Instruments.

Selon une troisième alternative, l'agent immunostimulant 2 est porteur d'au moins un groupe anionique, typiquement il s'agit d'une séquence nucléotidique mono ou double brin, comprenant moins de 200 bases pour une séquence nucléotidique mono brin ou moins de 200 paires de base pour une séquence nucléotidique double brin, par exemple l'oligodéoxynucléotide (ODN) CpG. L'émulsion 2 comprend alors généralement un tensioactif 2 cationique et est alors typiquement préparée en suivant le procédé de préparation décrit p. 41 à 44 de la demande WO 2014/032953 (en utilisant l'agent immunostimulant 2 porteur d'au moins un groupe anionique à la place des séquences nucléotidiques susceptibles de moduler des mécanismes endogènes d'ARN interférence lors de l'étape (iv)).

L'émulsion 2 est généralement une nanoémulsion. Par cette troisième alternative, on obtient une nanoémulsion homogène dans laquelle le diamètre moyen des gouttelettes est compris entre 20 et 250 nm, typiquement entre 40 et 200 nm.

Quelle que soit l'alternative du procédé, avant préparation de la composition immunogène selon l'invention, c'est-à-dire le mélange avec l'émulsion 1, l'émulsion 2 peut être diluée et/ou stérilisée, par exemple par filtration ou dialyse. Cette étape permet d'éliminer les éventuels agrégats qui pourraient s'être formés au cours de la préparation de l'émulsion.

### [Utilisations de la composition immunogène]

### • Méthode de production d'anticorps

L'invention concerne également une méthode de production d'anticorps monoclonaux ou polyclonaux mettant en œuvre la composition immunogène définie ci-dessus.

Ainsi, l'invention concerne une méthode de production d'anticorps polyclonaux, comprenant les étapes consistant en :
(a) l'immunisation d'un animal avec une composition immunogène telle que définie ci-dessus, de manière à induire une réponse immune humorale (ou des réponses immunes humorales) contre ledit antigène, et
(b) la récolte des anticorps polyclonaux induits dirigés contre ledit antigène.

L'invention concerne également une méthode de production d'anticorps monoclonaux, comprenant les étapes consistant en :
(i) l'immunisation d'un animal avec une composition immunogène selon l'invention,
(ii) récupération et isolement des lymphocytes B de l'animal immunisé à l'étape (i),
(iii) production d'un hybridome et culture dudit l'hybridome afin de produire des anticorps monoclonaux dirigés contre l'antigène présent dans ladite composition immunogène,
(iv) récolte et purification des anticorps monoclonaux produits à l'étape (iii).

L'immunisation aux étapes (a) et (i) est réalisée en injectant la composition immunogène selon l'invention à un animal en une dose efficace pour induire une réponse immune humorale à un antigène tel que défini ci-dessus. L'homme du métier est capable de déterminer les conditions nécessaires à l'immunisation des animaux. Plusieurs protocoles d'immunisation sont ainsi possibles en fonction de l'antigène présent dans la composition immunogène selon l'invention, par exemple en variant les doses, les intervalles des injections, la durée du traitement.

L'animal utilisé dans les méthodes de production d'anticorps selon l'invention est un animal classiquement utilisé pour produire des anticorps, à savoir un rongeur (souris, rat, hamster), un lapin, une chèvre, un mouton, un singe, une poule, un cochon d'inde, ou un cheval.

Optionnellement, dans les méthodes de production d'anticorps selon l'invention, une étape de contrôle, dans le sang de l'animal immunisé, de la présence d'anticorps dirigé contre l'antigène présent dans la composition immunogène selon l'invention est réalisée après les étapes (a) ou (i) d'immunisation. Cette étape de contrôle est réalisée par des techniques classiques connues de l'homme du métier, par exemple en titrant la quantité d'anticorps dans le sérum de l'animal immunisé par ELISA.

Dans la méthode de production d'anticorps polyclonaux selon l'invention, l'étape (b) de récolte des anticorps polyclonaux induits dirigés contre ledit antigène est réalisée par des techniques classiques connues de l'homme du métier. Cette étape comprend notamment une collecte du sang de l'animal immunisé (avec ou sans sacrifice de l'animal), suivie de l'isolement du sérum qui contient les anticorps polyclonaux, et optionnellement de la purification des anticorps polyclonaux.

Dans la méthode de production d'anticorps monoclonaux selon l'invention, la récupération et l'isolement des lymphocytes B à l'étape (iii) est réalisée par des techniques classiques connues de l'homme du métier. Cette étape fait notamment intervenir le sacrifice de l'animal immunisé, suivi du prélèvement de la rate, et de l'isolement des lymphocytes B à partir de la rate prélevée.

La production d'un hybridome à l'étape (iv) est réalisée selon des techniques classique connues de l'homme du métier, et implique notamment la fusion des lymphocytes B isolés à l'étape (iii) avec un myélome, de sorte à produire un hybridome. La fusion est par exemple réalisée en utilisant du polyéthylène glycol ou par électroporation. L'hybridome ainsi obtenu est ensuite cultivé dans des conditions appropriées facilement déterminables par l'homme du métier de sorte à permettre à l'hybridome de sécréter des anticorps. Selon l'échelle de production d'anticorps désirée, cette étape de culture de l'hybridome peut notamment être réalisée en bio-réacteur.

Dans une dernière étape (v), les anticorps ainsi sécrétés sont récoltés et purifiés à l'aide de techniques classique connues de l'homme du métier, comme par exemple par chromatographie liquide haute performance, par chromatographie d'affinité en utilisant la protéine G, ou encore par précipitation à l'ammonium.

### • Utilisation en tant que médicament ou vaccin

L'invention a également pour objet une composition immunogène telle que définie ci-dessus pour son utilisation en tant que médicament ou vaccin.

L'invention concerne également une composition immunogène telle que définie ci-dessus pour son utilisation pour induire une réponse immune (ou des réponses immunes) contre l'antigène présent dans la composition immunogène. De préférence, la composition immunogène telle que définie ci-dessus est utilisée pour induire une réponse immune contre l'antigène présent dans la composition immunogène chez un individu auquel ladite composition immunogène est administrée.

Dans certains modes de réalisation, ladite réponse immune est une réponse immune humorale contre l'antigène présent dans la composition immunogène.

Dans certains modes de réalisation, la composition immunogène selon l'invention est utilisée pour traiter ou prévenir une infection, un cancer, une maladie inflammatoire, une allergie, une maladie liée à l'âge comme la dégénérescence maculaire liée à l'âge (DMLA), ou une maladie neuro-dégénérative, selon la nature de l'antigène présent dans la composition immunogène.

Par « infection », on entend une infection causée par n'importe quel pathogène, i.e. un virus, une bactérie, une levure ou un parasite. De préférence, l'infection est une infection due à un pathogène à partir duquel au moins un antigène tel que défini ci-dessus est dérivé.

Par « allergie », on entend une allergie due à n'importe quel allergène. De préférence, l'allergie est une allergie due à un allergène à partir duquel au moins un antigène tel que défini ci-dessus est dérivé.

Par « maladie inflammatoire », on entend ici une maladie associée à une inflammation. Des exemples de maladies inflammatoires sont bien connus de l'homme du métier et incluent en particulier l'athérosclérose, l'ischémie myocardique, l'acné, l'asthme, les maladies auto-immunes, la prostatite, la glomérulonéphrite, les hypersensibilités, les maladies inflammatoires chroniques intestinales, les maladies inflammatoires pelviques, la polyarthrite rhumatoïde, le rejet de greffe, la vasculite, la cystite interstitielle, les allergies et les myopathies inflammatoires.

Par « cancer », on entend n'importe quel cancer. De préférence, le cancer est un cancer à partir duquel au moins un antigène tumoral tel que défini ci-dessus est dérivé.

La composition immunogène peut être une composition à visée prophylactique ou à visée thérapeutique, ou encore les deux.

De préférence, ladite composition immunogène selon l'invention est un vaccin.

Dans certains modes de réalisation, ladite composition est administrée à un humain, incluant un homme, une femme ou un enfant, ou à un mammifère non humain, incluant un primate (singe), un félin (chat), un canin (chien), un bovin (vache), un ovin (mouton, chèvre), un équin (cheval), un porcin (cochon), un rongeur (rat, souris, hamster, cochon d'inde), ou un lapin.

### • Méthode d'immunisation

Avantageusement, la composition selon l'invention est très stable dans des milieux biologiques. Sans vouloir être liés par des mécanismes particuliers, on suppose que les gouttelettes de phases dispersées 1 et 2, qui ont un diamètre inférieur à 250 nm, sont préférentiellement captées par les cellules immunes, en particulier car les gouttelettes ont un tropisme « naturel » pour les ganglions lymphatiques riches en cellules lymphoides et dendritiques.

L'invention concerne également une méthode d'immunisation contre une maladie chez individu le nécessitant comprenant l'administration à l'individu d'une composition immunogène ou d'un vaccin selon l'invention. De préférence, ladite composition immunogène ou ledit vaccin est administré(e) en une dose immunoprotectrice.

Par « individu le nécessitant », on entend un individu qui développe ou qui risque de développer une maladie. L'individu peut être un humain, incluant un homme, une femme ou un enfant, ou un mammifère non humain, incluant un primate (singe), un félin (chat), un canin (chien), un bovin (vache), un ovin (mouton, chèvre), un équin (cheval), un porcin (cochon), un rongeur (rat, souris, hamster, cochon d'inde), ou un lapin.

Dans certains modes de réalisation, la maladie est une infection, une allergie, une maladie inflammatoire ou un cancer. De préférence, ladite infection est une infection virale, bactérienne, parasitaire, ou une maladie à prion, causée par un pathogène ou un agent à partir duquel l'antigène tel que défini ci-dessus est dérivé. De préférence, ladite allergie est une allergie due à un allergène tel que défini ci-dessus. De préférence, ledit cancer est un cancer à partir duquel l'antigène tel que défini ci-dessus est dérivé.

Le mode d'administration peut être n'importe quel mode d'administration utilisé dans le domaine des vaccins. La composition immunogène peut notamment être administrée par voie intradermique, intramusculaire, topique, trans-cutanée, cutanée, mucosale, intranasale. De préférence, lorsque la composition immunogène selon l'invention est sous forme de gel, elle est administrée par voie cutanée ou mucosale.

Par « dose immunoprotectrice », on entend une quantité capable d'induire une réponse immune humorale et/ou cellulaire spécifique. La réponse immune humorale est évaluée par la détection de la présence d'anticorps neutralisants dans le sérum de l'hôte vacciné selon des techniques connues de l'homme du métier. La réponse immune cellulaire est évaluée par la détection de la présence de lymphocytes T CD4+, T CD8+, et/ou les cellules NK dans le sérum de l'hôte vacciné selon des techniques connues de l'homme du métier. La quantité de composition ou de vaccin selon la présente invention ainsi que la fréquence d'administration sera déterminée par des études cliniques, par le médecin ou par le pharmacien. La « dose immunoprotectrice » spécifique à chacun des individus pourra dépendre d'un certain nombre de facteurs comme la nature et la sévérité du désordre à traiter, la composition utilisée, l'âge, le poids, l'état de santé général, le sexe et le régime de l'individu, le mode d'administration, la durée du traitement (en monodose ou en plusieurs doses), les médicaments utilisés en combinaison et d'autres facteurs bien connus des spécialistes médicaux.

Avantageusement, la composition immunogène selon l'invention permet d'induire de fortes réponses immunes à la fois humorales et cellulaires.

Le volume d'administration peut varier selon le mode d'administration. De préférence, lors d'une administration par voie sous-cutanée, le volume peut être compris entre 0.1 ml et 10 ml.

Le moment optimal d'administration de la composition immunogène selon l'invention est d'environ 1 semaine à 6 mois, de préférence 1 mois à 3 mois, avant la première exposition au pathogène. La composition immunogène peut être administrée en tant qu'agent prophylactique chez les hôtes à risque de développer une maladie telle que définie ci-dessus.

La composition immunogène selon l'invention peut être administrée en une dose unique, ou optionnellement, l'administration peut impliquer l'utilisation d'une première dose suivie d'une seconde dose (dose « booster ») qui est administrée, par exemple 2 à 6 mois plus tard, comme déterminé de manière appropriée par l'homme du métier.

L'invention est illustrée au vu des figures et exemples qui suivent.

### FIGURES

La figure 1 représente un schéma illustrant une coupe d'une gouttelette de la phase dispersée 1. Sur la couronne sont représentés des antigènes représentés par 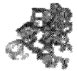 greffés de façon covalente aux gouttelettes (par les tensioactifs 1 de formule (I)), des agents de ciblage représentés par 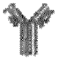 (lipide mannosylé ou anticorps) et des agents immunostimulants représentés par 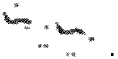
La figure 2 représente le schéma réactionnel de déprotection du précurseur du tensioactif 1 de formule (LI') par clivage du groupe -S-Pyridinyle pour libérer la fonction thiol qui servira ultérieurement à greffer l'antigène ovalbumine (exemple 1 paragraphe 1.1.2.).
La figure 3 représente un schéma illustrant une coupe d'une gouttelette de l'émulsion prémix 1 de l'exemple 1 paragraphe 1.1.2.. Le cœur des gouttelettes (représenté par 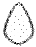 ) comprend l'huile 1 et le lipide solubilisant 1, et la couronne comprend le lipide amphiphile 1 (représenté par 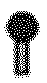 ), le co-tensioactif 1 et le tensioactif 1 de formule (LI') (représentés par 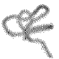 ).
La figure 4 représente le schéma réactionnel de modification chimique de l'ovalbumine pour lui greffer un groupe maléimide (servant ultérieurement à greffer l'ovalbumine modifiée aux gouttelettes de l'émulsion prémix 1) et un fluorophore (exemple 1 paragraphe 1.2.1.).
La figure 5 représente le schéma réactionnel de greffage de l'ovalbumine modifiée sur une gouttelette de prémix puis blocage des fonctions thiols restantes avec Mal-OH (exemple 1 paragraphe 1.2.2.).
La figure 6 fournit l'intensité de fluorescence (ordonnée) en fonction du volume d'élution en mL (abscisses) pour l'ovalbumine (trait plein) ou pour les gouttelettes (trait en pointillés) (exemple 1 paragraphe 1.2.2.).
La figure 7 fournit le nombre moyen d'ovalbumine greffé par gouttelette (ordonnée) en fonction de la masse en mg de précurseur de tensioactif 1 de formule (LI') dans l'émulsion 1 (abscisses), les carrés correspondant aux points expérimentaux et la ligne en pointillé à l'extrapolation linéaire (exemple 1 paragraphe 1.2.2.).
La figure 8 est une photographie d'un gel de SDS-PAGE de 1) l'ovalbumine fonctionnalisée par le tensioactif 1 de formule (LI') ; 2) l'ovalbumine libre ; 3) les gouttelettes fonctionnalisées par l'ovalbumine et issues d'une émulsion prémix 1 B ; 4) les gouttelettes fonctionnalisées par l'ovalbumine et issues d'une émulsion prémix 1 B' (exemple 1 paragraphe 1.2.2.).
La figure 9 fournit la viabilité d'une lignée de fibroblastes 3T3 en % (ordonnées) en fonction de la proportion en µg/mL d'émulsion 1 (abscisses) (exemple 1 paragraphe 1.2.5.).
   Courbe losanges pleins, trait en pointillés : fibroblastes incubés en présence de gouttelettes possédant 0% molaire de tensioactif 1 fonctionnalisable.
   Courbe carrés pleins, trait plein : fibroblastes incubés en présence de gouttelettes possédant 0,35% molaire de tensioactif 1 fonctionnalisable et dont les fonctions thiols ont été « désactivées » par réaction avec un maléimide-OH.
   Courbe carrés vides, trait plein : fibroblastes incubés en présence de gouttelettes possédant 0,88% molaire de tensioactif 1 fonctionnalisable et dont les fonctions thiols ont été désactivées par réaction avec un maléimide-OH.
   Courbe triangles pleins, trait plein : fibroblastes incubés en présence de gouttelettes possédant 0,35% molaire de tensioactif 1 fonctionnalisable sur lesquels l'ovalbumine a été greffée.
   Courbe triangles vides, trait plein : fibroblastes incubés en présence de gouttelettes possédant 0,88% molaire de tensioactif 1 fonctionnalisable sur lesquels l'ovalbumine a été greffée.

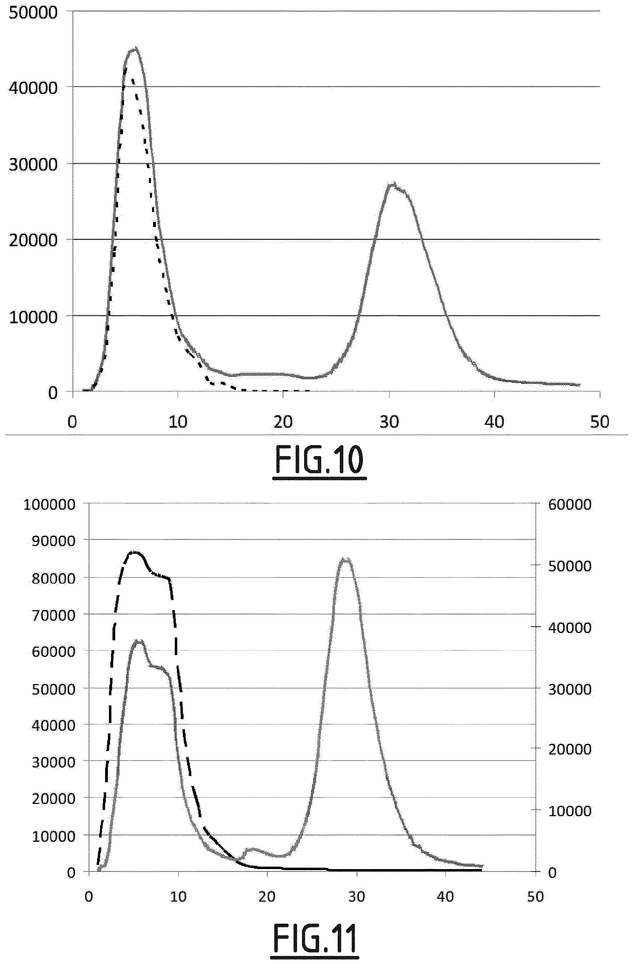

La figure 10 fournit l'intensité de fluorescence (ordonnée) en fonction du volume d'élution en mL (abscisses) pour le peptide (trait plein) ou pour les gouttelettes (trait en pointillés) (exemple 2).
La figure 11 fournit l'intensité de fluorescence (ordonnée) en fonction du volume d'élution en mL (abscisses) pour le peptide (trait plein) ou pour les gouttelettes (trait en pointillés) (exemple 3).

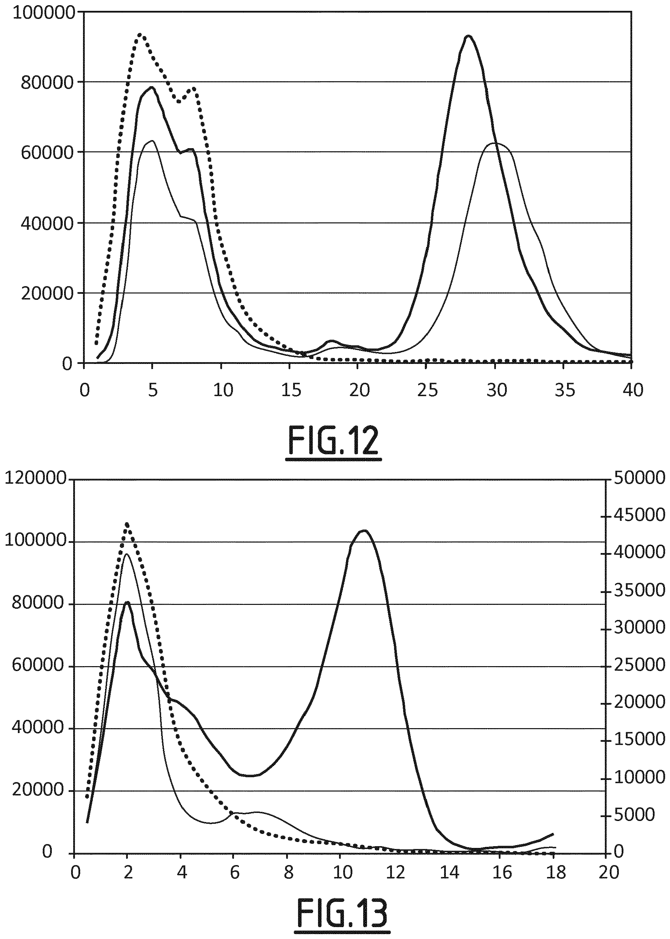

La figure 12 fournit l'intensité de fluorescence (ordonnée) en fonction du volume d'élution en mL (abscisses) pour les peptides (traits pleins) ou pour les gouttelettes (trait en pointillés) (exemple 4).
La figure 13 représente l'intensité de fluorescence (ordonnées) en fonction du volume en mL (abscisses) pour l'ovalbumine (trait plein épais), l'anticorps (trait plein fin) et les gouttelettes (trait en pointillés) (exemple 7).

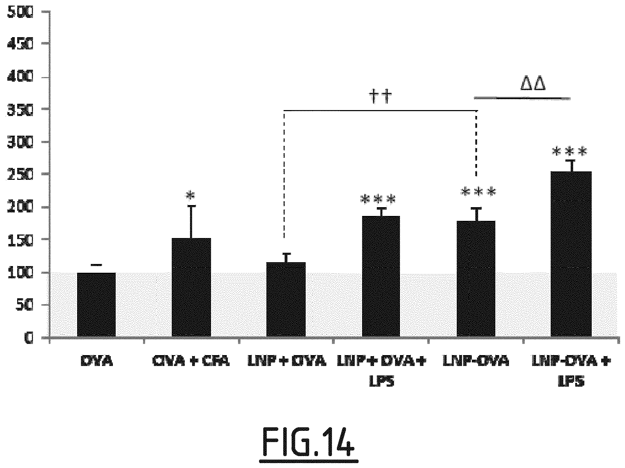

La figure 14 représente les résultats d'immunisation obtenus pour n = 4 individus, à savoir le pourcentage d'anticorps anti-ovalbuline (%OVA) (ordonnées) en fonction de la composition utilisée :
   - OVA pour l'administration d'ovalbumine seule sans vecteur (comparatif),
   - OVA + CFA pour l'administration d'ovalbumine seule sans vecteur et d'adjuvant CFA (comparatif),
   - LNP+OVA pour l'administration d'ovalbumine et de gouttelettes d'émulsion 1, l'ovalbumine n'étant pas liée aux gouttelettes (comparatif),
   - LNP+OVA+LPS pour l'administration d'ovalbumine, de gouttelettes d'émulsion 1 et d'agent immunostimulant LPS, l'ovalbumine n'étant pas liée aux gouttelettes et le LPS n'étant pas incorporé dans les gouttelettes (comparatif),
   - LNP-OVA pour l'administration d'ovalbumine liée de façon covalente aux gouttelettes d'émulsion 1,
   - LNP-OVA+LPS pour l'administration d'ovalbumine liée de façon covalente aux gouttelettes d'émulsion 1, et le LPS n'étant pas incorporé dans les gouttelettes.
      * p < 0,05 ; *** p < 0,001 comparé aux contrôles OVA ; †† p < 0,01 ; ΔΔ p < 0,01 (exemple 8).

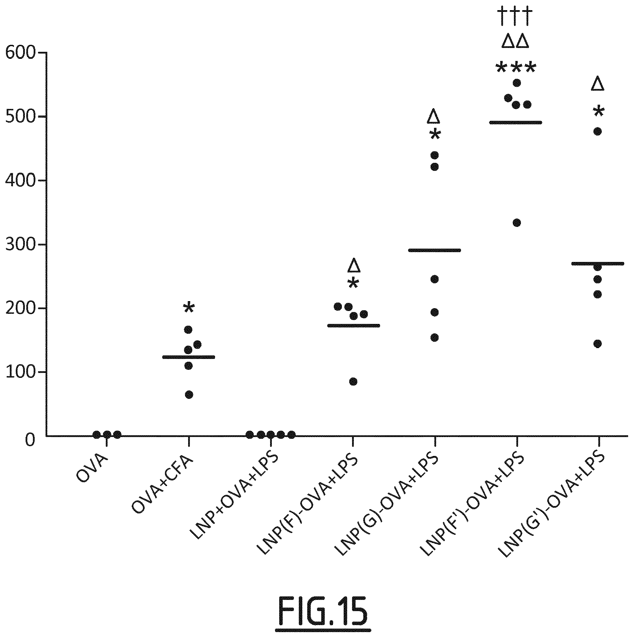

La figure 15 représente les résultats d'immunisation obtenus pour n = 5 individus, à savoir la proportion Ig total anti-OVA (ng/mL) dans le sera des souris immunisées par l'ovalbumine (ordonnées) en fonction de la composition utilisée :
   - OVA pour l'administration d'ovalbumine seule sans vecteur (comparatif),
   - OVA + CFA pour l'administration d'ovalbumine seule sans vecteur et d'adjuvant CFA (comparatif),
   - LNP+OVA+LPS pour l'administration d'ovalbumine, de gouttelettes d'émulsion 1 F à l'exemple 1.1.2 et d'agent immunostimulant LPS, l'ovalbumine n'étant pas liée aux gouttelettes de l'émulsion 1 et le LPS n'étant pas incorporé dans les gouttelettes de l'émulsion 1 (comparatif),
   - LNP(F)-OVA+LPS pour l'administration de gouttelettes d'émulsion 1 F greffées par l'ovalbumine obtenue à l'exemple 1.2.2 et d'agent immunostimulant LPS, le LPS n'étant pas incorporé dans les gouttelettes,
   - LNP(G)-OVA+LPS pour l'administration de gouttelettes d'émulsion 1 G greffées par l'ovalbumine obtenue à l'exemple 1.2.2 et d'agent immunostimulant LPS, le LPS n'étant pas incorporé dans les gouttelettes,
   - LNP(F')-OVA+LPS pour l'administration de gouttelettes d'émulsion 1 F' greffées par l'ovalbumine obtenue à l'exemple 1.2.2 et d'agent immunostimulant LPS, le LPS n'étant pas incorporé dans les gouttelettes,
   - LNP(G')-OVA+LPS pour l'administration de gouttelettes d'émulsion 1 G' greffée par l'ovalbumine obtenue à l'exemple 1.2.2 et d'agent immunostimulant LPS, le LPS n'étant pas incorporé dans les gouttelettes.
      test Wilconson: *
      * p<0,05; * * * p<0,001 en comparaison avec OVA
      Δ p<0,05; ΔΔ p<0,01 en comparaison avec OVA+CFA
      ttt p<0,001 en comparaison avec LNP(F)-OVA + LPS
      (exemple 8).

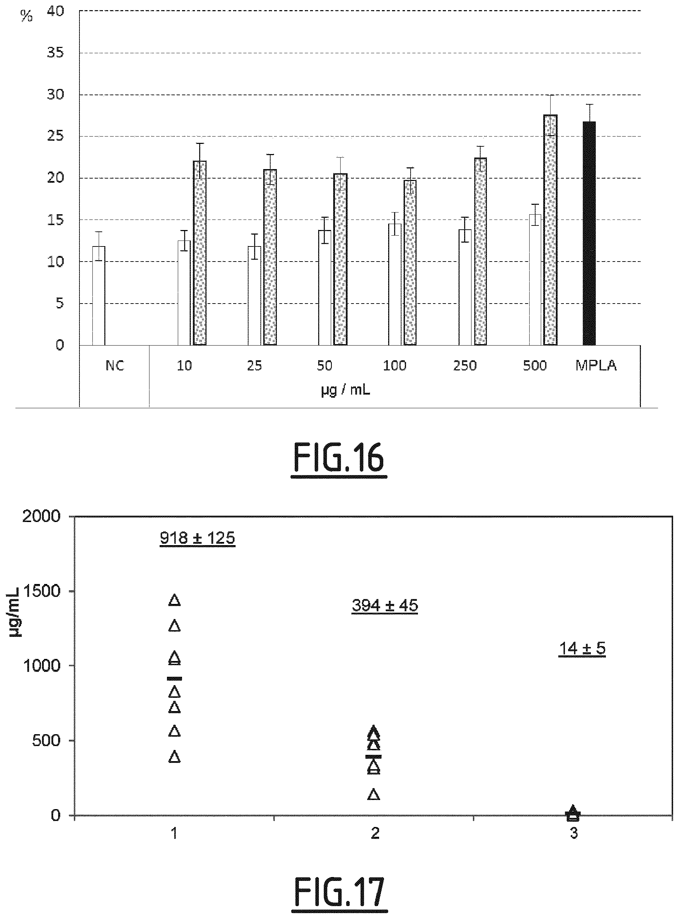

La figure 16 représente le pourcentage de cellules dendritiques activées en fonction de la concentration en µg/mL de phase dispersée (exemple 10). Pour chaque concentration de phase dispersée sont représentées le pourcentage de cellules dendritiques activées d'une part lorsqu'une phase dispersée exempte d'agent immunostimulant y ont été ajoutées (colonnes à gauche, en grisé) (comparatif) et d'autre part, lorsqu'une phase dispersée 2 comprenant du MPLA y ont été ajoutés (colonne de droite, en pointillés) (émulsion 2). La première colonne à gauche (NC) est un contrôle négatif dans lequel rien n'a été ajouté aux cellules dendritiques. La dernière colonne à droite (MPLA) est un comparatif dans lequel du MPLA libre a été ajouté (contrôle positif).
La figure 17 représente les résultats du dosage ELISA de l'exemple 13, à savoir la proportion d'anticorps anti-ovalbumine produits en µg/mL suite à :
   - 1 : l'administration de la composition immunogène selon l'invention, ou
   - 2 : la co-administration de l'émulsion 1 et de l'immunostimulant libre, ou
   - 3 : la co-administration de l'antigène libre et de l'immunostimulant libre.

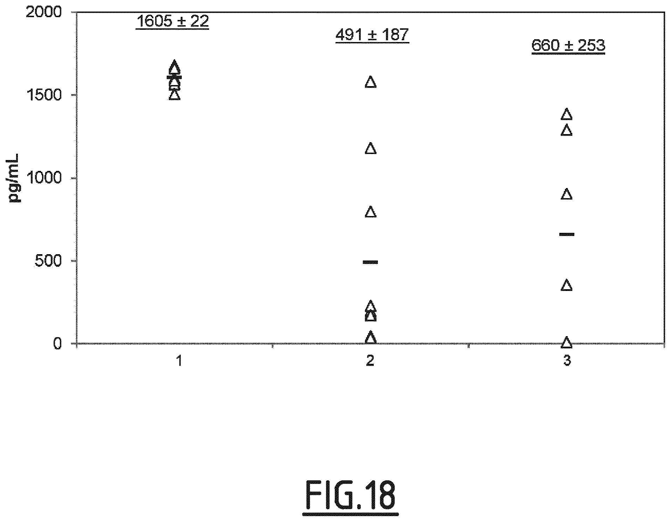

La figure 18 représente la différence Δ[IFNg] en pg/mL de taux d'IFNg entre l'échantillon exposé à l'ovalbumine pendant la culture et l'échantillon contrôle (exemple 13) pour :
   - 1 : un échantillon exposé à l'ovalbumine obtenu suite à l'administration de la composition immunogène selon l'exemple 12, ou
   - 2 : un échantillon exposé à l'ovalbumine obtenu suite à la co-administration de l'émulsion 1 et de l'immunostimulant libre, ou
   - 3 : un échantillon exposé à l'ovalbumine obtenu suite à la co-administration de l'antigène libre et de l'immunostimulant libre.

### EXEMPLES

### Exemple 1 : Préparation d'une émulsion 1 comprenant un tensioactif 1 porteur d'un antigène (ovalbumine) de formule (I')

### 1.1. Préparation de l'émulsion prémix 1

Une émulsion prémix 1 comprenant un tensioactif 1 de formule (LI') dans laquelle R₂ représente C₁₇H₃₅, A₂ représente NH et n représente 100, soit de formule suivante : 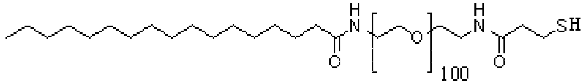 a été préparée comme suit.

### 1.1.1. Préparation d'un précurseur du tensioactif 1 de formule (LI')

Un précurseur du tensioactif 1 de formule (LI') dans lequel la fonction thiol terminale est protégée par un groupe -S-Pyridinyle a été préparé en suivant le schéma réactionnel suivant :

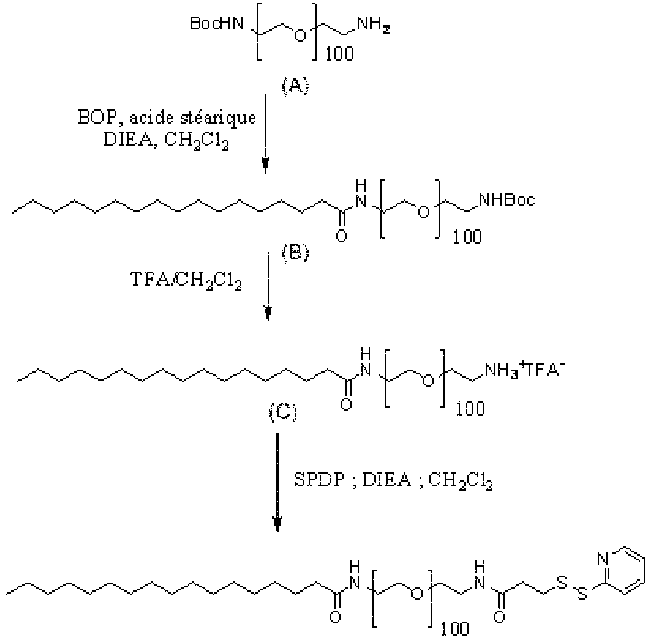

### Synthèse du composé (B)

De l'acide stéarique (2 g ; 0,6 mmol) et du Benzotriazole-1-yl-oxy-tris-(dimethylamino)-phosphonium hexafluorophosphate (BOP) (265,2 mg ; 0,6 mmol) ont été dissous dans du CH₂Cl₂ (15 mL). Après 10 minutes d'agitation, du BocNH-PEG100-NH₂ (PM : 4928 ; 2 g ; 0,4 mmol) (composé (A)) et de la diisopropyléthylamine (DIEA) (104,5 mL ; 0,6 mmol) ont été ajoutés au milieu réactionnel. La disparition de l'amine de départ a été vérifiée par chromatographie sur couche mince (CCM) (CH₂Cl₂/MeOH). Après 2 heures sous agitation, le produit a été précipité dans l'éther à froid, dissous dans un minimum d'eau puis dialysé contre de l'eau milli Q (cut off 1000). La solution a ensuite été récupérée et l'eau a été enlevée soit par évaporation (éthanol comme azéotrope) soit par lyophilisation, pour donner 1,5 g de composé (B) (poudre blanche), soit un rendement de 70 %.
CCM (CH₂Cl₂/ MeOH 9/1): Rf=0,5
RMN ¹H (300 MHz ; CDCl3) : d : 0,87 (t ; *J*=6,5 Hz ; 3H ; C**H₃**-CH₂) ; 1,24 (m ; 28H ; 14C**H₂**) ; 1,44 (s ; 9H ; C(C**H₃**)₃) ; 1,67 (quin ; 2H ; C**H₂**-CH₂-CONH) ; 2,42 (t ; *J*=7,5 Hz ; 2H ; C**H₂**-CONH) ; 3,3 (t ; J=5,0 Hz ; 2H ; BocNH-C**H₂**) ; 3,45-3,8 (m ; 362H ; xC**H₂**(PEG), CH₂CONH-C**H₂**)

### Synthèse du composé (C)

Le composé (B) (1,5 g ; 0,29 mmol) a été dissous dans 10 mL de dichlorométhane et 4 mL d'acide trifluoroacétique (TFA). La conversion en composé (C) a été suivie par CCM (révélateur ninhydrine). Après 1 heure sous agitation, le solvant a été évaporé par coévaporation avec du toluène (qui élimine le TFA). Le produit a été séché sous vide pour donner 1,3 g de composé (C) (poudre blanche), soit un rendement de 86,7 %
CCM (CH₂Cl₂/ MeOH 9/1): Rf=0,27
RMN ¹H (300 MHz ; CDCl3) : d : 0,87 (t ; *J*=6,5 Hz ; 3H ; C**H₃**-CH₂) ; 1,24 (m ; 28H ; 14C**H₂**) ; 1,60 (quin ; 2H ; C**H₂**-CH₂-CONH) ; 2,15 (t ; *J*=7,5 Hz ; 2H ; C**H₂**-CONH) ; 3,17 (bt ; 2H ; C**H₂**-NH₃⁺) ; 3,4 (m ; 2H ; CH₂CONH-C**H₂**) ; 3,5-3,8 (m ; 360H ; xC**H₂**(PEG)) ; 6,14 (bs ; 1H ; N**H**CO) ; 7,9 (bs ; 2H ; N**H₂**/N**H₃**+)

### Synthèse du précurseur du tensioactif 1 de formule (LI')

Sous argon, du composé (C) (0,5 g ; 0,1 mmol) et de la DIEA (52 mL ; 0,3 mmol) ont été dissous dans du dichlorométhane (10 mL). Après 5 minutes sous agitation du Succinimidyl 3-(2-pyridyldithio)propionate (SPDP) (93 mg ; 0,3 mmol) ont été ajoutés dans le milieu réactionnel. La disparition de l'amine a été suivie par CCM (CH₂Cl₂/MeOH 9/1). Après 1 heure de réaction, le produit a été précipité à deux reprises dans l'éther pour donner après filtration 400 mg de précurseur (poudre jaunâtre) soit un rendement de 76 % CCM (CH₂Cl₂/ MeOH 9/1): Rf=0,42
RMN 1 H (300 MHz ; CDCl3) : d : 0,88 (t ; *J*=6,5 Hz ; 3H ; C**H₃**-CH₂) ; 1,25 (m ; 28H ; 14C**H₂**) ; 1,63 (quin ; 2H ; C**H₂**-CH₂-CONH) ; 2,17 (t ; *J*=7,5 Hz ; 2H ; C**H₂**-CONH) ; 2,62 (t ; *J*=7 Hz ; 2H ; C**H₂**-SS) ; 3,09 (t ; *J*=7 Hz ; 2H ; NHCO-C**H₂**-CH₂-SS) ; 3,42 (m ; 2H ; C**H₂**-NHCO) ; 3,48-3,8 (m ; 360H ; xC**H₂**(PEG) ; C**H₂**-NHCO) ; 6,11 (bt ; 1H ; N**H**) ; 6,79 (bt ; 1H ; N**H**) ; 7,11 (m ; 1H ; C**H**pyr) ; 7,67 (m ; 2H ; 2C**H**pyr) ; 8,49 (m ; 1H ; C**H**pyr)

### 1.1.2. Préparation d'émulsions prémix 1 comprenant le tensioactif 1 de formule (LI')

L'émulsion prémix 1 a été préparée en suivant les procédures décrites dans WO 2010/018223 avec les compositions indiquées dans les tableaux 2 et 3, la dissolution complète du Myrj S40 et du tensioactif 1 de formule (LII) dans la phase aqueuse ayant nécessité de chauffer la solution à 60°C. Les phases aqueuse et huileuse sont alors mélangées puis émulsifiées par sonication selon les paramètres décrits dans le tableau 4.

**Tableau 2 : Formulation des émulsions prémix 1 de diamètre 120 nm comprenant le précurseur du tensioactif 1 de formule (LI')**

| | Lipide amphiphile lécithine S75 (Lipoïd) (mg) | Lipide solubilisant Suppocire® NB (Gattefossé) (mg) | Lipide cationique (DOTAP) (mg) | Huile 1 de soja (Croda) (mg) | PBS 1X (µL) | co-tensioactif 1 MYRJ S40 (CRODA) (PEG 40) | | précurseur | | Ratio précurseur / (précurseur + co-tensioactif 1) (%) | |
|---|---|---|---|---|---|---|---|---|---|---|---|
| | | | | | | mg | mmol | mg | mmol | % mol | % mas |
| A | 45 | 450 | 0 | 150 | 1140 | 214 | 107 | 1 | 0.19 | 0,18 | 0,47 |
| B | 45 | 450 | 0 | 150 | 1140 | 213 | 106,5 | 2 | 0.37 | 0,35 | 0,94 |
| C | 45 | 450 | 0 | 150 | 1140 | 210 | 105 | 5 | 0.94 | 0,88 | 2,33 |
| D | 45 | 450 | 0 | 150 | 1140 | 205 | 102,5 | 10 | 1.87 | 1,79 | 4,65 |
| E | 45 | 450 | 0 | 150 | 1140 | 195 | 97,5 | 20 | 3.75 | 3,70 | 9,30 |
| F | 45 | 450 | 0 | 150 | 1140 | 210 | 105 | 5 | 0.94 | 0,88 | 2,33 |
| G | 11 | 450 | 34 | 150 | 1140 | 210 | 105 | 5 | 0.94 | 0,88 | 2,33 |

| | | | | | | | | | | | |
|---|---|---|---|---|---|---|---|---|---|---|---|
| Dans le tableau 2, « précurseur » signifie « précurseur du tensioactif 1 de formule (LI'), « %mol » signifie %molaire et « %mas » signifie %massique. Dans les 7 émulsions prémix 1, la masse totale de (précurseur du tensioactif 1 de formule (LI') + co-tensioactif 1) est toujours de 215 mg. | | | | | | | | | | | |

**Tableau 3 : Formulation des émulsions prémix 1 de diamètre 80 nm comprenant le précurseur du tensioactif 1 de formule (LI').**

| | Lipide amphiphile lécithine S75 (Lipoïd) (mg) | Lipide solubilisant 1 Suppocire® NB (Gatte fossé) (mg) | Lipide cationique (DOTAP) (mg) | Huile 1 de soja (Croda) (mg) | PBS 1X (µL) | co-tensioactif 1 MYRJ S40 (CRODA) (PEG 40) | | précurseur | | Ratio Précurseur / (précurseur + co-tensioactif 1) (%) | |
|---|---|---|---|---|---|---|---|---|---|---|---|
| | | | | | | mg | mmol | mg | mmol | % mol | % mass |
| A' | 50 | 307,5 | 0 | 102,5 | 1240 | 299 | 149,5 | 1 | 0,19 | 0,13 | 0,33 |
| B' | 50 | 307,5 | 0 | 102,5 | 1240 | 298 | 149 | 2 | 0,37 | 0,25 | 0,67 |
| C' | 50 | 307,5 | 0 | 102,5 | 1240 | 295 | 147,5 | 5 | 0,94 | 0,63 | 1,67 |
| D' | 50 | 307,5 | 0 | 102,5 | 1240 | 290 | 145 | 10 | 1,87 | 1,28 | 3,33 |
| E' | 50 | 307,5 | 0 | 102,5 | 1240 | 280 | 140 | 20 | 3,75 | 2,61 | 6,67 |
| F' | 50 | 307,5 | 0 | 102,5 | 1240 | 295 | 147,5 | 5 | 0,94 | 0,63 | 1,67 |
| G' | 12 | 307,5 | 38 | 102,5 | 1240 | 295 | 147,5 | 5 | 0,94 | 0,63 | 1,67 |

| | | | | | | | | | | | |
|---|---|---|---|---|---|---|---|---|---|---|---|
| Dans le tableau 3, « précurseur » signifie « précurseur du tensioactif 1 de formule (LI'), « %mol » signifie %molaire et « %mas » signifie %massique. Dans les 7 émulsions prémix 1, la masse totale de (précurseur du tensioactif 1 de formule (LI') + co-tensioactif 1) est de 300 mg. | | | | | | | | | | | |

L'augmentation de la quantité de précurseur du tensioactif 1 de formule (LI') dans les émulsions 1 n'a pu être faite que pour des rapports molaires tensioactif 1 de formule (LI') + co-tensioactif 1) / (précurseur du tensioactif 1 de formule (LI') + co-tensioactif 1) inférieurs à 5%. Au-delà de ces rapports molaires, les émulsions 1 ne sont pas stables et les gouttelettes s'agrègent pour former un milieu très visqueux qui ne peut pas être utilisé pour le greffage de l'antigène.

**Tableau 4 : Paramètres de sonication utilisés avec un sonificateur AV505® (Sonics, Newtown, USA)**

| Sonde (φ) | Puissance Pmax | Temps de sonication | Pulse on/off |
|---|---|---|---|
| 3 mm | 28% | 20 min | 10s / 30s |

Les émulsions prémix 1 ainsi produites comprennent des gouttelettes comprenant un précurseur du tensioactif 1 de formule (LI') dont la fonction thiol est protégée par le groupe -S-Pyridinile qui ont été déprotégées pour pouvoir greffer l'anticorps de façon covalente aux gouttelettes.

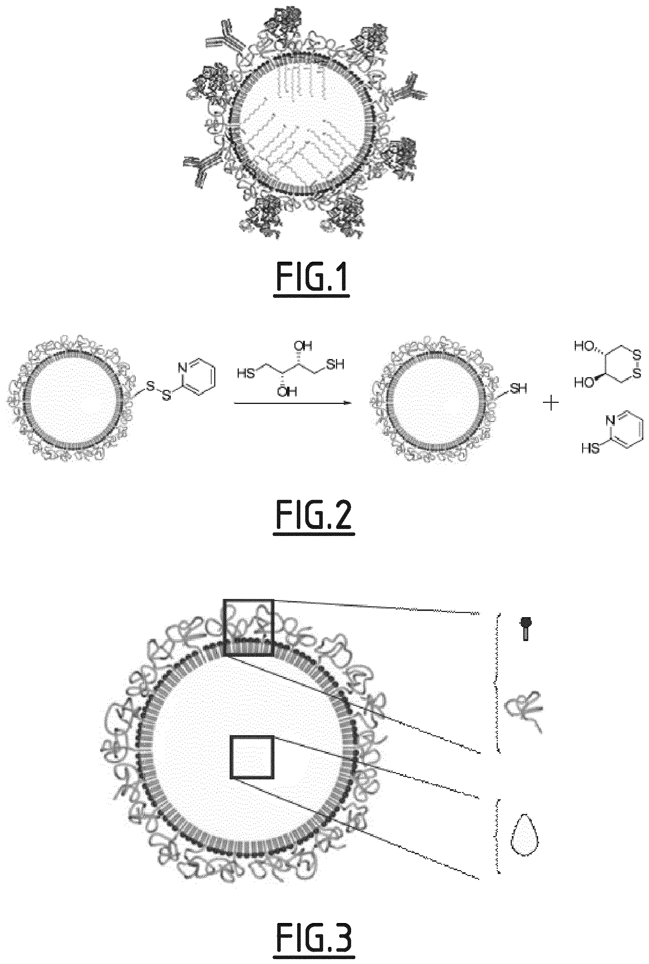

Pour ce faire, les émulsions prémix 1 ont été incubées avec 4 mg de dithiothréitol pendant 1 heure sous agitation magnétique à température ambiante. Les émulsions prémix 1 ont ensuite purifiées par dialyse (seuil de coupure : 12 kDa, contre du PBS 1X, quatre fois 1h puis une nuit) pour éliminer les composants non-intégrés aux gouttelettes ainsi que les produits secondaires issus de la déprotection, comme illustré sur la figure 2. Une coupe d'une gouttelette d'une émulsion prémix 1 est schématisée sur la figure 3.

Afin de vérifier que les tensioactifs 1 de formule (LI') étaient bien incorporés aux gouttelettes et porteurs de fonctions thiols libres, un fluorophore-maléimide (Fluoprobe 647-H maleimide d'Interchim) a été greffé sur ces fonctions thiols afin de les doser. Les résultats, fournis au tableau 5, ont démontré plus de 95% des tensioactifs 1 de formule (LI') sont incorporés dans les gouttelettes sous leur forme -SH.

**Tableau 5 : Dosage des fonctions -SH sur les gouttelettes**

| Emulsions prémix | A | B | C | D | A' | B' | C' | D' |
|---|---|---|---|---|---|---|---|---|
| Quantité de précurseur du tensioactif 1 de formule (LI') introduits initialement dans la formulation (mg) | 1 | 2 | 5 | 10 | 2,5 | 5 | 7,5 | 10 |
| %age de précurseur du tensioactif 1 de formule (LI') réellement incorporé dans les gouttelettes | 96,8 | 96,3 | 97,4 | 98,4 | 40,2 | 40,1 | 59,7 | 71,5 |
| Nombre moyen de fonctions -SH par gouttelettes théorique pour une incorporation de 1 00% | 125 | 250 | 625 | 1260 | 104 | 209 | 313 | 418 |
| Nombre moyen de fonctions -SH par gouttelettes réel tenant compte de l'incorporation réelle | 121 | 241 | 609 | 1238 | 42 | 84 | 187 | 299 |
| Taille moyenne (nm) après déprotection | 116,6 ± 2,4 | 118,4 ± 1,8 | 115,1 ± 2,7 | 112,2 ± 2,5 | 78,9 ± 0,7 | 75,9 ± 4,9 | 77,5 ± 0,5 | 74,1 ± 5,2 |

De plus, les tailles des gouttelettes des 8 émulsions prémix 1 A, B, C, D, A', B', C' et D' du tableau 5 ont été mesurées par DLS (appareil Zetasizer Nano ZS de chez Malvern Instruments, UK). Les gouttelettes des 4 émulsions prémix 1 A, B, C et D ont un diamètre de l'ordre de 120 nm. Les gouttelettes des 4 émulsions prémix 1 A', B', C' et D'ont un diamètre de l'ordre de 80 nm.

### 1.2. Greffage de l'ovalbumine sur les gouttelettes des émulsions prémix 1 préparées selon 1.1.

### 1.2.1. Préparation du composé de formule (LII') par modification chimique de l'ovalbumine pour y greffer le groupe G₂ de type maléimide

L'ovalbumine a été choisie comme antigène modèle à greffer sur les gouttelettes car elle est connue pour posséder deux épitopes de classes différentes connues pour produire une réponse cellulaire (MHC-I) et une réponse humorale (MHC-II) :
- OVA 257-264 : SIINFEKL (SEQ ID N° 1),
- OVA 323-339: ISQAVHAAHAEINEAGR (SEQ ID N° 2).

C'est une protéine globulaire de 45 kDa avec un pH isoélectrique de 4.5. Elle possède 6 fonctions cystéine dont aucune n'est accessible chimiquement sans dénaturation préalable de la protéine et 20 fonctions lysine dont 3 seulement sont accessibles chimiquement sans dénaturation préalable de la protéine (Steven et al., Biochem J., 1958, 70, 179-182).

Afin de greffer l'ovalbumine sur les fonctions thiols présentes en surface des gouttelettes des émulsions prémix 1 préparées selon le paragraphe 1.1., il a été nécessaire d'introduire une ou plusieurs fonctions maléimide sur la protéine, via un linker bifonctionnel : le Sulfosuccinimidyl 4-(N-maleimidomethyl)cyclohexane-1-carboxylate (Sulfo-SMCC) selon le schéma réactionnel suivant. Pour cela, on a fait réagir l'ovalbumine en solution dans le PBS 1X avec 10 à 50 équivalents de Sulfo-SMCC sous agitation magnétique pendant 1h à température ambiante.

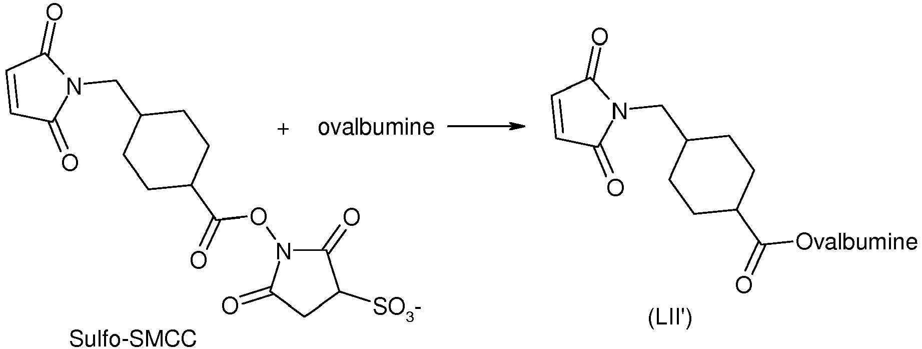

Afin de pouvoir quantifier ultérieurement le rendement de la réaction de modification chimique de l'ovalbumine, un échantillon a été prélevé et l'ovalbumine a été marquée avec un fluorophore de type NHS-ester ou isothiocyanate, par exemple du FITC, en ajoutant ce dernier au milieu réactionnel pour une heure supplémentaire.

La protéine a ensuite été séparée de l'excès de réactifs par chromatographie d'exclusion stérique sur colonne PD-10.

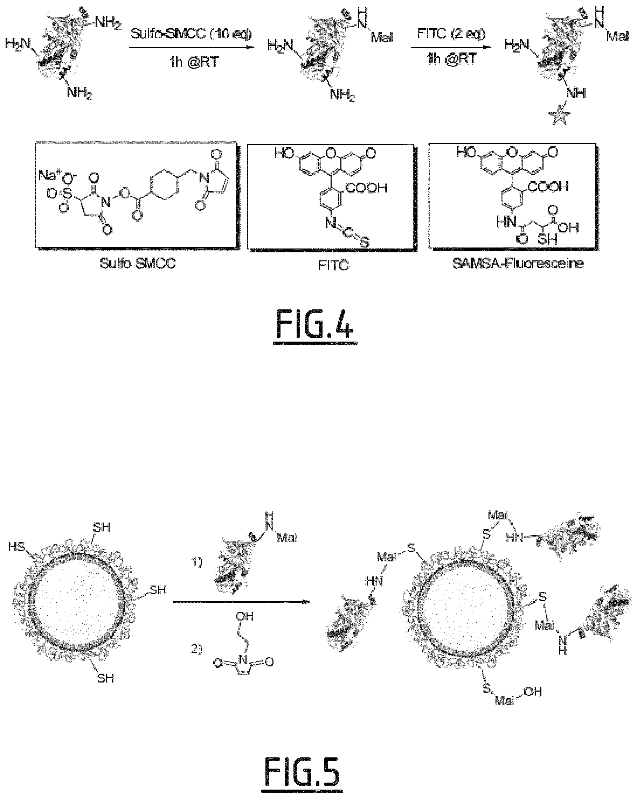

Afin de quantifier le nombre de fonctions maléimides réactives introduites sur la protéine, on a dosé ces dernières en fluorescence par réaction avec un fluorophore porteur d'une fonction thiol tel que la SAMSA-Fluoresceine comme illustré sur la figure 4.

Ce dosage en fluorescence a donné les résultats suivants pour 10 et 50 équivalents de Sulfo-SMCC :
- 10 eq. : 0,73 maléimide par ovalbumine (soit 24 % de rendement de fonctionnalisation)
- 50 eq. : 1,23 maléimide par ovalbumine (soit 41 % de rendement de fonctionnalisation) Ces conditions semblent donc idéales pour obtenir une moyenne de un maléimide par ovalbumine et donc éviter la formation de liaisons covalentes entre les gouttelettes : gouttelette-ovalbumine-gouttelette lors du greffage.

### 1.2.2. Procédé de greffage - Réaction entre le tensioactif 1 de formule (LI') porteur d'un groupe G₁ de type -SH et le composé de formule (LII') porteur d'un groupe G₂ de type maléimide.

Le composé de formule (LII') (ovalbumine fonctionnalisée par un malémide) a été purifié, en solution dans le PBS 1X, et a été placé sous agitation magnétique à 0-4°C dans un bain d'eau glacée. L'émulsion prémix 1 a alors été ajoutée lentement au goutte à goutte avec un ratio composé de formule (LII')/thiol contenu dans l'émulsion prémix 1 de 1,1/1. Le greffage a lieu par formation du tensioactif 1 de formule (I) suivante :

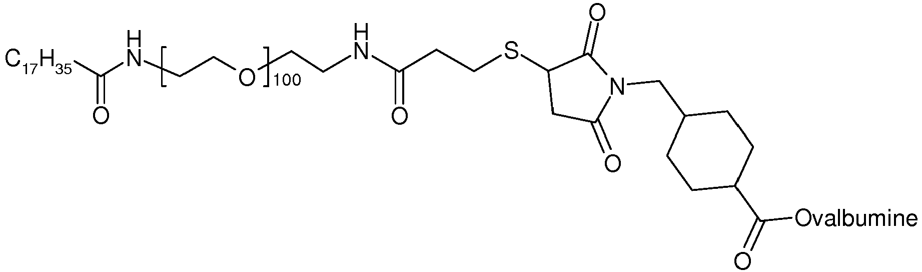

L'agitation a été maintenue plusieurs heures jusqu'à ce que la température remonte à 20°C puis on a ajouté de la 1-(2-hydroxyethyl)-1H-pyrrole-2,5-dione (Mal-OH) avec un ratio Mal-OH/thiol de 3/1 de façon à consommer les fonctions thiols n'ayant pas réagi, comme illustré sur la figure 5.

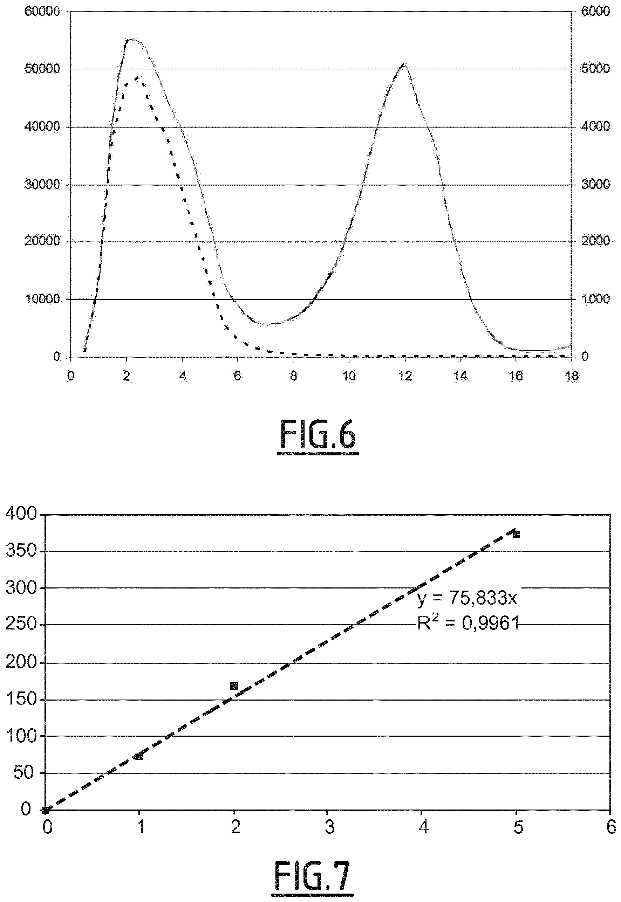

Les émulsions 1 obtenues ont alors été purifiées par chromatographie d'exclusion stérique sur résine Superdex 200 en récoltant des fractions de 500 µL après le passage du volume mort de la colonne. Les profils d'élution des gouttelettes chargées en fluorophores et de la protéine marquée ont été suivis en fluorescence. Un exemple de résultat de fluorescence est donné en Figure 6.

Le signal de fluorescence de l'ovalbumine greffée est corrélé au signal de fluorescence des gouttelettes. Le pourcentage ovalbumine greffée/ovalbumine libre a été obtenu en faisant le rapport entre les aires sous la courbe pour l'ovalbumine greffée et non greffée. On a alors calculé le nombre de moles d'ovalbumine greffée, le rendement de fonctionnalisation et le nombre moyen d'ovalbumine par gouttelette. Pour une même émulsion prémix 1, la relation entre le nombre moyen d'ovalbumine par gouttelette et la quantité de thiols disponibles est linéaire, comme le montre l'exemple de l'émulsion 1 B indiqué en figure 7.

En considérant que l'ovalbumine est une protéine globulaire de diamètre 6 nm (d'après Malvern) il a été possible de calculer le pourcentage de surface d'une gouttelette couverte par l'ovalbumine greffée. On a obtenu une couverture de surface maximale de 23%.

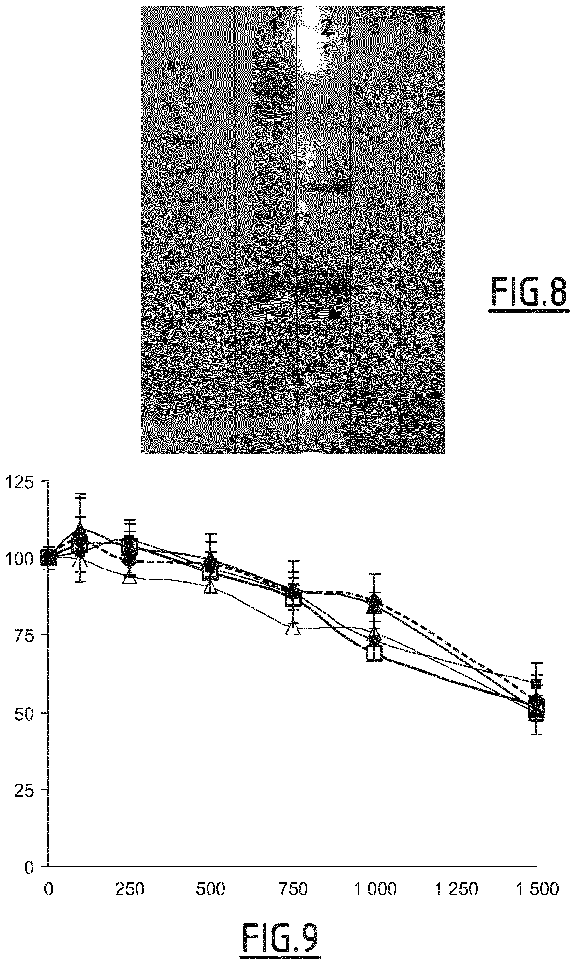

Les gouttelettes porteuses d'ovalbumine ont été analysées en SDS-PAGE après purification par chromatographie d'exclusion stérique. Aucune raie spécifique à l'ovalbumine n'apparait dans les gouttelettes purifiées, ce qui montre l'efficacité de la séparation par chromatographie d'exclusion stérique (figure 8).

### 1.2.3. Caractérisation physicochimique des émulsions 1 obtenues

Le diamètre hydrodynamique et le potentiel de surface des gouttelettes sur lesquelles l'ovalbumine a été greffée ont été déterminés par DLS/ELS (appareil Zetasizer Nano ZS de chez Malvern Instruments, UK).

Une augmentation du diamètre hydrodynamique a été observée après greffage de l'ovalbumine (Tableau 6). Cette augmentation dépend du nombre d'ovalbumine greffé sur les gouttelettes et semble atteindre un plateau.

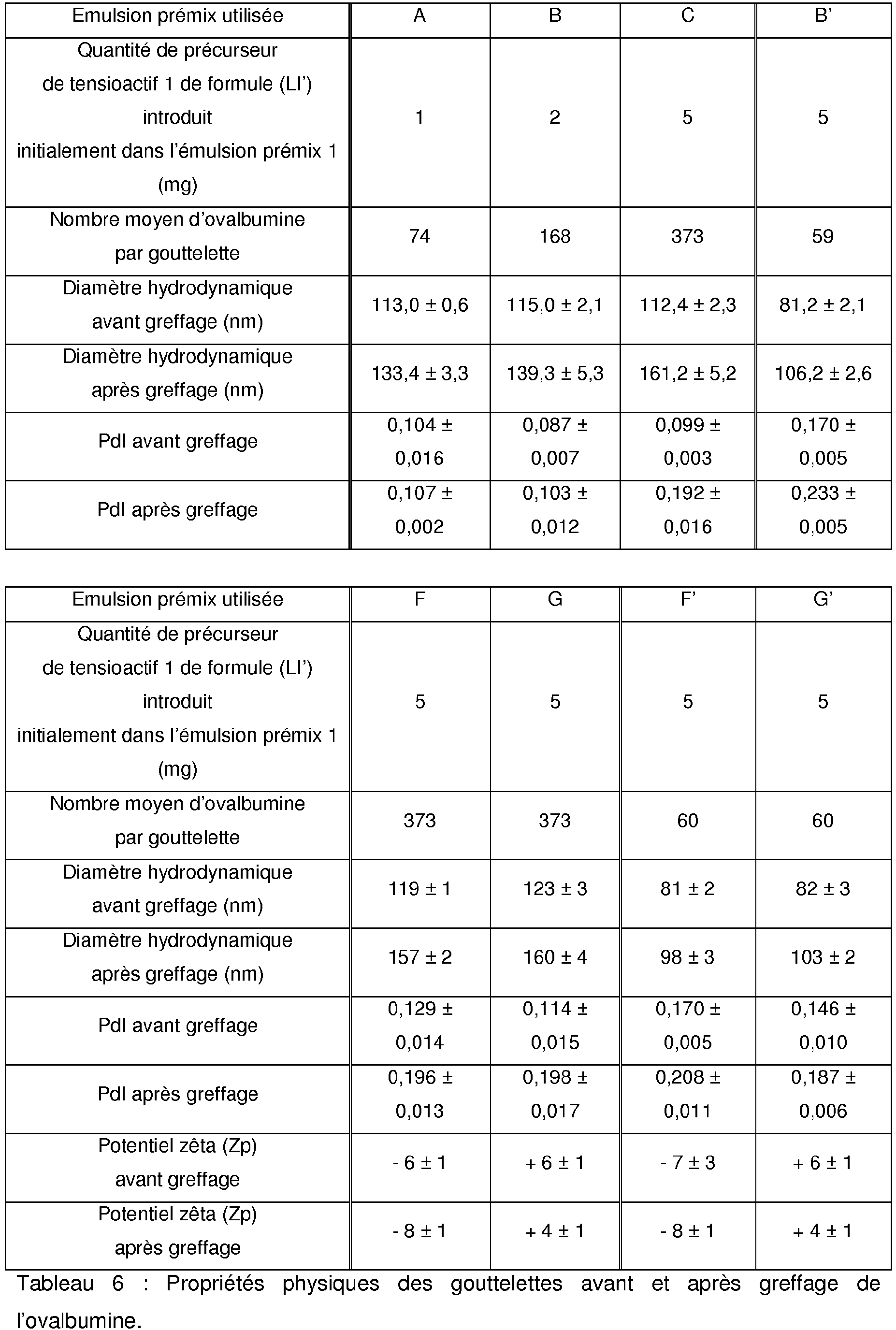

Malgré l'augmentation de taille observée après le greffage de l'ovalbumine, le diamètre hydrodynamique est resté dans une gamme de taille intéressante pour les applications en vaccination, c'est-à-dire une taille de gouttelette inférieure à 200 nm pour favoriser l'internalisation cellulaire.

En ce qui concerne le potentiel de surface des gouttelettes, il est resté le même avant et après greffage, à savoir compris entre -7 et -10 mV (mesuré dans le PBS 0.1 X).

### 1.2.4. Stabilité colloïdale des émulsions 1 obtenues

La stabilité des gouttelettes sur lesquelles l'ovalbumine a été greffée possédant le taux de charge en ovalbumine le plus élevé (émulsion 1 C greffée par l'ovalbumine obtenue en 1.2.2.) a été vérifiée en observant l'évolution du diamètre hydrodynamique, du potentiel de surface Pdl et du potentiel zeta des gouttelettes placées à 4°C pendant 150 jours. Aucune évolution de ces paramètres dans le temps n'a été observée, ce qui démontre la stabilité des émulsions 1.

### 1.2.5. Toxicité des gouttelettes sur lesquelles l'ovalbumine a été greffée

Des gouttelettes sur lesquelles l'ovalbumine a été greffée (émulsion 1 A greffée par l'ovalbumine et émulsion 1 C greffée par l'ovalbumine obtenue en 1.2.2., Figure 9 courbes avec les triangles) ont été soumises à un test de toxicité afin de vérifier leur impact sur une lignée de fibroblastes 3T3. Des émulsions exemptes de tensioactif 1 de formule (I) ou (LI') (sans ovalbumine, Figure 9 courbes avec les carrés, et sans tensioactif 1 fonctionnalisable, Figure 9 courbe trait en pointillés, losanges) ont été utilisées comme contrôle. La concentration en gouttelettes (en µg/mL) est indiquée en abscisses et la viabilité cellulaire (en % de cellules vivantes) est indiquée en coordonnées. L'IC50 déterminé par cette méthode était le même pour toutes les gouttelettes sur lesquelles l'ovalbumine a été greffée et identique à celui des émulsions contrôles. Ceci démontre que le greffage en surface de l'ovalbumine n'affecte en rien la bonne tolérance des fibroblastes 3T3 à ces dernières.

### Exemple 2 : Préparation d'une émulsion 1 comprenant un tensioactif 1 porteur d'un antigène (peptide) de formule (I')

Le peptide qui a été utilisé dans ces expériences constitue l'épitope à réponse cellulaire (MHC-I) de l'ovalbumine, soit la séquence OVA 257-264 : S-I-I-N-F-E-K-L (8 mers) SEQ ID NO :1. Ce peptide a été modifié de façon à y greffer un fluorophore : la 6-carboxy-fluorescine (Fam™), par ajout d'une lysine à l'extrémité C-terminale, et une fonction maléimide (Mal) sur l'extrémité N-terminale par le sous-traitant Smartox Biotechnology. Le peptide obtenu possède donc la séquence suivante : Mal-S-I-I-N-F-E-K-L-K-6-carboxy- fluorescine (SEQ ID NO :3).

Ce peptide a été mis à réagir avec une émulsion prémix 1 B telle que préparée dans l'exemple 1 (voir 1.1.2.) mais encapsulant en plus un agent d'intérêt 1 : un fluorophore, le DiD. La purification du mélange réactionnel par chromatographie d'exclusion stérique sur gel a alors montré un signal de fluorescence du peptide (Figure 10, trait plein) corrélé à un signal de fluorescence des gouttelettes (Figure 10, trait en pointillés). Selon les résultats de la Figure 10, on estime que chaque gouttelette porte en moyenne 181 peptides (rendement de couplage de 60%).

### Exemple 3 : Préparation d'une émulsion 1 comprenant un tensioactif 1 porteur d'un antigène (peptide) de formule (I')

Le peptide qui a été utilisé dans ces expériences constitue l'épitope à réponse humorale (MHC-II) de l'ovalbumine, soit la séquence OVA 323-339 : I-S-Q-A-V-H-A-A-H-A-E-I-N-E-A-G-R (17 mers) (SEQ ID NO :2). Le peptide Ova 323-339 a été modifié de façon à y greffer un fluorophore : la carboxytetramethylrhodamine (Tamra™) sur l'extrémité N-terminale et une fonction maléimide (Mal) sur l'extrémité C-terminale par le sous-traitant Smartox Biotechnology. Le peptide obtenu possède donc la séquence suivante : Mal-I-S-Q-A-V-H-A-A-H-A-E-I-N-E-A-G-R-carboxytetramethylrhodamine (SEQ ID NO :4). Ce peptide a été mis à réagir avec une émulsion prémix 1 B telle que préparée dans l'exemple 1 (voir 1.1.2.) mais encapsulant un fluorophore, le DiD. La purification du mélange réactionnel par chromatographie d'exclusion stérique sur gel a alors montré un signal de fluorescence du peptide (Figure 11, trait plein) corrélé à un signal de fluorescence des gouttelettes (Figure 11, trait en pointillés). Selon les résultats de la Figure 11, on estime que chaque gouttelette porte en moyenne 99 peptides (rendement de couplage de 40%).

### Exemple 4 : Préparation d'une émulsion 1 comprenant un tensioactif 1 porteur de deux antigènes (peptides) de formule (I')

Les peptides qui ont été utilisés dans ces expériences constituent l'épitope à réponse cellulaire (MHC-I) de l'ovalbumine, soit la séquence OVA 257-264 S-I-I-N-F-E-K-L (8 mers) (SEQ ID NO :1) et l'épitope à réponse humorale (MHC-II) de l'ovalbumine, soit la séquence OVA 323-339 I-S-Q-A-V-H-A-A-H-A-E-I-N-E-A-G-R (17 mers) (SEQ ID NO :2). Le peptide OVA 257-264 a été modifié de façon à y greffer un fluorophore : la 6-carboxy-fluorescine (Fam™), par ajout d'une lysine à l'extrémité C-terminale, et une fonction maléimide (Mal) sur l'extrémité N-terminale par le sous-traitant Smartox Biotechnology. Le peptide obtenu possède donc la séquence suivante : Mal-S-I-I-N-F-E-K-L-K-6-carboxy-fluorescine (SEQ ID NO :3). Le peptide Ova 323-339 a été modifié de façon à y greffer un fluorophore : la carboxytetramethylrhodamine (Tamra™) à l'extrémité N-terminale et une fonction maléimide (Mal) à l'extrémité C-terminale par le sous-traitant Smartox Biotechnology. Le peptide obtenu possède donc la séquence suivante : Mal-I-S-Q-A-V-HA-A-H-A-E-I-N-E-A-G-R-carboxytetramethylrhodamine (SEQ ID NO :4). Ces peptides sont alors mis à réagir avec une émulsion prémix 1 B telle que préparée dans l'exemple 1 (voir 1.1.2.) mais encapsulant un fluorophore, le DiD. La purification du mélange réactionnel par chromatographie d'exclusion stérique sur gel a alors montré un signal de fluorescence des peptides (Figure 12, deux courbes en traits pleins) corrélé à un signal de fluorescence des gouttelettes (Figure 12, trait en pointillés). Selon les résultats de la Figure 12, on estime que chaque gouttelette porte en moyenne 76 peptides OVA 257-264 et 51 peptides OVA 323-339 (rendement de couplage de 43% pour chacun des peptides).

### Exemple 5 : Préparation d'une émulsion 1 comprenant un tensioactif 1 porteur d'un antigène de formule (I') et un agent immunostimulant 1 : le MPLA

Le second volet consiste, après avoir greffé de façon covalente l'antigène ovalbumine dans les gouttelettes, à incorporer dans les gouttelettes un immunostimulant MPLA qui va augmenter la réponse immunitaire. Cet adjuvant a été inséré dans la couronne des gouttelettes.

### 5.1. Encapsulation du MPLA dans des émulsions prémix 1 comprenant le tensioactif 1 de formule (LI')

Le MPLA (ou lipide A) est un lipide immunostimulant, autorisé depuis 2009 par la FDA pour une utilisation chez l'homme (Cervarix, Papillomavirus, GSK). II est l'un des constituants principaux du Lipopolysaccharide (LPS) qui constitue lui-même en partie la paroi des bactéries.

Une fois purifié, le lipide A présente toujours une activité immunostimulante comme le LPS mais avec une toxicité moindre, ce qui en fait un adjuvant de premier choix. De par sa nature lipidique, il s'insère très bien à la surface des nano-objets hydrophobes tels que les nanoparticules de PLGA, ou encore les liposomes, avec des rendements d'encapsulation allant de corrects à très bons. Le MPLA est généralement encapsulé à des taux de charge massiques allant de 0,1 à 1 %, taux de charges qui suffisent à observer un effet positif sur l'activation du système immunitaire.

Pour encapsuler le MPLA dans les gouttelettes, ce dernier a été dissous dans la phase huileuse fondue avant sonication. Le MPLA a été encapsulé à des taux de charge massiques théoriques de 0,1, 0,4 et 1,3 % pour lesquels peu de modifications des propriétés physicochimiques ont été observées. La taille des gouttelettes contenant le MPLA n'augmente significativement que pour un taux de charge massique théorique de 1.3 % (Tableau 7).

**Tableau 7 : caractérisation physicochimique des gouttelettes comprenant du MPLA encapsulé (dites « LNP(MPLA) ») à différents taux de charge.**

| DLE théorique MPLA (% m/m) | Diamètre hydrodynamique (nm) | Pdl |
|---|---|---|
| 0 | 109,2 ± 0,8 | 0,104 ± 0,027 |
| 0,1 | 109,1 ± 0,4 | 0,096 ± 0,011 |
| 0,4 | 124,7 ± 4,8 | 0,154 ± 0,007 |
| 1,3 | 133,6 ± 1,1 | 0,181 ± 0,013 |

De ce fait, le taux de charge de 0,4 % a été conservé par la suite. Le dosage du MPLA par des dosages d'endotoxines via le kit LAL (Lysat de Limulus Amebocyte) montre un rendement d'incorporation de 95% soit un taux de charge de 0,38 %.

### 5.2. Greffage de l'ovalbumine sur les gouttelettes des émulsions prémix 1 préparées selon 5.1.

Par la suite, des gouttelettes contenant le MPLA à 0,4 % (m/m) et le précurseur du tensioactif 1 de formule (LI') (porteur du groupe terminal SPDP) (5 mg introduits dans la formulation) ont été préparées, activées et purifiées comme décrit dans l'exemple 1. Le greffage et la purification ont été réalisés dans les mêmes conditions. Les émulsions 1 obtenues présentent des propriétés physicochimiques identiques aux émulsions 1 préparées sans MPLA.

Le greffage de l'ovalbumine sur les gouttelettes comprenant du MPLA encapsulé (dites « LNP(MPLA) ») a alors été réalisé comme décrit dans l'exemple 1 et les gouttelettes comprenant du MPLA et de l'ovalbumine (dites « LNP-MPLAOva ») obtenues possèdent des propriétés physicochimiques proches des LNP-Ova.

La réaction de greffage a conduit à un nombre moyen d'ovalbumine par LNP(MPLA) de 413, proche des 373 obtenus dans les mêmes conditions pour les LNP sans MPLA selon l'exemple 1.

### 5.3. Stabilité colloïdale des LNP(MPLA)-Ova

L'étude de la stabilité à 4°C de ces LNP(MPLA)-Ova n'a pas montré de modification particulière au niveau des caractéristiques physicochimiques et confirme que la modification de surface par l'ovalbumine ainsi que l'intercalation du lipide A au sein de la couronne des gouttelettes ne diminue pas leur stabilité colloïdale (Tableau 8).

**Tableau 8 : Stabilité des LNP(MPLA)-Ova conservées à 4°C**

| Temps à 4°C (jours) | Diamètre hydrodynamique (nm) | Pdl |
|---|---|---|
| 0 | 148,6 ± 3,0 | 0,120 ± 0,011 |
| 50 | 151,1 ± 4,1 | 0,147 ± 0,005 |

### Exemple 6 : Ciblage des cellules immunes par une émulsion 1 comprenant un agent biologique de ciblage 1 : le Mannan (LNP-Mannan)

Le mannan est un polymère linéaire de mannose. Il est produit par *Saccharomyces Cerevisiae* et ne possède pas de masse molaire moyenne définie.

Le mannan a été fonctionnalisé de la même façon que l'ovalbumine (voir 1.2.1) et mis à réagir avec une émulsion prémix 1 B (voir 1.1). Les gouttelettes obtenues possèdent un taux de charge en Mannan de 0,5% et ont été testées. Pour ce taux de charge, aucune modification de taille ou de surface significative n'est apparue.

Des macrophages ont été incubés avec des LNP-Mannan contenant un fluorophore (DiD) puis la fluorescence au sein des cellules a été mesurée par cytométrie en flux après une heure et trois heures d'incubation. Les résultats montrent que les gouttelettes porteuses de Mannan sont significativement plus captées par les macrophages après une heure d'incubation (Figure 9 et tableau 9). De plus, au bout de 3h d'incubation, l'intensité moyenne de fluorescence dans les cellules en contact avec les LNP-Mannan est significativement plus importante, ce qui montre que ces cellules ont captés plus de gouttelettes que les cellules mises en contact avec les gouttelettes contrôles.

**Tableau 9 : résultats de capture cellulaire sur les macrophages**

| | Proportion de cellules positives après 1h d'incubation | Proportion de cellules positives après 3h d'incubation | Intensité moyenne de fluorescence par cellule après 1h d'incubation | Intensité moyenne de fluorescence par cellule après 3h d'incubation |
|---|---|---|---|---|
| Contrôle | 0,3 ± 0,01 % | 73,2 ± 0,7 % | 1873 ± 225 | 1843 ± 25 |
| Mannan | 43,5 ± 0,6 % | 85,2 ± 1,5 % | 1442 ± 44 | 2593 ± 36 |

### Exemple 7 : Préparation d'une émulsion 1 comprenant un tensioactif 1 porteur d'un antigène (ovalbumine) de formule (I') et un tensioactif 1 porteur d'un anticorps de formule (I')

Une possibilité pour cibler les cellules du système immunitaire a été de réaliser un greffage covalent d'anticorps sur les fonctions thiols du tensioactif 1 de formule (LI') tout en maintenant la possibilité de greffer l'ovalbumine de façon covalente aux gouttelettes par la suite.

Pour se faire, un anticorps modèle fonctionnalisé par le Sulfo-SMCC, le Cetuximab, et portant un fluorophore A, l'Alexa700-NHS, a été mis à réagir avec une émulsion prémix 1 telle que préparée dans l'exemple 1 mais encapsulant en plus un fluorophore B, le DiO, dans un rapport stœchiométrique Cetuximab/SH 1/10, tel que seule une faible proportion des fonctions thiols sera occupée par l'anticorps. Le milieu réactionnel a alors été mis à réagir, sans purification préalable, avec l'ovalbumine modifiée chimiquement pour porter :
- un groupe maléimide permettant le greffage aux fonctions thiols du tensioactif 1 de formule (LI') restant (modification chimique comme dans l'exemple 1 (voir 1.2.1.)) et
- un fluorophore C, le Cy5-NHS.

Les fluorophores A, B et C ont été choisis de telle manière qu'il n'était pas possible d'avoir de transfert d'énergie entre eux. La purification du mélange réactionnel final par chromatographie d'exclusion stérique sur gel a alors montré l'existence de gouttelettes bi-fonctionnalisées porteuses à la fois d'anticorps et d'ovalbumine (signal de fluorescence A, Figure 13, courbe en trait plein fin) corrélé à un signal de fluorescence B, Figure 13, courbe en trait en pointillés et à un signal de fluorescence C, Figure 13, courbe en trait plein épais), comme illustré en figure 13. Selon les résultats de la Figure 13, on estime que chaque gouttelette porte en moyenne 44 anticorps (rendement de couplage de 80 %) et 244 ovalbumines (rendement de couplage de 42 %).

### Exemple 8 : Utilisation biologique de l'émulsion 1 selon l'exemple 1 Immunisation - validation in vivo des gouttelettes sur lesquelles l'ovalbumine a été greffée

Ci-après, les gouttelettes sur lesquelles l'ovalbumine a été greffée obtenues dans l'exemple 1 sont nommées « LNP-OVA ».

Des souris BALB de 8 semaines ont reçu une première injection de LNP-Ova avec ou sans LPS. Les contrôles négatifs sont obtenus en injectant l'ovalbumine libre avec ou sans LPS ainsi que l'ovalbumine libre accompagné de gouttelettes LNP « nues » (c'est à dire sans protéine greffée à leur surface) avec ou sans LPS. Au bout de 21 jours, une deuxième injection de rappel est effectuée et les souris sont sacrifiées au 28ème jour. Les taux d'anticorps Anti-Ova dans les *sera* sont alors déterminés par ELISA.

Pourcentage d'anticorps anti-ovalbuline (%OVA) en fonction de la composition utilisée (figure 14)

Les gouttelettes LNP sont celles de l'émulsion 1 greffée par l'ovalbumine obtenue à l'exemple 1.2.2

L'immunisation avec l'ovalbumine libre sans LPS donne une réponse immunitaire faible normalisée à 100% (OVA, Figure 14). L'ajout d'un adjuvant vétérinaire classique (CFA : adjuvant complet de Freund) à l'injection d'ovalbumine donne une réponse immunitaire plus forte mais avec une forte hétérogénéité interindividuelle (OVA+CFA, Figure 14). Un contrôle négatif a été réalisé en injectant des gouttelettes nues avec l'ovalbumine libre, avec ou sans adjuvant (LNP+OVA, LNP+OVA+LPS, Figure 14). Les réponses observées sont les mêmes que pour OVA et OVA+CFA, ce qui montre que les gouttelettes nues sont très bien tolérées et n'activent pas le système immunitaire. Finalement, l'injection de - LNP-Ova conduit à une réponse immunitaire significativement plus importante que l'ovalbumine libre (LNP-OVA vs OVA, Figure 14) et du même ordre de grandeur que les réponses obtenues avec l'ovalbumine libre et adjuvants. Ce résultat démontre que la simple vectorisation de l'ovalbumine par les gouttelettes produit un effet similaire à l'ajout d'un adjuvant. Pour rappel, les LNP-Ova ne contiennent aucun adjuvant. L'ajout d'un adjuvant en plus des LNP-Ova augmente encore plus et de façon significative la réponse (LNP-OVA+LPS, Figure 14). En conclusion, ces expériences d'immunisation valident l'intérêt de la vectorisation de l'antigène ovalbumine par l'émulsion 1.

Proportion Ig total anti-OVA (ng/mL) en fonction de la composition utilisée (figure 15)

Pour chaque composition, 50 µg d'ovalbumine (greffée ou non aux gouttelettes d'émulsion) ont été injectés. La quantité d'ovalbumine injectée est donc la même pour toutes les compositions injectées.

L'immunisation avec l'ovalbumine libre sans LPS a donné une réponse immunitaire faible (OVA, Figure 15).

L'ajout d'un adjuvant utilisé classiquement dans le domaine vétérinaire (CFA : adjuvant complet de Freund) à l'injection d'ovalbumine a induit une réponse immunitaire plus importante que celle de la protéine administrée seule, caractérisée par un taux plus important d'anticorps circulants anti-ovalbumine (OVA+CFA, Figure 15).

Dans tous les cas, l'injection de LNP-OVA (c'est-à-dire les émulsions 1 présentant de l'ovalbumine greffée de façon covalente en surface) a provoqué une réponse immunitaire significativement plus importante que celles induites par l'ovalbumine libre ou par l'ovalbumine dans le CFA (Figure 15).

En particulier, le taux d'anticorps anti-ovalbumine le plus élevé pour une même dose administrée d'ovalbumine est observé suite à l'injection de l'émulsion 1 F' greffée par l'ovalbumine obtenue à l'exemple 1.2.2, caractérisée notamment par une moindre variabilité inter-individuelle de réponse.

Pour les formulations de gouttelettes neutres (émulsions 1 F et F' greffées par l'ovalbumine obtenue à l'exemple 1.2.2, exemptes de lipide cationique), la taille semble être un critère prépondérant dans le niveau de réponse induite, dans la mesure où l'émulsion 1 F greffée par l'ovalbumine obtenue à l'exemple 1.2.2 (diamètre 157 nm) a induit une réponse significativement :
- plus importante que OVA+CFA et
- moins importante que celle obtenue avec l'émulsion 1 F' greffée par l'ovalbumine obtenue à l'exemple 1.2.2 (diamètre 98 nm).

En revanche, les formulations de gouttelettes cationiques (émulsions 1 G et G' greffées par l'ovalbumine obtenue à l'exemple 1.2.2, comprenant le lipide cationique DOTAP), induisent une réponse humorale de même ordre, qui est toutefois plus importante que celle obtenue avec OVA+CFA (Figure 15). Dans ce cas précis, la taille ne semble pas être un facteur déterminant.

Ces résultats démontrent que la vectorisation de l'ovalbumine par les gouttelettes des émulsions 1 potentialise les réponses immunes. En effet, il est important d'observer que les réponses immunes induites sont très faibles lorsque la protéine antigénique OVA est administrée dans une solution contenant des gouttelettes nues (sur lesquelles OVA n'est pas greffée de façon covalente) et le LPS (LNP+OVA+LPS, figure 15), ce qui souligne l'importance de l'existence de la liaison covalente entre l'ovalbumine et les gouttelettes. En outre, les rates des souris ont été prélevées au sacrifice, dissociées et les splénocytes ainsi récoltés ont été remis en culture et réexposés à la protéine ovalbumine. Puis, les surnageants ont été collectés et les cytokines ont été dosées par la technique CBA (pour cytometry beads assay).

Les résultats ont montré que l'émulsion 1 F' greffée par l'ovalbumine obtenue à l'exemple 1.2.2 utilisée dans le protocole d'immunisation est celle qui induit la plus forte sécrétion de cytokines IL-17, INFγ, IL10 et TNFα, comparée aux autres d'émulsion 1 F, G et G' greffées par l'ovalbumine obtenue à l'exemple 1.2.2 et à l'ovalbumine seule (OVA) ou ovalbumine dans le CFA (OVA+CFA) (tableau 10).

Il est intéressant de noter que la sécrétion d'IFNγ est également plus importante, comparativement à l'ovalbumine dans le CFA (OVA+CFA) lorsque l'émulsion 1 F greffée par l'ovalbumine obtenue à l'exemple 1.2.2 a été utilisée dans le protocole d'immunisation. De façon similaire, le taux de TNFα est plus élevé lorsque l'émulsion 1 G greffée par l'ovalbumine obtenue à l'exemple 1.2.2 a été utilisée dans le protocole d'immunisation.

**Tableau 10: Proportions de cytokines IL-17, INFγ, IL10 et TNFα mesurées dans les rates des souris**

| | OVA (comparatif) | OVA+CFA (comparatif) | OVA + LNP + LPS (comparatif) | LNP(F) - OVA + LPS | LNP(F') - OVA + LPS | LNP(G) - OVA + LPS | LNP(G') -OVA + LPS |
|---|---|---|---|---|---|---|---|
| IL-10 (pg/mL) | 22 ± 21 | 31 ± 11 | 16 ± 7 | 27 ± 11 | 54 ± 26 | 34 ± 25 | 21 ± 13 |
| IL-17 (pg/mL) | 133 ± 31 | 217 ± 177 | 16 ± 8 | 275± 161 | 415 ± 209 | 196 ± 144 | 180 ± 69 |
| TNFα (pg/mL) | 73 ± 36 | 151 ± 54 | 150 ± 66 | 174 ± 38 | 315 ± 126 | 324 ± 148 | 161 ± 45 |
| IFN-γ (pg/mL) | 22 ± 34 | 19 ± 20 | 4 ± 5 | 79 ± 41 | 213 ± 120 | 41 ± 31 | 35 ± 28 |

### Exemple 9 : Préparation d'une émulsion 2 comprenant du MPLA comme agent immunostimulant 2

Une émulsion 2 comprenant du MPLA à titre d'agent immunostimulant 2 a été préparée en suivant les procédures décrites dans WO 2010/018223 avec les compositions indiquées dans le tableau 11. Les phases aqueuse et huileuse ont alors été mélangées puis émulsifiées par sonication selon les paramètres décrits dans le tableau 4. A titre comparatif, une émulsion exempte d'agent immunostimulant a également été préparée dans les mêmes conditions. Les deux émulsions avaient un diamètre de gouttelettes mesuré au Zetasizer Malvern de 80 nm.

**Tableau 11 : Formulation d'une émulsion 2 comprenant du MPLA comme agent immunostimulant 2 et d'une émulsion comparative exempte d'agent immunostimulant.**

| | Lipide amphiphile 2 lécithine S75 (Lipoïd) (mg) | Lipide solubilisant 2 Suppocire® NB (Gattefossé) (mg) | Huile 2 de soja (Croda) (mg) | PBS 1X (µL) | glycérol (agent épaississant) (mg) | co-tensioactif 2 MYRJ S40 (CRODA) (PEG 40) (mg) | agent immunostimulant 2 (MPLA) (COGER-Distributeur d'AVANTI POLAR LIPIDS) (mg) |
|---|---|---|---|---|---|---|---|
| émulsion 2 (MPLA) | 7,6 | 75,6 | 24,8 | 1250 | 960 | 44 | 4 |
| comparatif | 7,6 | 75,6 | 24,8 | 1250 | 960 | 44 | 0 |

### Exemple 10 : Utilisation d'une émulsion 2 comprenant du MPLA comme agent immunostimulant 2 pour activer des cellules dendritiques

Les émulsions préparées à l'exemple 9, ainsi que du MPLA libre (contrôle) ont été testés sur des cultures primaires de cellules dendritiques (cellules isolées de moelle osseuse de souris).

L'activation des cellules dendritiques a été suivie par marquage anti-CD86 et analyse au cytomètre de flux (appareil BD LSR2 2 lasers 488/633nm).

Les résultats sont illustrés sur la figure 16. On a observé qu'après une incubation de 1 jour des cellules dendritiques en présence de l'émulsion comparative exempte d'agent immunostimulant, celle-ci n'active pas les cellules dendritiques, alors que l'émulsion 2 comprenant du MPLA les active au même niveau que l'activation obtenue avec le MPLA libre (contrôle).

### Exemple 11 : Préparation d'une émulsion 2 comprenant l'ODN CpG comme agent immunostimulant 2

L'émulsion A3 du tableau 1 de l'exemple de la demande WO 2014/032953 comprenant du Lipoid à titre de lipide amphiphile 2, du DOTAP à titre de tensioactif cationique 2, du Myrj S40 à titre de co-tensioactif 2, du DOPE à titre de lipide fusogène 2, de la Suppocire NB à titre de lipide solubilisant 2, de l'huile de soja à titre d'huile 2, a été reproduite en suivant les conditions opératoire décrites dans cette demande. Les gouttelettes de la phase dispersée 2 avaient un diamètre de 45 nm.

L'ODN CpG (Invivogen) a alors été complexé sur cette émulsion A3 en suivant la procédure décrite au paragraphe « Complexation avec des siARN : Préparation de formulations « finales » comprenant des séquences nucléotiques siARN » :
- sauf que de l'ODN CpG a été utilisé à la place du SiARN,
- avec un ratio N/P (rapport de la quantité de charges positives du tensioactif cationique 2 dans la formulation A3 sur la quantité de charge négative des groupes phosphate de l'ODN CpG) de 24/1,
- avec une durée d'agitation du mélange émulsion A3 / ODN CpG de 20 min.

### Exemple 12 : Préparation d'une composition immunogène selon l'invention comprenant une phase dispersée 1 comprenant un tensioactif 1 porteur d'un antigène et une phase dispersée 2 comprenant un agent immunostimulant 2

L'émulsion de diamètre de gouttelettes de 80 nm obtenue à l'exemple 1.2.2. en greffant de manière covalente l'ovalbumine à l'émulsion prémix 1 C' du tableau 3 de l'exemple 1 a été utilisée à titre d'émulsion 1. Un fluorophore (DiD de Invitrogen à 0,11% en poids par arpport au poids de phase dispersée 1) avait été ajouté dans la phase huileuse de l'émulsion prémix 1 C'.

L'émulsion de l'exemple 11 comprenant l'ODN CpG comme agent immunostimulant 2 a été utilisé à titre d'émulsion 2.

La composition immunogène a été préparée en mélangeant cette émulsion 1 et cette émulsion 2 dans des proportions telles que le ratio massique ovalbumine / ODN CpG était de 1/1.

### Exemple 13 : Utilisation biologique de la composition immunogène selon l'exemple 12

### Immunisation - validation in vivo de la composition immunogène comprenant une phase dispersée 1 comprenant un antigène et une phase dispersée 2 comprenant un agent immunostimulant

### 13.1. Protocole d'immunisation

Le potentiel de la composition immunogène de l'exemple 12 à induire des réponses immunitaires importantes et durables a été évalué *in vivo* en immunisant des souris selon le procédé suivant. Les souris choisies étaient des Balb/c. La première injection a eu lieu lorsque les souris étaient âgées de 6 semaines. Une injection rappel a été réalisée 2 à 3 semaines plus tard. Le volume de composition immunogène était calculé pour qu'à chaque injection, chaque souris reçoive au total 20µg d'antigène et 20µg d'immunostimulant.

### 13.2. Prélèvement du matériel biologique

Une semaine après la dernière injection, les souris ont été euthanasiées par dislocation cervicale. Après dissection de l'animal, environ 200µL de sang ont rapidement été prélevés par ponction cardiaque. Le sang a été laissé à décanter à 4°C pendant une journée afin de séparer le sérum des globules rouges. Le sérum a alors été prélevé et congelé pour analyse ultérieure.

Lors de la dissection, la rate a également été extraite. Les splénocytes ont été isolés par dilacération et ont subi un traitement d'1min au tampon RBC (Red Blood Cell Lysis buffer) afin de lyser les globules rouges et ne récupérer par centrifugation que les cellules d'intérêt, à savoir les cellules dendritiques et les lymphocytes. Ces cellules isolées du système immunitaire ont été mises en culture à 37°C dans du milieu RPMI complété avec 10% de sérum de veau fœtal, 1% d'acides aminés non essentiels, 1% de pénicilline streptomycine et 1% de sodium-pyruvate (Gibco). La culture a été faite en plaques 12 puits avec 500µL par puit, 10⁶ cellules/mL et a duré 3 jours. Chaque échantillon de cellules a été doublement mis en culture afin de comparer l'exposition ou non à l'antigène (0 ou 10µg par puit). Après le délai de 3 jours, les plaques ont été centrifugées afin de séparer les cellules du surnageant (contenant les molécules sécrétées par les cellules), ce dernier étant récupéré et congelé pour analyse ultérieure.

### 13.3. Analyse des réponses immunitaires

La réponse immunitaire induite par la vaccination a été évaluée sous deux angles : la réponse immunitaire humorale (production d'anticorps spécifiques de l'antigène) et la réponse immunitaire cellulaire (conduisant à l'activation des cellules CD8+ tueuses qui ciblent les cellules de l'organisme infectées par le pathogène).

Dans un premier temps, la réponse humorale a été mesurée par dosage ELISA des anticorps spécifiques de l'antigène ovalbumine dans le sérum des souris. Trois groupes ont été testés :
- administration de la composition immunogène selon l'invention,
- co-administration de l'émulsion 1 (émulsion obtenue à l'exemple 1.2.2. en greffant de manière covalente l'ovalbumine à l'émulsion prémix 1 C' du tableau 3 de l'exemple 1) et de l'immunostimulant libre (comparatif)
- Co-administration de l'antigène libre et de l'immunostimulant libre (contrôle).

Les résultats sont présentés sur la figure 17. L'administration de la composition immunogène selon l'invention, qui comprend un antigène vectorisé dans la phase dispersée 1 et un agent immunostimulant vectorisé dans la phase dispersée 2 a permis d'obtenir un taux d'anticorps spécifiques de l'ovalbumine :
- plus de 2 fois supérieur à la co-administration de l'émulsion 1 et de l'immunostimulant libre, et
- 65 fois supérieur à la co-administration de l'antigène libre et de l'immunostimulant libre.

Dans un deuxième temps, la réponse immunitaire cellulaire a été mesurée par dosage des interferons gamma (IFNg) présents dans les surnageants de la culture des splénocytes. Ces IFNg sont des cytokines sécrétées par les cellules du système immunitaire, ce qui conduit à une activation de la réponse immune des cellules T CD8+. Les résultats sont présentés comme la différence de taux d'IFNg entre l'échantillon exposé à l'ovalbumine pendant la culture et l'échantillon contrôle, qui correspond aux cellules de rate remises en culture et non exposées de nouveau à l'antigène spécifique, OVA par exemple.

Les résultats sont présentés sur la figure 18. L'administration de la composition immunogène selon l'invention, qui comprend un antigène vectorisé dans la phase dispersée 1, c'est-à-dire l'ovalbumine, et un agent immunostimulant vectorisé dans la phase dispersée 2, c'est-à-dire le CpG, induit une sécrétion d'IFNg :
- 3 fois supérieure à la co-administration de l'émulsion 1 et de l'immunostimulant libre
- 3 fois supérieure à la co-administration de l'antigène libre et de l'immunostimulant libre.

La réponse immunitaire a donc été améliorée par l'administration la composition immunogène selon l'invention, qui comprend un antigène vectorisé dans la phase dispersée 1 et un agent immunostimulant vectorisé dans la phase dispersée 2, et ce à la fois d'un point de vue quantitatif (nombre d'anticorps spécifiques produits) mais également d'un point de vue qualitatif (induction d'une réponse immunitaire cellulaire élevée, habituellement très difficile à obtenir).

## Revendications

1. Composition immunogène comprenant une phase aqueuse continue et au moins deux phases dispersées 1 et 2 sous forme de gouttelettes, où :
- la phase dispersée 1 comprend :
- un lipide amphiphile 1,
- un lipide solubilisant 1 comprenant au moins un glycéride d'acide gras,
- un co-tensioactif 1 comprenant au moins une chaine composée de motifs d'oxyde d'alkylène,
- un tensioactif 1 porteur d'un antigène de formule (I) suivante :
(L₁-X₁-H₁-Y₁)ᵥ-G-Z₁-Ag (I),
dans laquelle :
- L₁ représente un groupe lipophile,
- X₁, Y₁, Z₁ et G représentent indépendamment un groupe de liaison,
- H₁ représente un groupe hydrophile comprenant une chaîne polyalcoxylée,
- v est un nombre entier de 1 à 8, et
- Ag représente un antigène,
dans laquelle le rapport molaire du tensioactif 1 porteur d'un antigène de formule (I) sur la somme du co-tensioactif 1 et du tensioactif 1 porteur d'un antigène de formule (I) est de 0,01 % à 5%, et
- la phase dispersée 2 comprend :
- un lipide amphiphile 2,
- un lipide solubilisant 2 comprenant au moins un glycéride d'acide gras,
- un co-tensioactif 2 comprenant au moins une chaine composée de motifs d'oxyde d'alkylène,
- un agent immunostimulant 2 porteur d'au moins un groupe anionique, et
- un tensioactif 2 cationique,
sous réserve que la phase dispersée 2 soit exempte de tensioactif porteur d'un antigène de formule (I).

2. Composition immunogène selon la revendication 1, dans laquelle, dans la formule (I) :
- L₁ est choisi parmi :
- un groupe R ou R-(C=O)-, où R représente une chaîne hydrocarbonée linéaire comprenant de 7 à 23 atomes de carbone,
- un ester ou un amide d'acides gras comprenant de 8 à 24 atomes de carbone et de phosphatidyléthanolamine, tel que le distéaryl phosphatidyléthanolamine (DSPE), et
- un poly(oxyde de propylène), et/ou
- X₁, Y₁ et Z₁ sont indépendamment choisis parmi :
- une liaison simple,
- un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
- un groupe Alk étant un alkylène comprenant de 1 à 8 atomes de carbone comprenant éventuellement un cycle (par exemple un radical 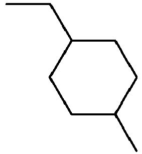 ), et
- un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents, et/ou
- H₁ est choisi parmi un poly(oxyde d'éthylène) comprenant typiquement de 3 à 500 unités oxyde d'éthylène, de préférence de 20 à 200 unités oxyde d'éthylène, et/ou
- G comprend au moins un groupe G' ayant l'une des formules suivantes : 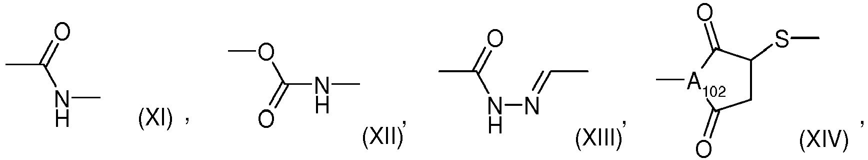 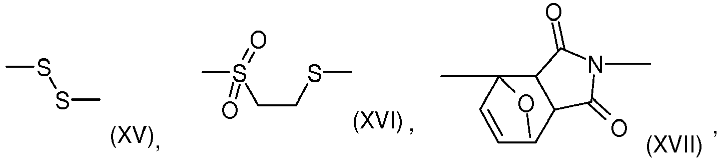 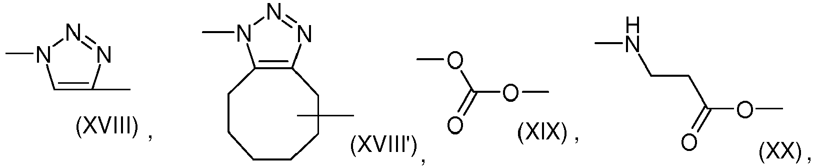 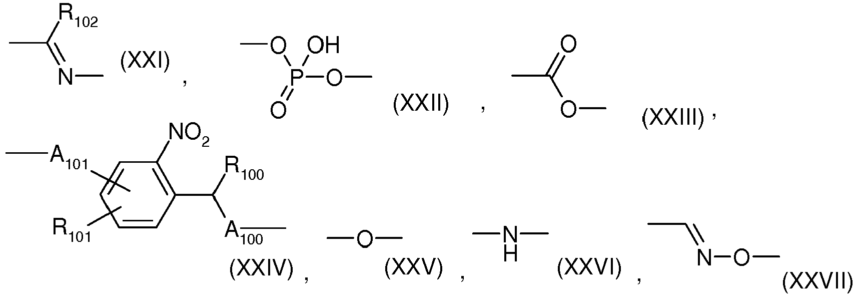 où A₁₀₂ représente CH ou N, R₁₀₂ représente H ou une chaîne hydrocarbonée linéaire comprenant de 1 à 6 atomes de carbone, A₁₀₁ représente -O-, -NH-(CO)- ou -O-(CO)-, R₁₀₀ représente H ou un méthyle, A₁₀₀ représente -O- ou -NH- et R₁₀₁ représente H, Me ou -OMe.

3. Composition immunogène selon la revendication 1 ou 2, dans laquelle, dans la formule (I), le radical L₁-X₁-H₁- consiste en l'un des groupes de formules suivantes : 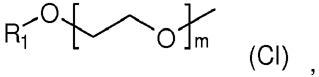 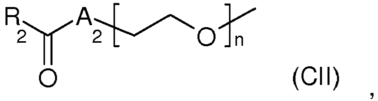 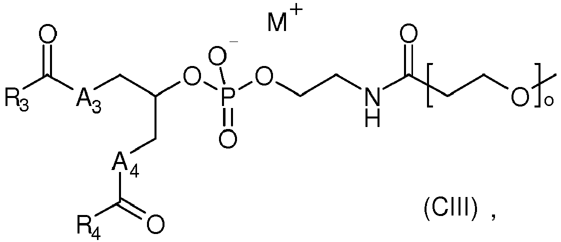 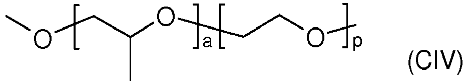 dans lesquelles :
- R₁, R₂, R₃ et R₄ représentent indépendamment une chaîne hydrocarbonée linéaire comprenant de 7 à 23 atomes de carbone,
- A₂, A₃ et A₄ représentent O ou NH,
- m, n, o et p représentent indépendamment des nombres entiers de 3 à 500, de préférence 20 à 200, et
- a représente un nombre entier de 20 à 120,
- M représente H ou un cation.

4. Composition immunogène selon l'une quelconque des revendications 1 à 3, dans laquelle le tensioactif 1 de formule (I) a la formule (I') suivante : 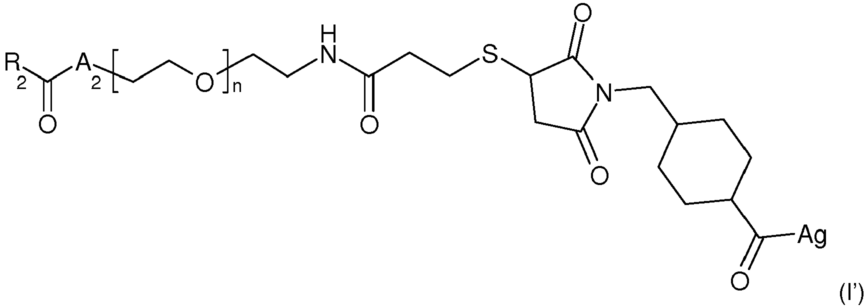 dans laquelle :
- R₂ représente une chaîne hydrocarbonée linéaire comprenant de 7 à 23 atomes de carbone, de préférence 17,
- A₂ représente O ou NH, de préférence NH, et
- n représente un nombre entier de 3 à 500, de préférence de 20 à 200, notamment 100,
- Ag représente un antigène.

5. Composition immunogène selon l'une quelconque des revendications 1 à 4, dans laquelle :
- le lipide amphiphile 1 et/ou le lipide amphiphile 2 est(sont) un phospholipide, et/ou
- le lipide solubilisant 1 et/ou le lipide solubilisant 2 est(sont) constitué(s) d'un mélange de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18, et/ou
- le co-tensioactif 1 et/ou le co-tensioactif 2 est(sont) choisi(s) parmi les composés conjugués polyéthylèneglycol/phosphatidyl-éthanolamine, les éthers d'acide gras et de polyéthylèneglycol, les esters d'acide gras et de polyéthylèneglycol et les copolymères blocs d'oxyde d'éthylène et d'oxyde de propylène, et la chaîne polyalcoxylée du co-tensioactif 1 et/ou du co-tensioactif 2 comprend de 10 à 200 motifs oxyde d'éthylène/oxyde de propylène.

6. Composition immunogène selon l'une quelconque des revendications 1 à 5, dans laquelle l'agent immunostimulant 2 est de type séquence nucléotidique et le co-tensioactif 1 et/ou 2 comprend au moins une chaîne poly(oxyde d'éthylène) comprenant au moins 25 unités d'oxyde d'éthylène.

7. Composition immunogène selon l'une quelconque des revendications 1 à 6, dans laquelle l'agent immunostimulant 2 est une séquence nucléotidique mono ou double brin, comprenant moins de 200 bases pour une séquence nucléotidique mono brin ou moins de 200 paires de base pour une séquence nucléotidique double brin, de préférence l'oligodéoxynucléotide CpG.

8. Composition immunogène selon l'une quelconque des revendications 1 à 7, dans laquelle :
- la phase dispersée 1 comprend un ligand biologique de ciblage 1 greffé ou non sur le co-tensioactif 1 et/ou la phase dispersée 2 comprend un ligand biologique de ciblage 2 greffé ou non sur le co-tensioactif 2, et/ou
- la phase dispersée 1 comprend un agent d'intérêt 1 choisi parmi un agent optique tel que un colorant, un chromophore, un fluorophore ou un agent physique, tel qu'un isotope radioactif et/ou la phase dispersée 2 comprend un agent d'intérêt 2 choisi parmi un agent optique tel que un colorant, un chromophore, un fluorophore ou un agent physique, tel qu'un isotope radioactif ou un photo-sensibilisateur.

9. Composition immunogène selon l'une quelconque des revendications 1 à 8, dans laquelle le tensioactif 2 cationique est choisi parmi choisi parmi le N[1-(2,3-dioléyloxy)propyl]-*N,N,N*-triméthylammonium, le 1,2-dioleyl-3-trimethylamonium-propane, le *N*-(2-hydroxyethyl)-*N,N*-dimethyl-2,3-bis(tetradecyloxy-1-propananium), le 1-[2-(oleoyloxy)ethyl]-2-oleyl-3-(2-hydroxyethyl)imidazolinium, et le dioctadecylamidoglycylspermine,.

10. Procédé de préparation de la composition immunogène selon l'une quelconque des revendications 1 à 9, comprenant le mélange d'une émulsion 1 comprenant une phase aqueuse continue et au moins une phase dispersée 1 telle que définie dans l'une quelconque des revendications 1 à 5 et 8, et d'une émulsion 2 comprenant une phase aqueuse continue et au moins une phase dispersée 2 telle que définie dans l'une quelconque des revendications 1 et 5 à 9.

11. Composition immunogène selon l'une quelconque des revendications 1 à 9 pour son utilisation en tant que médicament ou vaccin.

12. Composition immunogène selon l'une quelconque des revendications 1 à 9 pour induire une réponse immune contre ledit antigène.

13. Composition immunogène pour son utilisation selon la revendication 12, pour traiter ou prévenir une infection, un cancer, une maladie inflammatoire, une allergie, une maladie liée à l'âge comme la dégénérescence maculaire liée à l'âge, ou une maladie neuro-dégénérative.

14. Composition immunogène pour son utilisation selon la revendication 13, dans laquelle ladite infection est une infection virale, bactérienne, parasitaire ou une maladie à prion.

15. Méthode de production d'anticorps polyclonaux, comprenant les étapes consistant en :
(a) l'immunisation d'un animal non-humain avec une composition immunogène telle que définie dans l'une quelconque des revendications 1 à 9, de manière à induire une réponse immune humorale contre ledit antigène, et
(b) la récolte des anticorps polyclonaux induits dirigés contre ledit antigène.

16. Méthode de production d'anticorps monoclonaux, comprenant les étapes consistant en :
(i) l'immunisation d'un animal non-humain avec une composition immunogène telle que définie dans l'une quelconque des revendications 1 à 9,
(ii) récupération et isolement des lymphocytes B de l'animal immunisé à l'étape (i),
(iii) production d'un hybridome et culture dudit l'hybridome afin de produire des anticorps monoclonaux dirigés contre l'antigène présent dans ladite composition immunogène,
(iv) récolte et purification des anticorps monoclonaux produits à l'étape (iii).

## Patentansprüche

1. Immunogene Zusammensetzung, die eine kontinuierliche wässrige Phase und mindestens zwei dispergierte Phasen 1 und 2 in Form von Tröpfchen enthält, wobei:
- die dispergierte Phase 1 enthält:
- ein amphiphiles Lipid 1,
- ein lösungsvermittelndes Lipid 1, das mindestens ein Fettsäureglycerid enthält,
- ein Co-Tensid 1, das mindestens eine Kette, die von Alkylenoxideinheiten zusammengesetzt ist, enthält,
- ein Tensid 1, das ein Antigen der folgenden Formel (I) trägt:
(L₁-X₁-₁-Y₁)ᵥ-G-Z₁-Ag (I)
in der:
- L₁ eine lipophile Gruppe darstellt,
- X₁, Y₁, Z₁ und G unabhängig eine Bindungsgruppe darstellen,
- H₁ eine hydrophile Gruppe darstellt, die eine polyalkoxylierte Kette enthält,
- v eine ganze Zahl von 1 bis 8 ist und
- Ag ein Antigen darstellt,
wobei das Molverhältnis des ein Antigen der Formel (I) tragenden Tensids 1 zur Summe des Co-Tensids1 und des ein Antigen der Formel (I) tragenden Tensids 1 0,01 % bis 5 % ist und
- die dispergierte Phase 2 enthält:
- ein amphiphiles Lipid 2,
- ein lösungsvermittelndes Lipid 2, das mindestens ein Fettsäureglycerid enthält,
- ein Co-Tensid 2, das mindestens eine Kette, die von Alkylenoxideinheiten zusammengesetzt ist, enthält,
- ein mindestens eine anionische Gruppe tragendes Immunostimulanzmittel 2 und
- ein kationisches Tensid 2,
unter der Voraussetzung, dass die dispergierte Phase 2 frei von einem ein Antigen der Formel (I) tragenden Tensid ist.

2. Immunogene Zusammensetzung nach Anspruch 1, bei der in der Formel (I):
- L₁ ausgewählt ist aus:
- einer Gruppe R oder R-(C=O)-, wobei R eine lineare Kohlenwasserstoffkette mit 7 bis 23 Kohlenstoffatomen darstellt,
- einemFettsäureester oder einem Fettsäureamid, das 8 bis 24 Kohlenstoffatome und Phosphatidylethanolamin, wie das Distearylphosphatidylethanolamin (DSPE), enthält und
- einem Poly(propylenoxid) und/oder
- X₁, Y₁ und Z₁ unabhängig ausgewählt sind aus:
- einer einfachen Bindung,
- einer Gruppe Z, ausgewählt aus -O-, -NH-, -O(OC)-, -(CO)O-, -(CO)NH-, -NH(CO)-, -O-(CO)-O-, -NH-(CO)-O-, -O-(CO)-NH- und -NH-(CO)-NH,
- einer Alkgruppe, die ein Alkylen mit 1 bis 8 Kohlenstoffatomen ist, das gegebenenfalls einen Ring, (beispielsweise ein Radikal 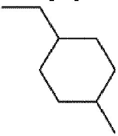 ) enthält, und
- einer Z-Alk-, Alk-Z, Alk-Z-Alk- oder Z-Alk-Z-Gruppe, bei der Alk und Z so sind, wie oben definiert, und bei der die zwei Gruppen Z der Gruppe Z-Alk-Z identisch oder unterschiedlich sind, und/oder
- H₁ ausgewählt ist aus einem Poly(ethylenoxid), das typischerweise 3 bis 500 Ethylenoxideinheiten, vorzugsweise 20 bis 200 Ethylenoxideinheiten, enthält, und/oder
- G mindestens eine Gruppe G' umfasst, die eine der folgenden Formeln aufweist: 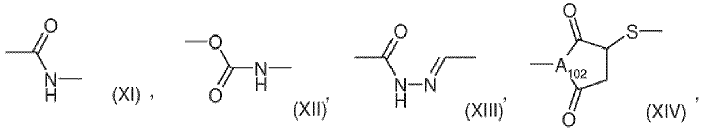 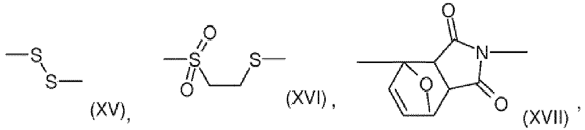 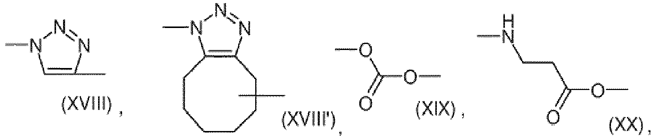 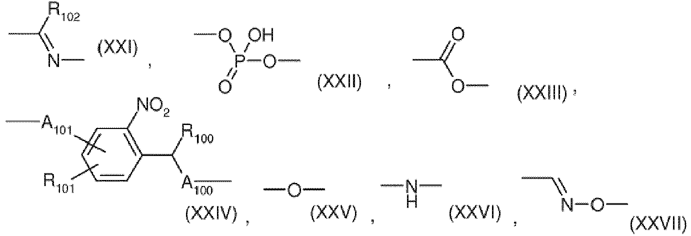 wobei A₁₀₂ CH oder N darstellt, R₁₀₂ H oder eine lineare Kohlenwasserstoffkette mit 1 bis 6 Kohlenstoffatomen darstellt, A₁₀₁ -O-, -NH-(CO)- oder -O-(CO)- darstellt, R₁₀₀ H oder ein Methyl darstellt, A₁₀₀ -O- oder -NH- darstellt und R₁₀₁ H, Me oder -OMe darstellt.

3. Immunogene Zusammensetzung nach Anspruch 1 oder 2, bei der in der Formel (I) das Radikal L₁-X₁-H₁ aus einer der Gruppen der folgenden Formeln besteht: 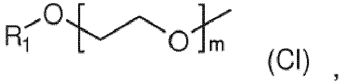 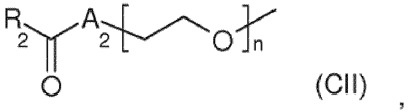 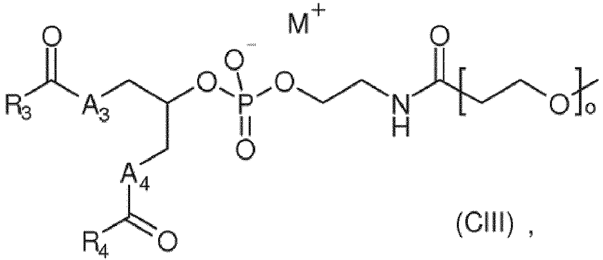 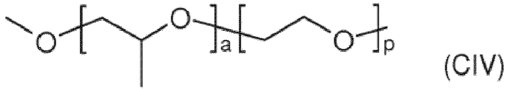 in denen:
- R₁, R₂, R₃ und R₄ unabhängig eine lineare Kohlenwasserstoffkette mit 7 bis 23 Kohlenstoffatomen darstellen,
- A₂, A₃ und A₄ O oder NH darstellen,
- m, n, o und p unabhängig ganze Zahlen von 3 bis 500, vorzugsweise 20 bis 200, darstellen und
- a eine ganze Zahl von 20 bis 120 darstellt,
- M H oder ein Kation darstellt.

4. Immunogene Zusammensetzung nach einem beliebigen der Ansprüche 1 bis 3, bei der das Tensid 1 der Formel (I) die folgende Formel (I') aufweist: 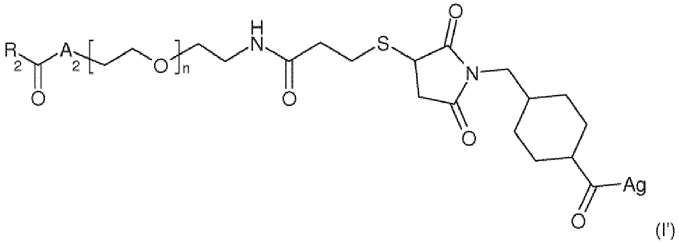 in der:
- R₂ eine lineare Kohlenwasserstoffkette mit 7 bis 23 Kohlenstoffatomen, vorzugsweise 17, darstellt,
- A₂ O oder NH, vorzugsweise NH, darstellt und
- n eine ganze Zahl von 3 bis 500, vorzugsweise 20 bis 200, insbesondere 100, darstellt,
- Ag ein Antigen darstellt.

5. Immunogene Zusammensetzung nach einem beliebigen der Ansprüche 1 bis 4, bei der:
- das amphiphile Lipid 1 und/oder das amphiphile Lipid 2 ein Phospholipid ist (sind) und/oder
- das lösungsvermittelnde Lipid 1 und/oder das das lösungsvermittelnde Lipid 2 aus einer Mischung von gesättigten Fettsäureglyceriden besteht (bestehen), die mindestens 10 Gew.-% C12-Fettsäuren, mindestens 5 Gew.-% C14-Fettsäuren, mindestens 5 Gew.-% C16-Fettsäuren und mindestens 5 Gew.-% C18-Fettsäuren aufweist, und/oder
- das Co-Tensid 1 und/oder das Co-Tensid 2 ausgewählt ist (sind) aus den konjugierten Polyethylenglycol/PhosphatidylEthanolamin-Verbindungen, den Fettsäureethern und Polyethylenglycolethern, den Fettsäureestern und Polyethylenglycolestern und den Blockcopolymeren des Ethylenoxids und Propylenoxids, und die polyalkoxylierte Kette des Co-Tensids 1 und/oder des Co-Tensids 2 10 bis 200 Ethylenoxid-/Propylenoxideinheiten enthält.

6. Immunogene Zusammensetzung nach einem beliebigen der Ansprüche 1 bis 5, bei der das Immunostimulanzmittel 2 vom Typ Nucleotidsequenz ist und das Co-Tensid 1 und/oder 2 mindestens eine Poly(ethylenoxid)-Kette mit mindestens 25 Ethylenoxideinheiten enthält.

7. Immunogene Zusammensetzung nach einem beliebigen der Ansprüche 1 bis 6, bei der das Immunostimulanzmittel 2 eine einzel- oder doppelsträngige Nucleotidsequenz ist, die weniger als 200 Basen für eine einzelsträngige Nucleotidsequenz oder weniger als 200 Basenpaare für eine doppelsträngige Nucleotidsequenz, vorzugsweise das Oligodeoxynucleotid CpG, enthält.

8. Immunogene Zusammensetzung nach einem beliebigen der Ansprüche 1 bis 7, bei der:
- die dispergierte Phase 1 einen biologischen Zielliganden 1, der auf das Co-Tensid 1 aufgepropft ist oder nicht, enthält und/oder die dispergierte Phase 2 einen biologischen Zielliganden 2, der auf das Co-Tensid 2 aufgepfropft ist oder nicht, enthält und/oder
- die dispergierte Phase 1 ein Agens 1 von Interesse enthält, das ausgewählt ist aus einem optischen Agens wie einem Farbstoff, einem Chromophor, Fluorophor oder einem physikalischen Agens wie einem radioaktiven Isotop, und/oder die dispergierte Phase 2 ein Agens 2 von Interesse enthält, das ausgewählt ist aus einem optischen Agens wie einem Farbstoff, einem Chromophor, Fluorophor oder einem physikalischen Agens wie einem radioaktiven Isotop oder einem Photosensibilisierer.

9. Immunogene Zusammensetzung nach einem beliebigen der Ansprüche 1 bis 8, bei dem das kationische Tensid 2 ausgewählt ist aus dem N[1-2(2,3-Dioleyloxy)propy]-N,N,N-Trimethylammonium, dem 1,2-Dioleyl-3-Trimethylammoniumpropan, dem N-(2-Hydroxyethyl)-N,N-Dimethyl-2,3-Bi(tetradecyloxy-1-Propananium), dem 1-[2-(Oleoyloxy)ethyl]-2-Oleyl-3-(2-Hydroxyethyl)imidazolinium und dem Dioctadecylamidoglycylspermin.

10. Verfahren zur Herstellung der immunogenen Zusammensetzung nach einem beliebigen der Ansprüche 1 bis 9, umfassend das Mischen einer Emulsion 1, die eine kontinuierliche wässrige Phase und mindestens eine dispergierte Phase 1, wie sie in einem beliebigen der Ansprüche 1 bis 5 und 8 definiert ist, enthält, und einer Emulsion 2, die eine kontinuierliche wässrige Phase und mindestens eine dispergierte Phase 2, wie sie in einem beliebigen der Ansprüche 1 und 5 bis 9 definiert ist, enthält.

11. Immunogene Zusammensetzung nach einem beliebigen der Ansprüche 1 bis 9 zur Verwendung als Medikament oder Impfstoff.

12. Immunogene Zusammensetzung nach einem beliebigen der Ansprüche 1 bis 9 zum Induzieren einer Immunantwort gegen das Antigen.

13. Immunogene Zusammensetzung zur Verwendung nach Anspruch 12 zum Behandeln oder Verhindern einer Infektion, eines Krebses, einer entzündlichen Krankheit, einer Allergie, einer altersbedingten Krankheit wie der altersbedingten Makuladegeneration oder einer neurodegenerativen Krankheit.

14. Immunogene Zusammensetzung zur Verwendung nach Anspruch 13, in der die Infektion eine virale, bakterielle, parasitäre Infektion oder eine Prionenerkrankung ist.

15. Verfahren zur Herstellung von polyklonalen Antikörpern, das Schritte umfasst, die bestehen aus:
(a) die Immunisierung eines nicht-menschlichen Tieres mit einer immunogenen Zusammensetzung, wie in einem beliebigen der Ansprüche 1 bis 9 definiert, um eine humorale Immunantwort gegen das Antigen zu induzieren, und
(b) die Gewinnung der induzierten polyklonalen Antikörper, die gegen das Antigen gerichtet sind.

16. Verfahren zum Herstellen von monoklonalen Antikörpern, das Schritte umfasst, die bestehen aus:
(i) die Immunisierung eines nicht-menschlichen Tieres mit einer immunogenen Zusammensetzung, wie einem beliebigen der Ansprüche 1 bis 9 definiert,
(ii) Gewinnung und Isolierung der Lymphozyten B des bei Schritt (i) immunisierten Tieres,
(iii) Herstellung eines Hybridoms und Kultur des Hybridoms, um monoklonale Antikörper zu erzeugen, die gegen das in der immunogenen Zusammensetzung vorhandenen Antigen gerichtet sind,
(iv) Gewinnung und Aufreinigung der bei Schritt (iii) erzeugen monoklonalen Antikörper.

## Claims

1. An immunogenic composition comprising a continuous aqueous phase and at least two dispersed phases 1 and 2 as droplets wherein:
- the dispersed phase 1 comprises:
- an amphiphilic lipid 1,
- a solubilizing lipid 1 comprising at least one fatty acid glyceride,
- a co-surfactant 1 comprising at least one chain consisting of alkylene oxide units,
- a surfactant 1 bearing an antigen of the following formula (I):
(L₁-X₁-H₁-Y₁)ᵥ-G-Z₁-Ag (I),
wherein:
- L₁ represents a lipophilic group,
- X₁, Y₁, Z₁ and G represent independently a binding group,
- H₁ represents a hydrophilic group comprising a polyalkoxylated chain,
- v is an integer from 1 to 8, and
- Ag represents an antigen,
wherein the molar ratio of the surfactant 1 bearing an antigen of formula (I) over the sum of the co-surfactant 1 and of the surfactant 1 bearing an antigen of formula (I) is from 0.01% to 5%, and
- the dispersed phase 2 comprises:
- an amphiphilic lipid 2,
- a solubilizing lipid 2 comprising at least one fatty acid glyceride,
- a co-surfactant 2 comprising at least one chain consisting of alkylene oxide units,
- an immunostimulating agent 2 bearing at least an anionic group, and
- a cationic surfactant 2,
with the proviso that the dispersed phase 2 is free of surfactant bearing an antigen of formula (I).

2. The immunogenic composition according to claim 1, wherein, in formula (I):
- L₁ is selected from:
- a group R or R-(C=O)-, wherein R represents a linear hydrocarbon chain comprising from 7 to 23 carbon atoms,
- an ester or amide of fatty acids comprising from 8 to 24 carbon atoms and phosphatidylethanolamine, such as distearyl phosphatidylethanolamine (DSPE), and
- a polypropylene oxide), and/or
- X₁, Y₁ and Z₁ are independently selected from:
- a single bond,
- a group Z selected from -O-, -NH-, -O(OC)-, -(CO)O-, -(CO)NH-, -NH(CO)-, -O-(CO)-O-, -NH-(CO)-O-, -O-(CO)-NH- and -NH-(CO)-NH,
- a group Alk being an alkylene comprising from 1 to 8 carbon atoms optionally comprising a ring (for example a radical 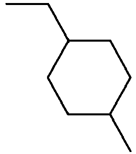 ), and
- a group Z-Alk, Alk-Z, Alk-Z-Alk or Z-Alk-Z wherein Alk and Z are as defined above and wherein both groups Z of the group Z-Alk-Z are identical or different, and/or
- H₁ is selected from a poly(ethylene oxide) typically comprising from 3 to 500 ethylene oxide units, preferably from 20 to 200 ethylene oxide units, and/or
- G comprises at least one group G' having one of the following formulae: 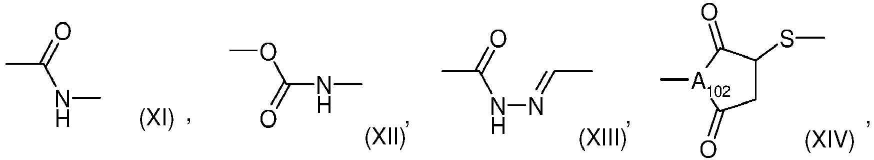 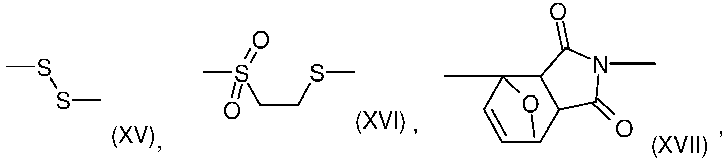 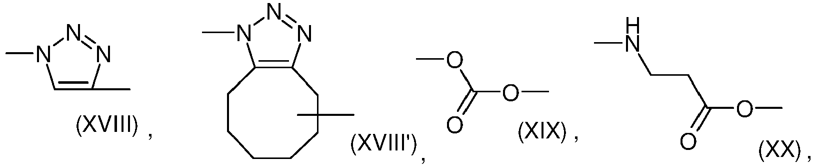 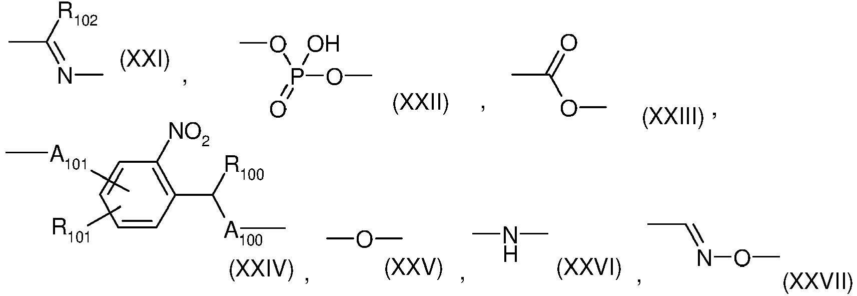 wherein A₁₀₂ represents CH or N, R₁₀₂ represents H or a linear hydrocarbon chain comprising from 1 to 6 carbon atoms, A₁₀₁ represents -O-, -NH-(CO)- or -O-(CO)-, R₁₀₀ represents H or a methyl, A₁₀₀ represents -O- or -NH- and R₁₀₁ represents H, Me or -OMe.

3. The immunogenic composition according to claim 1 or 2, wherein, in formula (I), the radical L₁-X₁-H₁- consists in one of the groups of the following formulae: 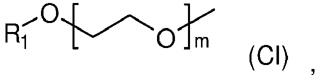 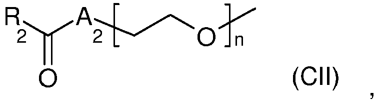 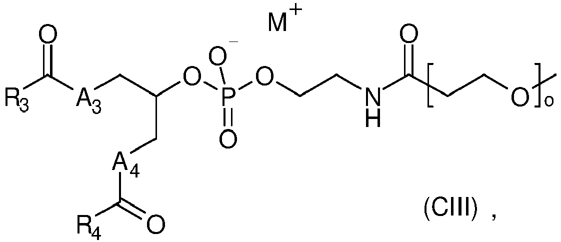 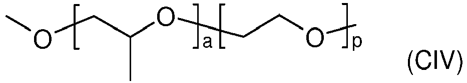 wherein:
- R₁, R₂, R₃ and R₄ represent independently a linear hydrocarbon chain comprising from 7 to 23 carbon atoms,
- A₁, A₂, A₃ and A₄ represent O or NH,
- m, n, o and p represent independently integers from 3 to 500, preferably 20 to 200, and
- a represents an integer from 20 to 120,
- M represents H or a cation.

4. The immunogenic composition according to any of claims 1 to 3, wherein the surfactant 1 of formula (I) has the following formula (I'): 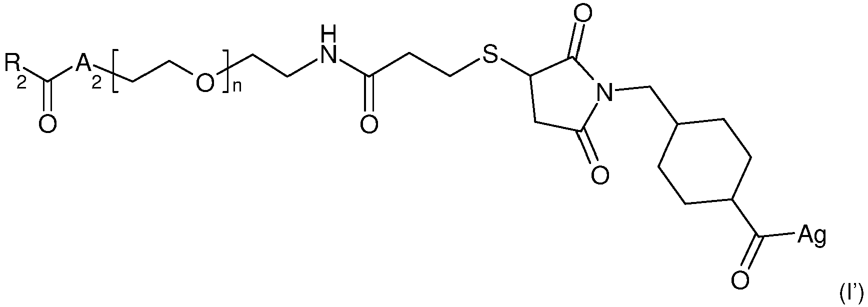 wherein:
- R₂ represents a linear hydrocarbon chain comprising from 7 to 23 carbon atoms, preferably 17,
- A₂ represents O or NH, preferably NH, and
- n represents an integer from 3 to 500, preferably from 20 to 200, notably 100,
- Ag represents an antigen.

5. The immunogenic composition according to any of claims 1 to 4, wherein:
- the amphiphilic lipid 1 and/or the amphiphilic lipid 2 is(are) a phospholipid, and/or
- the solubilizing lipid 1 and/or the solubilizing lipid 2 consist(s) of a mixture of saturated fatty acid glycerides including at least 10% by weight of C₁₂ fatty acids, at least 5% by weight of C₁₄ fatty acids, at least 5% by weight of C₁₆ fatty acids and at least 5% by weight of C18 fatty acids, and/or
- the co-surfactant 1 and/or the co-surfactant 2 is(are) selected from polyethyleneglycol/phosphatidyl-ethanolamine conjugate compounds, fatty acid and polyethyleneglycol ethers, fatty acid and polyethyleneglycol esters and block copolymers of ethylene oxide and propylene oxide, and the polyalkoxylated chain of the co-surfactant 1 and/or the co-surfactant 2 comprise(s) from 10 to 200 ethylene oxide/propylene oxide units.

6. The immunogenic composition according to any of claims 1 to 5, wherein the immunostimulating agent 2 is of the nucleotide sequence type and the co-surfactant 1 and/or 2 comprises at least a poly (ethylene oxide) chain comprising at least 25 ethylene oxide units.

7. The immunogenic composition according to any of claims 1 to 6, wherein the immunostimulating agent 2 is a single or double strand nucleotide sequence, comprising fewer than 200 bases for a single strand nucleotide sequences or fewer than 200 base pairs for a double strand nucleotide sequence, preferably oligodeoxynucleotide CpG.

8. The immunogenic composition according to any of claims 1 to 7, wherein:
- the dispersed phase 1 comprises a biological targeting ligand 1 either grafted or not on the co-surfactant 1, and/or the dispersed phase 2 comprises a biological targeting ligand 2 either grafted or not on the co-surfactant 2, and/or
- the dispersed phase 1 comprises an agent 1 of interest selected from an optical agent such as a coloring agent, a chromophore, a fluorophore or a physical agent, such as a radio-active isotope or a photo-sensitizer, and/or the dispersed phase 2 comprises an agent 2 of interest selected from an optical agent such as a coloring agent, a chromophore, a fluorophore or a physical agent, such as a radio-active isotope or a photo-sensitizer.

9. The immunogenic composition according to any of claims 1 to 8, wherein the cationic surfactant 2, notably selected from among *N*[1-(2,3-dioleyloxy)propyl]-*N,N,N-*trimethylammonium, 1,2-dioleyl-3-trimethylamonium-propane, *N*-(2-hydroxyethyl)-*N,N-*dimethyl-2,3-bis(tetradecyloxy-1-propananium), le 1-[2-(oleoyloxy)ethyl]-2-oleyl-3-(2-hydroxyethyl)imidazolinium, and dioctadecylamidoglycylspermine.

10. A method for preparing the immunogenic composition as defined in any of claims 1 to 9, comprising mixing an emulsion 1 comprising a continuous aqueous phase and at least a dispersed phase 1 as defined in anyone of claims 1 to 5 and 8, and an emulsion 2 comprising a continuous aqueous phase and at least a dispersed phase 2 as defined in anyone of claims 1 to 5 and 9.

11. The immunogenic composition according to any of claims 1 to 9 for its use as a drug or as a vaccine.

12. The immunogenic composition according to any of claims 1 to 9 for inducing an immune response against said antigen.

13. The immunogenic composition for its use according to claim 12, for treating or preventing an infection, a cancer, an inflammatory disease, an allergy, an age-related disease such as age-related macular degeneration, or a neurodegenerative disease.

14. The immunogenic composition for its use according to claim 13, wherein said infection is a viral, bacterial, parasite infection or prion type disease.

15. A method for producing polyclonal antibodies, comprising the steps consisting in:
(a) immunizing a non-human animal with an immunogenic composition as defined according to any of claims 1 to 9, so as to induce a humoral immune response against said antigen, and
(b) harvesting the induced polyclonal antibodies directed against said antigen.

16. A method for producing monoclonal antibodies, comprising the steps consisting in:
(i) immunizing a non-human animal with an immunogenic composition as defined according to any of claims 1 to 9,
(ii) recovering and isolating the B lymphocytes of the immunized animal in step (i),
(iii) producing a hybridoma and cultivating said hybridoma in order to produce monoclonal antibodies directed against the antigen present in said immunogenic composition,
(iv) harvesting and purifying the monoclonal antibodies produced in step (iii).


### SEQUENCE LISTING

<110> Commissariat à l'Energie Atomique et aux Energies Alternatives (C.E.A.) INSTITUT NATIONAL DE LA SANTE ET DE LA RECHERCHE MEDICALE (INSERM)
<120> Composition immunogène sous forme d'émulsion comprenant deux phases dispersées, l'une comprenant un antigène et l'autre comprenant un agent immunostimulant
<130> BET 16P2998
<160> 4
<170> PatentIn version 3.5
<210> 1
   <211> 8
   <212> PRT
   <213> Artificial Sequence
<220>
   <223> Acides aminés 257 à 264 de l'ovalbumine
<400> 1
   Ser Ile Ile Asn Phe Glu Lys Leu
   1               5
<210> 2
   <211> 17
   <212> PRT
   <213> Artificial Sequence
<220>
   <223> Acides aminés 323 à 339 de l'ovalbumine
<400> 2
   Ile Ser Gln Ala Val His Ala Ala His Ala Glu Ile Asn Glu Ala Gly
   1               5                   10                  15
   Arg
<210> 3
   <211> 9
   <212> PRT
   <213> Artificial Sequence
<220>
   <223> Peptide de l'ovalbumine modifié
<220>
   <221> MISC_FEATURE
   <222> (1)..(1)
   <223> Xaa est une maléimide-sérine
<220>
   <221> MISC_FEATURE
   <222> (9)..(9)
   <223> Xaa est une lysine-6carboxyfluorescine
<400> 3
   Xaa Ile Ile Asn Phe Glu Lys Leu Xaa
   1               5
<210> 4
   <211> 17
   <212> PRT
   <213> Artificial Sequence
<220>
   <223> Peptide de l'ovalbumine modifié
<220>
   <221> MISC_FEATURE
   <222> (1)..(1)
   <223> Xaa est une maléimide-isoleucine
<220>
   <221> MISC_FEATURE
   <222> (17)..(17)
   <223> Xaa est une arginine-carboxytetramethylrhodamine
<400> 4
   Xaa Ser Gln Ala Val His Ala Ala His Ala Glu Ile Asn Glu Ala Gly
   1               5                   10                  15
   Xaa